# EUROPEAN PATENT SPECIFICATION

(11) **EP 1 534 736 B1**
(45) Date of publication and mention of the grant of the patent: **02.06.2010**
(21) Application number: 03785123.5
(22) Date of filing: 07.08.2003
(51) Int. Cl.: C07K 5/00, C07K 14/00, C12N 15/11, C12N 15/85, C07H 21/04, A61K 39/395

(54) **NOGO RECEPTOR ANTAGONISTS**
ANTAGONISTEN DES NOGO-REZEPTORS
ANTAGONISTES DE RECEPTEUR NOGO

(30) Priority: 10.08.2002 US 402866 P
(43) Date of publication of application: 01.06.2005
(73) Proprietor: Yale University, New Haven, CT 06511 (US); Biogen Idec MA Inc., Cambridge, Massachusetts 02142 (US)
(72) Inventor: LEE, Daniel, H., S., Sudbury, MA 01776 (US); PEPINSKY, R., Blake, Arlington, MA 02474 (US); LI, Weiwei, Waltham, MA 02453 (US); RABACCHI, Sylvia, A., Belmont, MA 02478 (US); RELTON, Jane, K., Belmont, MA 02478 (US); WORLEY, Dane, S., Somerville, MA 02144 (US); STRITTMATTER, Stephen, M., Guilford, CT 06437 (US); SAH, Dinah, Y., W., Boston, MA 02114 (US)
(74) Representative: Adams, Harvey Vaughan John
(86) International application number: PCT/US2003/025004
(87) International publication number: WO 2004/014311

(56) References cited:
- WO-A-01/51520
- WO-A-03/002602
- OERTLE T. ET AL.: 'Nogo-A inhibits neurite outgrowth and cell spreading with three discrete regions' J. NEUROSCI. vol. 23, no. 13, 02 July 2003, pages 5393 - 5406, XP002973436

## Description

### Field of the Invention

This invention relates to neurobiology and molecular biology. More particularly, this invention relates to immunogenic Nogo receptor-1 polypeptides, Nogo receptor-1 antibodies, antigen-binding fragments thereof, soluble Nogo receptors and fusion proteins thereof and nucleic acids encoding the same. This invention further relates to compositions comprising, and methods for making and using, such Nogo receptor antibodies, antigen-binding fragments thereof, immunogenic Nogo receptor-1 polypeptides, soluble Nogo receptors and fusion proteins thereof and nucleic acids encoding the same.

### Background of the Invention

Axons and dendrites of neurons are long cellular extensions from neurons. The distal tip of an extending axon or neurite comprises a specialized region, known as the growth cone. Growth cones sense the local environment and guide axonal growth toward the neuron's target cell. Growth cones respond to several environmental cues, for example, surface adhesiveness, growth factors, neurotransmitters and electric fields. The guidance of growth at the cone involves various classes of adhesion molecules, intercellular signals, as well as factors that stimulate and inhibit growth cones. The growth cone of a growing neurite advances at various rates, but typically at the speed of one to two millimeters per day.

Growth cones are hand shaped, with broad flat expansion (microspikes or filopodia) that differentially adhere to surfaces in the embryo. The filopodia are continually active, some filopodia retract back into the growth cone, while others continue to elongate through the substratum. The elongations between different filopodia form lamellipodia.

The growth cone explores the area that is ahead of it and on either side with its lamellipodia and filopodia. When an elongation contacts a surface that is unfavorable to growth, it withdraws. When an elongation contacts a favorable growth surface, it continues to extend and guides the growth cone in that direction. The growth cone can be guided by small variations in surface properties of the substrata. When the growth cone reaches an appropriate target cell a synaptic connection is created.

Nerve cell function is greatly influenced by the contact between the neuron and other cells in its immediate environment (U. Rutishauser, T. M. Jessell, Physiol. Rev. 1988, 68, p. 819). These cells include specialized glial cells, oligodendrocytes in the central nervous system (CNS), and Schwann cells in the peripheral nervous system (PNS), which ensheathe the neuronal axon with myelin (an insulating structure of multi-layered membranes) (G. Lemke, in An Introduction to Molecular Neurobiology, Z. Hall, Ed. [Sinauer, Sunderland, Mass., 1992], p. 281).

While CNS neurons have the capacity to regenerate after injury, they are inhibited from doing so because of the presence of inhibitory proteins present in myelin and possibly also by other types of molecules normally found in their local environment (Brittis and Flanagan, Neuron 2001, 30, pp. 11-14; Jones et al., J. Neurosci. 2002, 22, pp. 2792-2803; Grimpe et al., J. Neurosci. 2002, 22, pp. 3144-3160).

Several myelin inhibitory proteins that are found on oligodendrocytes have been characterized, *e.g*., NogoA (Chen et al., Nature, 2000, 403, 434-439; Grandpre et al., Nature 2000, 403, 439-444), myelin associated glycoprotein (MAG, McKerracher et al, Neuron 1994, 13, 805-811; Mukhopadhyay et al, Neuron 1994, 13, 757-767) and oligodendrocyte glycoprotein (OM-gp, Mikol and Stefansson, J. Cell. Biol. 1988, 106, 1273-1279). Each of these proteins has been separately shown to be a ligand for the neuronal Nogo receptor-1 (Wang et al., Nature 2002, 417, 941-944; Liu et al., Science, 2002, 297, 1190-93; Grandpre et al., Nature 2000, 403, 439-444; Chen et al., Nature, 2000, 403, 434-439; Domeniconi et al., Neuron, 2002, 35, 283-90).

Nogo receptor-1 is a GPI-anchored membrane protein that contains 8 leucine rich repeats (Fournier et al., Nature 2001, 409, 341-346; WO 01/51520). Upon interaction with an inhibitory protein (e.g., NogoA, MAG and OM-gp), the Nogo receptor-1 complex transduces signals that lead to growth cone collapse and inhibition of neurite outgrowth.

There is an urgent need for molecules that inhibit Nogo receptor-1 binding to its ligands and attenuate myelin-mediated growth cone collapse and inhibition of neurite outgrowth.

### Summary of the Invention

The subject matter of the present invention is set out in the appended claims. More generally, however, the present disclosure relates to soluble Nogo receptor-1 polypeptides and fusion proteins comprising them, and antibodies and antigenic fragments thereof directed against specific immunogenic regions of Nogo receptor-1. The disclosure also relates to immunogenic Nogo receptor-1 polypeptides that bind to the antibodies of the disclosure. The disclosure further relates to nucleic acids encoding the polypeptides of this disclosure, vectors and host cells comprising such nucleic acids and methods of making the peptides. The antibodies, soluble receptors and receptor fusion proteins of this disclosure antagonize or block Nogo receptor-1 and are useful for inhibiting binding of Nogo receptor-1 to its ligands, inhibiting growth cone collapse in a neuron and decreasing the inhibition of neurite outgrowth or sprouting in a neuron.

In some instances, the disclosure provides an immunogenic polypeptide selected from the group consisting of SEQ ID NO: 1, SEQ ID NO: 2, SEQ ID NO: 3, SEQ ID NO: 4 and SEQ ID NO: 5.

In some instances, the disclosure provides nucleic acids encoding said immunogenic polypeptides, vectors comprising said nucleic acids and host cells comprising said nucleic acids or vectors. In some instances, the nucleic acid is operably linked to an expression control sequence.

In some instances, the disclosure provides a method of producing the immunogenic polypeptide comprising the steps of (a) culturing a host cell comprising the nucleic acid encoding the immunogenic peptide or the vector encoding the same; and (b) recovering the polypeptide from the host cell or culture medium.

In some instances, the disclosure provides a method of producing an antibody that specifically binds a Nogo receptor-1,comprising the steps of (a) immunizing a host with a polypeptide selected from the group consisting of SEQ ID NO: 1, SEQ ID NO: 2, SEQ ID NO: 3, SEQ ID NO: 4 and SEQ ID NO: 5 or a host cell expressing said polypeptide; and (b) recovering the antibody. In some instances, the antibody or antigen-binding fragment thereof is produced by this method. In some instances, the antibody or antigen-binding fragment thereof specifically binds to a polypeptide selected from the group consisting of SEQ ID NO: 1, SEQ ID NO: 2, SEQ ID NO: 3, SEQ ID NO: 4 and SEQ ID NO: 5. In some instances, the antibody or antigen-binding fragment (a) inhibits growth cone collapse of a neuron; (b) decreases the inhibition of neurite outgrowth and sprouting in a neuron; and (c) inhibits Nogo receptor-1 binding to a ligand. In some instances, the antibody or antigen-binding fragment promotes survival of a neuron at risk of dying. In some instances, the neuron at risk of dying is in an animal, *e.g.,* a mammal. In some instances, the neurite outgrowth and sprouting is axonal growth. In some instances, the neuron is a central nervous system (CNS) neuron.

In some instances, the antibody or antigen-binding fragment is a monoclonal antibody. In some instances, the antibody or antigen-binding fragment is a murine antibody. In some instances, the antibody is a humanized antibody, a chimeric antibody, or a single chain antibody.

In some instances, the present disclosure provides a hybridoma cell line selected from the group consisting of HB 7E11 (ATCC^{®} accession No. PTA-4587), HB 1H2 (ATCC^{®} accession No. PTA-4584), HB 3G5 (ATCC^{®} accession No. PTA-4586), HB 5B10 (ATCC^{®} accession No. PTA-4588) and HB 2F7 (ATCC^{®} accession No. PTA-4585). In some instances, the present disclosure provides an antibody or antigen-binding fragment thereof that is produced by the hybridoma cell line.

In some instances, the antibody or antigen-binding fragment thereof comprises a light chain comprising an amino acid sequence selected from the group consisting of (a) the amino acid sequence of SEQ ID NO: 15; (b) the amino acid sequence of SEQ ID NO: 16; and (c) an amino acid sequence comprising the CDR1, CDR2, and CDR3 amino acid sequences of SEQ ID NOs: 22, 23, and 24. In some instances, the antibody or antigen-binding fragment thereof comprises a heavy chain comprising an amino acid sequence selected from the group consisting of (a) the amino acid sequence of SEQ ID NO: 17; (b) the amino acid sequence of SEQ ID NO: 18; and (c) an amino acid sequence comprising the CDR1, CDR2, and CDR3 amino acid sequences of SEQ ID NOs: 19, 20, and 21. In some instances, the present disclosure provides a nucleic acid encoding said antibody or antigen-binding fragment thereof. In some instances, the nucleic acid is operably linked to an expression control sequence. In some instances, the disclosure provides a vector comprising said nucleic acid. In some instances, the disclosure provides a host cell comprising said nucleic acid or comprising a vector comprising the nucleic acid.

In some instances, the antibody or antigen-binding fragment thereof competitively inhibits the binding of an antibody produced by the hybridoma cell line of the present disclosure to a Nogo receptor-1 or to an immunogenic polypeptide selected from the group consisting of SEQ ID NO: 1, SEQ ID NO: 2, SEQ ID NO: 3, SEQ ID NO: 4 and SEQ ID NO: 5.

In some instances, the disclosure provides a method of inhibiting Nogo receptor-1 binding to a ligand, comprising the step of contacting Nogo receptor-1 with an antibody or antigen-binding fragment of this disclosure. In some instances, the ligand is selected from the group consisting of NogoA, NogoB, NogoC, MAG and OM-gp.

In some instances, the disclosure provides a method for inhibiting growth cone collapse in a neuron, comprising the step of contacting the neuron with the antibody or antigen-binding fragment thereof of this disclosure. In some instances, the disclosure provides a method for decreasing the inhibition of neurite outgrowth or sprouting in a neuron, comprising the step of contacting the neuron with the antibody or antigen-binding fragment of this disclosure. In some instances, the neuron is a CNS neuron. In some of these methods, the neurite outgrowth or sprouting is axonal growth.

In some instances, the disclosure provides a method of promoting survival of a neuron at risk of dying, comprising contacting the neuron with an effective amount of (a) an anti-Nogo receptor-1 antibody or antigen-binding fragment thereof; or (b) a soluble Nogo receptor-1 polypeptide. In some instances, the soluble Nogo receptor-1 polypeptide is a fusion protein, *e.g*., an Fc-fusion protein. In some instances, the fusion protein is the sNogoR344-Fc protein. In some instances, the neuron is *in vitro*. In some instances, the neuron is in a mammal displaying signs or symptoms of, *e.g*., multiple sclerosis, ALS, Huntington's disease, Alzheimer's disease, Parkinson's disease, diabetic neuropathy, stroke, traumatic brain injuries and spinal cord injury.

In some instances, the disclosure provides a method of promoting survival of a neuron at risk of dying, the neuron being in a mammal, comprising (a) providing a cultured host cell expressing (i) an anti-Nogo receptor-1 antibody or antigen-binding fragment thereof; or (ii) a soluble Nogo receptor-1 polypeptide; and (b) introducing the host cell into the mammal at or near the site of the neuron.

In some instances, the disclosure provides a gene therapy method of promoting survival of a neuron in a mammal, which neuron is at risk of dying, comprising administering at or near the site of the neuron a viral vector comprising a nucleotide sequence that encodes (a) an anti-Nogo receptor-1 antibody or antigen-binding fragment; or (b) a soluble Nogo receptor-1 polypeptide, wherein the anti-Nogo receptor-1 antibody, antigen-binding fragment, or soluble Nogo receptor-1 polypeptide is expressed from the nucleotide sequence in the mammal in an amount sufficient to promote survival of the neuron.

In some instances, the disclosure provides a soluble Nogo receptor-1 polypeptide consisting essentially of a N-terminal domain (NT), 8 leucine rich repeat domains (LRR) and a LRR C-terminal domain (LRRCT) of Nogo receptor-1. In some instances, said soluble Nogo receptor-1 polypeptide is joined to a signal sequence. In some instances, the LRR comprises a heterologous LRR. In some instances, the disclosure provides a soluble Nogo receptor-1 polypeptide selected from the group consisting of: amino acid residues 26-344 of SEQ ID NO: 6; amino acid residues 26-310 of SEQ ID NO: 7; amino acid residues 26-344 of SEQ ID NO: 8; amino acid residues 26-310 of SEQ ID NO: 9; amino acid residues 27-344 of SEQ ID NO: 8; and amino acid residues 27-310 of SEQ ID NO: 9. In some instances, the disclosure provides a nucleic acid encoding said soluble Nogo receptor-1 polypeptide. In some instances, the nucleic acid is operably linked to an expression control sequence. In some instances, the disclosure provides a vector comprising said nucleic acid. In some instances, the disclosure provides a host cell comprising said nucleic acid or a vector comprising the nucleic acid.

In some instances, the disclosure provides a method of producing a soluble Nogo receptor-1 polypeptide of the disclosure comprising the steps of (a) culturing a host cell comprising a nucleic acid encoding the soluble Nogo receptor-1 polypeptide or a vector comprising the nucleic acid; and (b) recovering the polypeptide from the host cell or culture medium.

In some instances, the disclosure provides a Nogo receptor-1 fusion protein comprising a soluble Nogo receptor-1 and a heterologous polypeptide. In some instances, the soluble Nogo receptor-1 polypeptide consists essentially of a N-terminal domain (NT), 8 leucine rich repeat domains (LRR) and a LRR C-terminal domain (LRRCT) of Nogo receptor-1. In some instances, the soluble Nogo receptor-1 polypeptide is joined to a signal sequence. In some instances, the Nogo receptor-1 fusion protein comprises a heterologous LRR. In some instances, the Nogo receptor-1 fusion protein comprises a polypeptide selected from the group consisting of: amino acid residues 26-344 of SEQ ID NO: 6; amino acid residues 26-310 of SEQ ID NO: 7; amino acid residues 26-344 of SEQ ID NO: 8; amino acid residues 26-310 of SEQ ID NO: 9; amino acid residues 27-344 of SEQ ID NO: 8; and amino acid residues 27-310 of SEQ ID NO: 9. In some instances, the heterologous polypeptide comprises an immunoglobulin constant region. In some instances, the immunoglobulin constant region is an immunoglobulin heavy chain constant region. In some instances, the immunoglobulin heavy chain constant region is an IgG heavy chain constant region. In some instances, the heterologous polypeptide is an Fc region. In some instances, the Nogo receptor-1 fusion protein is a dimer.

In some instances, the disclosure provides a nucleic acid encoding the Nogo receptor-1 fusion protein. In some instances, the nucleic acid encoding the Nogo receptor-1 fusion protein is operably linked to an expression control sequence.

In some instances, the disclosure provides a vector comprising the nucleic acid encoding the Nogo receptor-1 fusion protein.

In some instances, the disclosure provides a host cell comprising the nucleic acid encoding the Nogo receptor-1 fusion protein or the vector comprising the nucleic acid encoding the Nogo receptor-1 fusion protein.

In some instances, the disclosure provides a method of producing the Nogo receptor-1 fusion protein comprising the steps of (a) culturing a host cell comprising a nucleic acid encoding the Nogo receptor-1 fusion protein or a vector comprising the nucleic acid; and (b) recovering the Nogo receptor-1 fusion protein from the host cell or culture medium.

In some instances, the disclosure provides a method of inhibiting Nogo receptor-1 binding to a ligand, comprising the step of contacting the ligand with the soluble Nogo receptor-1 polypeptide or the Nogo receptor-1 fusion protein of this disclosure.

In some instances, the disclosure provides a method of modulating an activity of a Nogo receptor-1 ligand, comprising the step of contacting the Nogo receptor-1 ligand with a soluble Nogo receptor-1 polypeptide or a Nogo receptor-1 fusion protein of the disclosure.

In some instances, the disclosure provides a method for inhibiting growth cone collapse in a neuron, comprising the step of contacting a Nogo receptor-1 ligand with a soluble Nogo receptor-1 polypeptide or a Nogo receptor-1 fusion protein of this disclosure. In some instances, the disclosure provides a method for decreasing the inhibition of neurite outgrowth or sprouting in a neuron, comprising the step of contacting a Nogo receptor-1 ligand with the soluble Nogo receptor-1 polypeptide or the Nogo receptor-1 fusion protein of this disclosure. In some instances, the neuron is a CNS neuron. In some instances, the ligand is selected from the group consisting of NogoA, NogoB, NogoC, MAG and OM-gp. In some instances, the neurite outgrowth or sprouting is axonal growth.

In some instances, the disclosure provides a composition comprising a pharmaceutically acceptable carrier and a component selected from (a) an antibody or an antigen-binding fragment according to this disclosure; (b) a soluble Nogo receptor-1 polypeptide according to this disclosure; and (c) a Nogo receptor-1 fusion protein according to this disclosure. In some instances, the composition further comprises one or more additional therapeutic agents.
Now as set out above, a part of the disclosure that is contained herein provides the subject matter of the present invention, as defined by the appended claims. However, the entirety of the disclosure that is contained herein shall be used to interpret the claims.

### Brief Description Of The Drawings

Figure 1 is a schematic representation of the structure of Nogo receptor-1. Human sNogoR310 contains residues 26-310 and sNogoR344 contains residues 26-344. Rat sNogoR310 contains residues 27-310 and sNogoR344 contains residues 27-344

Figure 2 depicts an antigenicity plot for the Nogo receptor-1 protein using the Vector Nti^{™} software. Rat P-617 is SEQ ID NO: 10 and rat P-618 is SEQ ID NO: 11.

Figure 3A is a graph depicting the binding activity of anti-Nogo receptor-1 antibody, 7E11. The graph presents the effect of 7E11 concentration on the binding of Nogo66 to Nogo receptor-1. Figure 3B depicts the binding activity of anti-Nogo receptor-1 antibody, 1H2. The graph presents the effect of 1H2 concentration on the binding of Nogo66 to sNogoR344-Fc (also referred to herein and in United States patent application 60/402,866 as Fc-sNogoR344 or Ig-sNogoR344). Fc-MAG did not compete with Nogo66 for binding to sNogoR344-Fc.

Figure 4 depicts the results of an ELISA for anti-Nogo-R-1 antibodies 1H2, 3G5 and 2F7. The effect of the antibodies on OD₄₅₀ in the presence of immobilized antigens was determined. The immobilized antigens were sNogoR310-Fc (also referred to herein and in United States patent application 60/402,866 as Fc-sNogoR310 or Ig-sNogoR310), sNogoR344-Fc, p-617, p-618, p-4, p-5 and ovalbumin and BSA.

Figure 5 is a graph depicting the effects of monoclonal antibody, 7E11, on rat DRG neurite outgrowth in the presence of varying amounts of myelin.

Figure 6A is a graph depicting the effect of binding of sNogoR310 to ¹²⁵I-Nogo66 and ¹²⁵I-Nogo40 in the presence of the following competitors: Nogo66, Nogo40 and anti-Nogo receptor-1 monoclonal antibody supernatant. Figure 6B depicts the binding activity of ¹²⁵I-Nogo66 to sNogoR310.

Figure 7 is a graph depicting the effect of sNogoR310-Fc on ¹²⁵I-Nogo40 binding to sNogoR310.

Figure 8 is a graph depicting the binding activity of sNogoR310-Fc to ¹²⁵I-Nogo40.

Figure 9A is a graph of the effect of sNogoR310 on neurite outgrowth/cell in the presence or absence of myelin. Figure 9B is a graph of the effect of sNogoR310 on neurite outgrowth in the presence or absence of myelin.

Figure 10A is a graph depicting the effect of sNogoR310-Fc on P4 rat DRG neurite outgrowth in the presence or absence of increasing amounts of myelin. Figure 10B depicts the number of neurites/cell following treatment with PBS, PBS + sNogoR310-Fc, 20ng myelin and myelin + sNogoR310-Fc.

Figure 11 is a graph depicting the effect of monoclonal antibody 5B10 on DRG neurite outgrowth/cell in the presence of increasing amounts of myelin.

Figure 12 is a graph depicting the effect of sNogoR310-Fc on the BBB score up to 30 days following induction of injury in a rat spinal cord transection model.

Figures 13A and 13B report the locomotor BBB score as a function of time after dorsal hemisection in the WT or transgenic mice from Line 08 or Line 01. Figure 13C graphs the maximal tolerated inclined plane angle as a function of time after injury for WT and transgenic mice. Figure 13D shows hindlimb errors during inclined grid climbing as a function of post-injury time. In all the graphs, means ± s.e.m. from 7-9 mice in each group are reported. The values from transgenic group are statistically different from the WT mice. (double asterisks, P < 0.01; Student's t-test).

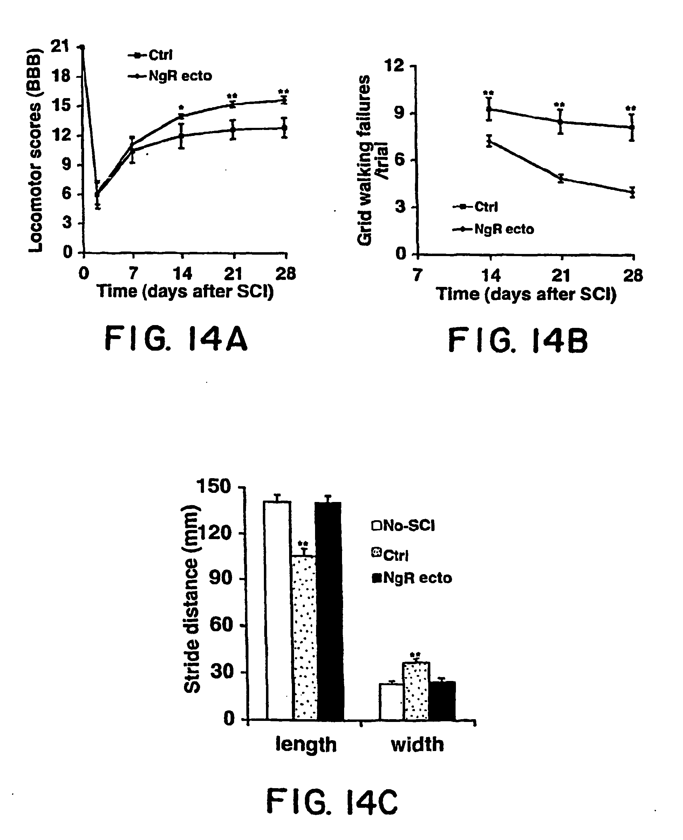

Figure 14A shows the locomotor BBB score as a function of time after dorsal hemisection in vehicle or sNogoR310-Fc treated animals. Figure 14B shows hindlimb errors during grid walking as a function of time after injury. Figure 14C shows footprint analysis revealing a shorter stride length and a greater stride width in control rats than uninjured or injured + sNogoR310-Fc rats. In all the graphs, means ± s.e.m. from 7-9 rats in each group are reported. The values of sNogoR310-Fc group are statistically different from the control (Figures 14A-B). The control values are statistically different from no-SCI or SCI + sNogoR310-Fc rats in Figure 14C. (asterisk, p< 0.05; double asterisks, p < 0.01; Student's t-test).

### DETAILED DESCRIPTION OF THE INVENTION

### Definitions and General Techniques

Unless defined otherwise, all technical and scientific terms used herein have the same meaning as commonly understood by one of ordinary skill in the art to which this invention belongs. In case of conflict, the present application including the definitions will control. Also, unless otherwise required by context, singular terms shall include pluralities and plural terms shall include the singular.

Although methods and materials similar or equivalent to those described herein can be used in the practice or testing of the present disclosure, including the present invention, suitable methods and materials are described below. The materials, methods and examples are illustrative only, and are not intended to be limiting. Other features and advantages of the invention will be apparent from the detailed description and from the claims.

Throughout this specification and claims, the word "comprise," or variations such as "comprises" or "comprising," will be understood to imply the inclusion of a stated integer or group of integers but not the exclusion of any other integer or group of integers.

In order to further define this invention, the following terms and definitions are herein provided.

As used herein, "antibody" means an intact immunoglobulin, or an antigen-binding fragment thereof. Antibodies of this disclosure, including antibodies of the present invention, can be of any isotype or class (*e.g*., M, D, G, E and A) or any subclass (*e.g*., G1-4, A1-2) and can have either a kappa (κ) or lambda (λ) light chain.

As used herein, "Fc" means a portion of the heavy chain constant region of an antibody that is obtainable by papain digestion.

As used herein, "NogoR fusion protein" means a protein comprising a soluble Nogo receptor-1 moiety fused to a heterologous polypeptide.

As used herein, "humanized antibody" means an antibody in which at least a portion of the non-human sequences are replaced with human sequences. Examples of how to make humanized antibodies may be found in United States Patent Nos. 6,054,297, 5,886,152 and 5,877,293.

As used herein, "chimeric antibody" means an antibody that contains one or more regions from a first antibody and one or more regions from at least one other antibody. The first antibody and the additional antibodies can be from the same or different species.

As used herein and in United States patent application 60/402,866, "Nogo receptor," "NogoR," "NogoR-1," "NgR," and "NgR-1" each means Nogo receptor-1.

### Immunogenic Nogo Receptor-1 Polypeptides

In one aspect, the present disclosure, including the present invention, relates to Nogo receptor-1 polypeptides that are immunogenic. In some instances of the present disclosure, the immunogenic polypeptide consists essentially of an amino acid sequence selected from the group consisting of: LDLSDNAQLRVVDPTT (rat) (SEQ ID NO: 1); LDLSDNAQLRSVDPAT (human) (SEQ ID NO: 2); AVASGPFRPFQTNQLTDEELLGLPKCCQPDAADKA (rat) (SEQ ID NO: 3); AVATGPYHPIWTGRATDEEPLGLPKCCQPDAADKA (human)(SEQ ID NO: 4); and CRLGQAGSGA (mouse) (SEQ ID NO: 5).

In some instances, e.g. in certain embodiments of the present invention, the disclosure relates to a nucleic acid encoding a polypeptide of any one of SEQ ID NOs: 1-5. In some instances, e.g. in certain embodiments of the invention, the nucleic acid molecule is linked to an expression control sequence (*e.g*., pCDNA(I)).

The present disclosure also relates to a vector comprising a nucleic acid coding for an immunogenic polypeptide of the disclosure. Certain vectors provide aspects of the present invention. In some instances, e.g. in some embodiments of the invention, the vector is a cloning vector. In some instances, e.g. in some embodiments of the invention, the vector is an expression vector. In some instances, e.g. in some embodiments of the invention, the vector contains at least one selectable marker.

The present disclosure also relates to host cells comprising the above-described nucleic acid or vector, e.g. a nucleic acid or vector of the present invention.

The present disclosure also relates to a method of producing an immunogenic polypeptide of the present disclosure, e.g. an immunogenic polypeptide of the present invention, comprising the step of culturing a host cell.
Host cells comprising a nucleic acid or vector of the present invention, and methods of culturing said host cells and recovering the immunogenic polypeptide of the present invention, form further aspects of the present invention as set out in the claims. In some instances, e.g. in some embodiments, the host cell is prokaryotic. In some instances, e.g. in some embodiments, the host cell is eukaryotic. In some instances, e.g. in some embodiments, the host cell is yeast.

### Antibodies

The present disclosure further relates to an antibody or an antigen-binding fragment thereof that specifically binds an immunogenic Nogo receptor-1 polypeptide of the disclosure, e.g. an immunogenic Nogo receptor-1 polypeptide of the invention. In some instances, the antibody or antigen-binding fragment binds a polypeptide consisting essentially of an amino acid sequence selected from the group consisting of SEQ ID NOs: 1-5. Antibodies and antigen-binding fragments of the present invention are set out in the appended claims. The antibody or antigen-binding fragment of the present disclosure, e.g. an antibody or antigen-binding fragment of the present invention, may be produced *in vivo* or *in vitro*. Production of the antibody or antigen-binding fragment is discussed below.

An antibody or an antigen-binding fragment thereof according to the present disclosure, e.g. according to the invention, inhibits the binding of Nogo receptor-1 to a ligand (*e.g*., NogoA, NogoB, NogoC, MAG, OM-gp) and decreases myelin-mediated inhibition of neurite outgrowth and sprouting, particularly axonal growth, and attenuates myelin mediated growth cone collapse.

In some instances, e.g. in some embodiments, the anti-Nogo receptor-1 antibody or antigen-binding fragment thereof is murine. In some instances, e.g. in some embodiments, the Nogo receptor-1 is from rat. In other instances, e.g. in other embodiments, the Nogo receptor-1 is human. In some instances, e.g. in some embodiments,the anti-Nogo receptor-1 antibody or antigen-binding fragment thereof is recombinant, engineered, humanized and/or chimeric.

In some instances, e.g. in some embodiments, the antibody is selected from the group consisting of: monoclonal 7E11 (ATCC^{®} accession No. PTA-4587); monoclonal 1H2 (ATCC^{®} accession No. PTA-4584); monoclonal 2F7 (ATCC^{®} accession No. PTA-4585); monoclonal 3G5 (ATCC^{®} accession No. PTA-4586); and monoclonal 5B10 (ATCC^{®} accession No. PTA-4588). In some instances, e.g. in some embodiments, the antibody is polyclonal antibody 46.

Exemplary antigen-binding fragments are, Fab, Fab', F(ab')₂, Fv, Fd, dAb, and fragments containing complementarity determining region (CDR) fragments, single-chain antibodies (scFv), chimeric antibodies, diabodies and polypeptides that contain at least a portion of an immunoglobulin that is sufficient to confer specific antigen-binding to the polypeptide (*e.g*., immunoadhesins).

As used herein, Fd means a fragment that consists of the V_{H} and C_{H1} domains; Fv means a fragment that consists of the V_{L} and V_{H} domains of a single arm of an antibody; and dAb means a fragment that consists of a V_{H} domain (Ward et al., Nature 341:544-546, 1989). As used herein, single-chain antibody (scFv) means an antibody in which a V_{L} region and a V_{H} region are paired to form a monovalent molecules via a synthetic linker that enables them to be made as a single protein chain (Bird et al., Science 242:423-426, 1988 and Huston et al., Proc. Natl. Acad. Sci. USA 85:5879-5883, 1988). As used herein, diabody means a bispecific antibody in which V_{H} and V_{L} domains are expressed on a single polypeptide chain, but using a linker that is too short to allow for pairing between the two domains on the same chain, thereby forcing the domains to pair with complementary domains of another chain and creating two antigen-binding sites (see *e.g.,* Holliger, P., et al., Proc. Natl. Acad. Sci. USA 90:6444-6448, 1993, and Poljak, R. J., et al., Structure 2:1121-1123, 1994). As used herein, immunoadhesin that specifically binds an antigen of interest, means a molecule in which one or more CDRs may be incorporated, either covalently or noncovalently.

In some instances, the disclosure provides a subunit polypeptide of a Nogo receptor-1 antibody of the present disclosure, e.g. of a Nogo receptor-1 antibody of the present invention, wherein the subunit polypeptide is selected from the group consisting of: (a) a heavy chain or a variable region thereof; and (b) a light chain or a variable region thereof.

In some instances, the disclosure provides a nucleic acid encoding the heavy chain or the variable region thereof, or the light chain or the variable region thereof, of a subunit polypeptide of a Nogo receptor-1 antibody of the present disclosure, e.g. of a Nogo receptor-1 antibody of the present invention.

In some instances, the disclosure provides a hypervariable region (CDR) of a Nogo receptor-1 antibody of the present disclosure, e.g. of a Nogo-receptor-1 antibody of the present invention, or a nucleic acid encoding a CDR.

### Immunization

Antibodies of the present disclosure, including antibodies of the present invention, can be generated by immunization of a suitable host (e.g., vertebrates, including humans, mice, rats, sheep, goats, pigs, cattle, horses, reptiles, fishes, amphibians, and in eggs of birds, reptiles and fish). Such antibodies may be polyclonal or monoclonal.

In some instances of the present disclosure, the host is immunized with an immunogenic Nogo receptor-1 polypeptide of the present disclosure, e.g. with an immunogenic Nogo receptor-1 polypeptide of the invention. In other instances, the host is immunized with Nogo receptor-1 associated with the cell membrane of an intact or disrupted cell and antibodies of the disclosure, e.g. antibodies of the invention, are identified by binding to an immunogenic Nogo receptor-1 polypeptide of the disclosure, e.g. to an immunogenic Nogo receptor-1 polypeptide of the invention. Certain methods of producing antibodies by immunizing hosts provide further aspects of the present invention as set out in the appended claims.

In some instances, e.g. in some embodiments, the Nogo receptor-1 antigen is administered with an adjuvant to stimulate the immune response. Adjuvants often need to be administered in addition to antigen in order to elicit an immune response to the antigen. These adjuvants are usually insoluble or undegradable substances that promote nonspecific inflammation, with recruitment of mononuclear phagocytes at the site of immunization. Examples of adjuvants include, but are not limited to, Freund's adjuvant, RIBI (muramyl dipeptides), ISCOM (immunostimulating complexes) or fragments thereof.

For a review of methods for making antibodies, see *e*.*g*., Harlow and Lane (1988), Antibodies, A Laboratory Manual, Yelton, D.E. et al. (1981); Ann. Rev. of Biochem., 50, pp. 657-80., and Ausubel et al. (1989); Current Protocols in Molecular Biology (New York: John Wiley & Sons). Determination of immunoreactivity with an immunogenic Nogo receptor-1 polypeptide of the disclosure, e.g. with an immunogenic Nogo receptor-1 polypeptide of the invention, may be made by any of several methods well known in the art, including, *e.g*., immunoblot assay and ELISA.

### Production of Antibodies and Antibody Producing Cell Lines

Monoclonal antibodies of the present disclosure, including monoclonal antibodies of the present invention, can made by standard procedures as described, *e.g*., in Harlow and Lane (1988), supra.

Briefly, at an appropriate period of time the animal is sacrificed and lymph node and/or splenic B-cells are immortalized by any one of several techniques that are well-known in the art, including but not limited to transformation, such as with EBV or fusion with an immortalized cell line, such as myeloma cells. Thereafter, the cells are clonally separated and the supernatants of each clone tested for production of an antibody specific for an immunogenic Nogo receptor-1 polypeptide of the disclosure, e.g. for an immunogenic Nogo receptor-1 polypeptide of the present invention. Methods of selecting, cloning and expanding hybridomas are well known in the art. Similarly, methods for identifying the nucleotide and amino acid sequence of the immunoglobulin genes are known in the art.

Other suitable techniques for producing an antibody of the present disclosure, e.g. an antibody of the present invention, involve *in vitro* exposure of lymphocytes to the Nogo receptor-1 or to an immunogenic polypeptide of the disclosure, e.g. to an immunogenic polypeptide of the invention, or alternatively, selection of libraries of antibodies in phage or similar vectors. See Huse et al., Science, 246, pp. 1275-81 (1989). Antibodies useful in the present disclosure, e.g., in the present invention, may be employed with or without modification.

Antigens (in this case Nogo receptor-1 or an immunogenic polypeptide of the disclosure, e.g. an immunogenic polypeptide of the present invention) and antibodies can be labeled by joining, either covalently or non-covalently, a substance that provides for a detectable signal. Various labels and conjugation techniques are known in the art and can be employed in practicing the invention. Suitable labels include, but are not limited to, radionucleotides, enzymes, substrates, cofactors, inhibitors, fluorescent agents, chemiluminescent agents, magnetic particles and the like. Patents teaching the use of such labels include U.S. Patents 3,817,837; 3,850,752; 3,939,350; 3,996,345; 4,277,437; 4,275,149 and 4,366,241. Also, recombinant immunoglobulins may be produced (see U.S. Patent 4,816,567).

In some instances of the present disclosure, e.g. in some embodiments of the invention, an antibody has multiple binding specificities, such as a bifunctional antibody prepared by any one of a number of techniques known to those of skill in the art including the production of hybrid hybridomas, disulfide exchange, chemical crosslinking, addition of peptide linkers between two monoclonal antibodies, the introduction of two sets of immunoglobulin heavy and light chains into a particular cell line, and so forth (see below for more detailed discussion).

The antibodies of this disclosure, e.g. the antibodies of the present invention, may also be human monoclonal antibodies, for example those produced by immortalized human cells, by SCID-hu mice or other non-human animals capable of producing "human" antibodies.

### Phage Display Libraries

Anti-Nogo receptor-1 antibodies of this disclosure, e.g. anti-Nogo receptor-1 antibodies of the present invention, can be isolated by screening a recombinant combinatorial antibody library. Exemplary combinatorial libraries are screened for binding to an immunogenic Nogo receptor-1 polypeptide of the disclosure, e.g. to an immunogenic Nogo receptor-1 polypeptide of the invention, such as a scFv phage display library, prepared using V_{L} and V_{H} cDNAs prepared from mRNA derived from an animal immunized with an immunogenic Nogo receptor-1 polypeptide of the disclosure, e.g. with an immunogenic Nogo receptor-1 polypeptide of the invention. Methodologies for preparing and screening such libraries are known in the art. There are commercially available methods and materials for generating phage display libraries (*e.g.*, the Pharmacia Recombinant Phage Antibody System, catalog no. 27-9400-01; the Stratagene SurfZAP^{™} phage display kit, catalog no. 240612; and others from MorphoSys). There are also other methods and reagents that can be used in generating and screening antibody display libraries (see *e.g.,* Ladner et al. U.S. Pat. No. 5,223,409; Kang et al. PCT Publication No. WO 92/18619; Dower et al. PCT Publication No. WO 91/17271; Winter et al. PCT Publication No. WO 92/20791; Markland et al. PCT Publication No. WO 92/15679; Breitling et al. PCT Publication No. WO 93/01288; McCafferty et al. PCT Publication No. WO 92/01047; Garrard et al. PCT Publication No. WO 92/09690; Fuchs et al. (1991) Bio/Technology 9:1370-1372; Hay et al. (1992) Hum. Antibod. Hybridomas 3:81-85; Huse et al. (1989) Science 246:1275-1281; McCafferty et al., Nature (1990) 348:552-554; Griffiths et al. (1993) EMBO J. 12:725-734; Hawkins et al. (1992) J. Mol. Biol. 226:889-896; Clackson et al. (1991) Nature 352:624-628; Gram et al. (1992) Proc. Natl. Acad. Sci. USA 89:3576-3580; Garrad et al. (1991) Bio/Technology 9:1373-1377; Hoogenboom et al. (1991) Nucl. Acids Res. 19:4133-4137; and Barbas et al. (1991) Proc. Natl. Acad. Sci. USA 88:7978-7982).

Following screening and isolation of an anti-Nogo receptor-1 antibody of the disclosure, e.g. an anti-Nogo receptor-1 antibody of the invention, from a recombinant immunoglobulin display library, the nucleic acid encoding the selected antibody can be recovered from the display package (*e.g.*, from the phage genome) and subcloned into other expression vectors by standard recombinant DNA techniques. If desired, the nucleic acid can be further manipulated to create other antibody forms of the present disclosure, e.g. other antibody forms of the invention, as described below. To express an antibody isolated by screening a combinatorial library, DNA encoding the antibody heavy chain and light chain or the variable regions thereof is cloned into a recombinant expression vector and introduced into a mammalian host cell, as described above.

### Class Switching

Anti-Nogo receptor-1 antibodies of the disclosure, including anti-Nogo receptor-1 antibodies of the invention, can be of any isotype. An antibody of any desired isotype can be produced by class switching. For class switching, nucleic acids encoding V_{L} or V_{H}, that do not include any nucleotide sequences encoding C_{L} or C_{H}, are isolated using methods well known in the art. The nucleic acids encoding V_{L} or V_{H} are then operatively linked to a nucleotide sequence encoding a C_{L} or C_{H} from a desired class of immunoglobulin molecule. This may be achieved using a vector or nucleic acid that comprises a C_{L} or C_{H} chain, as described above. For example, an anti-Nogo receptor-1 antibody of the disclosure, e.g. an anti-Nogo receptor-1 antibody of the invention, that was originally IgM may be class switched to an IgG. Further, the class switching may be used to convert one IgG subclass to another, *e.g.,* from IgG1 to IgG2.

### Mutated Antibodies

Antibodies or antigen-binding fragments of the present disclosure, e.g. antibodies or antigen-binding fragments of the present invention, may be mutated in the variable domains of the heavy and/or light chains to alter a binding property of the antibody. For example, a mutation may be made in one or more of the CDR regions to increase or decrease the K_{d} of the antibody for Nogo receptor-1, to increase or decrease K_{off}, or to alter the binding specificity of the antibody. Techniques in site-directed mutagenesis are well known in the art. See *e.g.,* Sambrook et al. and Ausubel et al., *supra*. In a preferred instance, e.g. in a preferred embodiment, mutations are made at an amino acid residue that is known to be changed compared to the germline in a variable region of an anti-Nogo receptor-1 antibody of the disclosure, e.g. of an anti-Nogo receptor-1 antibody of the invention, as appropriate. In some instances, e.g. in some embodiments, mutations are made at one or more amino acid residues that are known to be changed compared to the germline in a variable region of an anti-Nogo receptor-1 antibody of the present disclosure, e.g. of an anti-Nogo receptor-1 antibody of the invention, as appropriate. In another instance, e.g. in another embodiment, a nucleic acid encoding an antibody heavy chain or light chain variable region is mutated in one or more of the framework regions. A mutation may be made in a framework region or constant domain to increase the half-life. A mutation in a framework region or constant domain also may be made to alter the immunogenicity of the antibody, to provide a site for covalent or non-covalent binding to another molecule, or to alter such properties as complement fixation. Mutations may be made in each of the framework regions, the constant domain and the variable regions in a single mutated antibody. Alternatively, mutations may be made in only one of the framework regions, the variable regions or the constant domain in a single mutated antibody.

### Fusion Antibodies and Immunoadhesins

A fusion antibody or immunoadhesin may be made which comprises all or a portion of an anti-Nogo receptor-1 antibody of the present disclosure, e.g. of an anti-Nogo receptor-1 antibody of the present invention, linked to another polypeptide. In some cases, only the variable region of the anti-Nogo receptor-1 antibody is linked to the polypeptide. In other cases, the V_{H} domain of an anti-Nogo receptor-1 antibody of the disclosure, e.g. of an anti-Nogo receptor-1 antibody of this invention, is linked to a first polypeptide, while the V_{L} domain of the antibody is linked to a second polypeptide that associates with the first polypeptide in a manner that permits the V_{H} and V_{L} domains to interact with one another to form an antibody binding site. In other cases, the V_{H} domain is separated from the V_{L} domain by a linker that permits the V_{H} and V_{L} domains to interact with one another (see below under Single Chain Antibodies). The V_{H}-linker- V_{L} antibody is then linked to a polypeptide of interest. The fusion antibody is useful to directing a polypeptide to a cell or tissue that expresses a Nogo receptor-1 ligand. The polypeptide of interest may be a therapeutic agent, such as a toxin, or may be a diagnostic agent, such as an enzyme that may be easily visualized, such as horseradish peroxidase. In addition, fusion antibodies can be created in which two (or more) single-chain antibodies are linked to one another. This is useful if one wants to create a divalent or polyvalent antibody on a single polypeptide chain, or if one wants to create a bispecific antibody.

### Single Chain Antibodies

The present disclosure includes a single chain antibody (scFv) that binds an immunogenic Nogo receptor-1 polypeptide of the present disclosure, e.g. an immunogenic Nogo receptor-1 polypeptide of the invention. To produce the ScFv, V_{H}- and V_{L}-encoding DNA is operatively linked to DNA encoding a flexible linker, *e.g.,* encoding the amino acid sequence (Gly₄-Ser)₃ (SEQ ID NO:10), such that the V_{H} and V_{L} sequences can be expressed as a contiguous single-chain protein, with the V_{L} and V_{H} regions joined by the flexible linker (see *e.g.,* Bird et al. (1988) Science 242:423-426; Huston et al. (1988) Proc. Natl. Acad. Sci. USA 85:5879-5883; McCafferty et al., Nature (1990) 348:552-554). The single chain antibody may be monovalent, if only a single V_{H} and V_{L} are used, bivalent, if two V_{H} and V_{L} are used, or polyvalent, if more than two V_{H} and VL are used. Certain single chain antibodies of the present disclosure form an aspect of the present invention, as set out in the appended claims.

### Chimeric Antibodies

Also provided is a bispecific antibody or antigen-binding fragment thereof in which one specificity is for an immunogenic Nogo receptor-1 polypeptide of the disclosure, e.g. for an immunogenic Nogo receptor-1 polypeptide of the invention. A chimeric antibody can be generated that specifically binds to an immunogenic Nogo receptor-1 polypeptide of the disclosure, e.g. to an immunogenic Nogo receptor-1 polypeptide of the invention, through one binding domain,and to a second molecule through a second binding domain. The chimeric antibody can be produced through recombinant molecular biological techniques, or may be physically conjugated together. In addition, a single chain antibody containing more than one V_{H} and V_{L} may be generated that binds specifically to an immunogenic polypeptide of the disclosure, e.g. to an immunogenic polypeptide of the invention, and to another molecule that is associated with attenuating myelin mediated growth cone collapse and inhibition of neurite outgrowth and sprouting. Such bispecific antibodies can be generated using techniques that are well known for example, Fanger et al. Immunol Methods 4: 72-81 (1994) and Wright and Harris, *supra.* and in connection with (iii) see *e.g.,* Traunecker et al. Int. J. Cancer (Suppl.) 7: 51-52 (1992).

In some cases, the chimeric antibodies are prepared using one or more of the variable regions from an antibody of the present disclosure, e.g. from an antibody of the invention. In another case, the chimeric antibody is prepared using one or more CDR regions from said antibody.

### Derivatized and Labeled Antibodies

An antibody or an antigen-binding fragment of the present disclosure, e.g. an antibody or antigen-binding fragment of the invention, can be derivatized or linked to another molecule (*e.g.,* another peptide or protein). In general, the antibody or antigen-binding fragment is derivatized such that binding to an immunogenic polypeptide of the disclosure, e.g. to an immunogenic polypeptide of the invention, is not affected adversely by the derivatization or labeling. For example, an antibody or antibody portion of the present disclosure, e.g. an antibody or antibody portion of the invention, can be functionally linked (by chemical coupling, genetic fusion, noncovalent association or otherwise) to one or more other molecular entities, such as another antibody (*e.g.*, a bispecific antibody or a diabody), a detection agent, a cytotoxic agent, a pharmaceutical agent, and/or a protein or peptide that can mediate association of the antibody or antigen-binding fragment with another molecule (such as a streptavidin core region or a polyhistidine tag).

In some instances, e.g. in some embodiments, a derivatized antibody is produced by crosslinking two or more antibodies (of the same type or of different types, *e.g.,* to create bispecific antibodies). Suitable crosslinkers include those that are heterobifunctional, having two distinctly reactive groups separated by an appropriate spacer (*e.g.,* m-maleimidobenzoyl-N-hydroxysuccinimide ester) or homobifunctional (*e.g.*, disuccinimidyl suberate). Such linkers are available from Pierce Chemical Company, Rockford, Ill.

In some instances, e.g. in some embodiments, the derivatized antibody is a labeled antibody. Exemplary, detection agents with which an antibody or antibody portion of the invention may be derivatized are fluorescent compounds, including fluorescein, fluorescein isothiocyanate, rhodamine, 5-dimethylamine-1-napthalenesulfonyl chloride, phycoerythrin, lanthanide phosphors and the like. An antibody also may be labeled with enzymes that are useful for detection, such as horseradish peroxidase, β-galactosidase, luciferase, alkaline phosphatase, glucose oxidase and the like. In instances, e.g. in embodiments that are labeled with a detectable enzyme, the antibody is detected by adding additional reagents that the enzyme uses to produce a detectable reaction product. For example, horseradish peroxidase with hydrogen peroxide and diaminobenzidine. An antibody also may be labeled with biotin, and detected through indirect measurement of avidin or streptavidin binding. An antibody may also be labeled with a predetermined polypeptide epitopes recognized by a secondary reporter (*e.g.,* leucine zipper pair sequences, binding sites for secondary antibodies, metal binding domains, epitope tags) .

An anti-Nogo receptor-1 antibody or an antigen-fragment thereof also may be labeled with a radio-labeled amino acid. The radiolabel may be used for both diagnostic and therapeutic purposes. The radio-labeled anti-Nogo receptor-1 antibody may be used diagnostically, for example, for determining Nogo receptor-1 levels in a subject. Further, the radio-labeled anti-Nogo receptor-1 antibody may be used therapeutically for treating spinal cord injury. Examples of labels for polypeptides include, but are not limited to, the following radioisotopes or radionucleotides - ³H, ¹⁴C, ¹⁵N, ³⁵S, ⁹⁰Y, ⁹⁹TC, ¹¹¹In, ¹²⁵I, ¹³¹I.

An anti-Nogo receptor-1 antibody or an antigen-fragment thereof may also be derivatized with a chemical group such as polyethylene glycol (PEG), a methyl or ethyl group, or a carbohydrate group. These groups may be useful to improve the biological characteristics of the antibody, *e.g.,* to increase serum half-life or to increase tissue binding.

### Characterization of Anti-Nogo receptor-1 Antibodies

### Class and Subclass of Anti-Nogo receptor-1 Antibodies

The class and subclass of anti-Nogo receptor-1 antibodies may be determined by any method known in the art. In general, the class and subclass of an antibody may be determined using antibodies that are specific for a particular class and subclass of antibody. Such antibodies are available commercially. The class and subclass can be determined by ELISA, Western Blot as well as other techniques. Alternatively, the class and subclass may be determined by sequencing all or a portion of the constant domains of the heavy and/or light chains of the antibodies, comparing their amino acid sequences to the known amino acid sequences of various class and subclasses of immunoglobulins, and determining the class and subclass of the antibodies.

### Binding affinity of anti-Nogo receptor-1 antibody to Nogo receptor-1

The binding affinity and dissociation rate of an anti-Nogo receptor-1 antibody of the disclosure, e.g. of an anti-Nogo receptor-1 antibody of the invention, to an immunogenic Nogo receptor-1 polypeptide of the disclosure, e.g. to an immunogenic Nogo receptor-1 polypeptide of the invention, may be determined by any method known in the art. For example, the binding affinity can be measured by competitive ELISAs, RIAs, BIAcore or KinExA technology. The dissociation rate also can be measured by BIAcore or KinExA technology. The binding affinity and dissociation rate are measured by surface plasmon resonance using, *e.g.,* a BIAcore.

The K_{d} of 7E11 and 1H2 were determined to be 1 x 10⁻⁷ M and 2 x 10⁻⁸ M, respectively.

### Inhibition of Nogo receptor-1 activity by anti- Nogo receptor-1 antibody

In some embodiments, an anti-Nogo receptor-1 antibody or an antigen-binding fragment of the present disclosure, e.g. an anti-Nogo receptor-1 antibody or antigen-binding fragment of the invention, inhibits the binding of Nogo receptor-1 to a ligand. The IC₅₀ of such inhibition can be measured by any method known in the art, *e.g.,* by ELISA, RIA, or Functional Antagonism. In some instances, e.g. in some embodiments, the IC₅₀ is between 0.1 and 500 nM. In some instances, e.g. in some embodiments, the IC₅₀ is between 10 and 400 nM. In yet other instances, e.g. in other embodiments, the antibody or portion thereof has an IC₅₀ of between 60 nM and 400 nM. The IC₅₀ of 7E11 and 1H2 were determined to be 400 nM and 60 nM, respectively, in a binding assay. See also Table 3, infra.

In sum, one of skill in the art, provided with the teachings of this disclosure, including this invention, has available a variety of methods which may be used to alter the biological properties of the antibodies of this disclosure, including the antibodies of this invention, including methods which would increase or decrease the stability or half-life, immunogenicity, toxicity, affinity or yield of a given antibody molecule, or to alter it in any other way that may render it more suitable for a particular application.

Compositions comprising, and uses of, the antibodies of the present disclosure, e.g. the antibodies of the present invention, are described below.

### Soluble Nogo Receptor-1 Polypeptides

### Protein

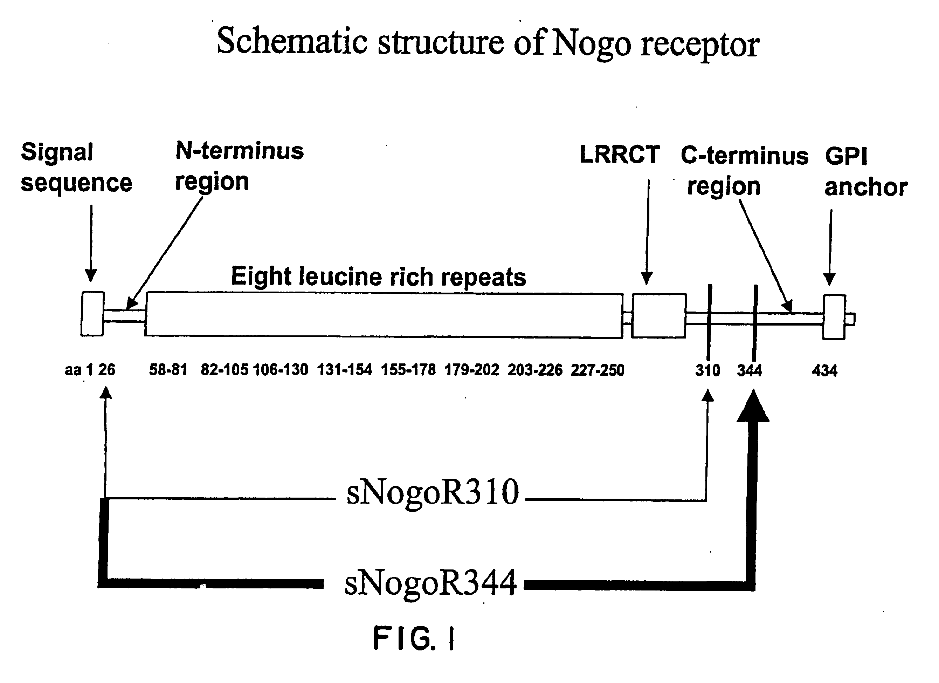

Full-length Nogo receptor-1 consists of a signal sequence, a N-terminus region (NT), eight leucine rich repeats (LRR), a LRRCT region (a leucine rich repeat domain C-terminal of the eight leucine rich repeats), a C-terminus region (CT) and a GPI anchor (see Fig. 1).

Some instances of the present disclosure, including some embodiments of the invention, provide a soluble Nogo receptor-1 polypeptide. Soluble Nogo receptor-1 polypeptides of the disclosure, including those of the invention, comprise an NT domain; 8 LRRs and an LRRCT domain and lack a signal sequence and a functional GPI anchor (*i.e.*, no GPI anchor or a GPI anchor that lacks the ability to efficiently associate to a cell membrane).

In some instances of the present disclosure, a soluble Nogo receptor-1 polypeptide comprises a heterologous LRR. In some instances, a soluble Nogo receptor-1 polypeptide comprises 2, 3, 4, 5, 6, 7, or 8 heterologous LRR's. A heterologous LRR means an LRR obtained from a protein other than Nogo receptor-1. Exemplary proteins from which a heterologous LRR can be obtained are toll-like receptor (TLR1.2); T-cell activation leucine repeat rich protein; deceorin; OM-gp; insulin-like growth factor binding protein acidic labile subunit slit and robo; and toll-like receptor 4.

In some instances, e.g in some embodiments of the present invention, the disclosure provides a soluble Nogo receptor-1 polypeptide of 319 amino acids (soluble Nogo receptor-1 344, sNogoR1-344, or sNogoR344) (residues 26-344 of SEQ ID NOs: 6 and 8 or residues 27-344 of SEQ ID NO: 8). In some instances, e.g. in some embodiments of the present invention, the disclosure provides a soluble Nogo receptor-1 polypeptide of 285 amino acids (soluble Nogo receptor-1 310, sNogoR1-310, or sNogoR310) (residues 26-310 of SEQ ID NOs: 7 and 9 or residues 27-310 of SEQ ID NO: 9). See Fig. 1.

**Table 1. Sequences of Human and Rat Nogo receptor-1 Polypeptides**

| | |
|---|---|
| SEQ ID NO: 6 (human 1-344) | |
| SEQ ID NO: 7 (human 1-310) | |
| SEQ ID NO: 8 (rat 1-344) | |
| SEQ ID NO: 9 (rat 1-310) | |

The soluble Nogo receptor-1 polypeptides of the disclosure, e.g. the soluble Nogo receptor-1 polypeptides of the invention, may be used to inhibit the binding of a ligand to Nogo receptor-1 and act as an antagonist of Nogo receptor-1 ligands. The soluble Nogo receptor-1 polypeptides of the disclosure, e.g. the soluble Nogo receptor-1 polypeptides of the invention, may be used to decrease inhibition of neurite outgrowth and sprouting in a neuron, such as axonal growth and to inhibit myelin mediated growth cone collapse in a neuron. In some cases, the neuron is a CNS neuron.

sNogoR310 and sNogoR344, surprisingly, block the binding of NogoA, NogoB, NogoC, MAG and OM-gp to Nogo receptor-1.

In some cases, the soluble Nogo receptor-1 polypeptide of the disclosure, e.g. the soluble Nogo receptor-1 polypeptide of the invention, is a component of a fusion protein that further comprises a heterologous polypeptide. In some cases, the heterologous polypeptide is an immunoglobulin constant domain. In some cases, the immunoglobulin constant domain is a heavy chain constant domain. In some cases, the heterologous polypeptide is an Fc fragment. In some cases the Fc is joined to the C-terminal end of the soluble Nogo receptor-1 polypeptide of the disclosure, e.g. of the soluble Nogo receptor-1 polypeptide of the invention. In some cases, the fusion Nogo receptor-1 protein is a dimer. Fusion proteins of the invention are set out in the appended claims.

### Nucleic Acid Molecules of the Present Invention

The present disclosure provides a nucleic acid that encodes a polypeptide of the disclosure, including the polypeptides of any one of SEQ ID NOs: 1-9. Certain nucleic acids of the present disclosure form aspects of the present invention, these being set out in the appended claims. In some instances of the disclosure, e.g. in some embodiments of the invention, the nucleic acid encodes a polypeptide selected from the group consisting of amino acid residues 26-344 of Nogo receptor-1 as shown in SEQ ID NOs: 6 and 8 or amino acid residues 27-344 of Nogo receptor-1 as shown in SEQ ID NO: 8. In some instances, e.g. in some embodiments, the nucleic acid molecule encodes a polypeptide selected from the group consisting of amino acid residues 26-310 of Nogo receptor-1 as shown in SEQ ID NOs: 7 and 9 or amino acid residues 27-310 of Nogo receptor-1 as shown in SEQ ID NO: 9. As used herein, "nucleic acid" means genomic DNA, cDNA, mRNA and antisense molecules, as well as nucleic acids based on alternative backbones or including alternative bases whether derived from natural sources or synthesized. In some instances, e.g. in some embodiments, the nucleic acid further comprises a transcriptional promoter and optionally a signal sequence each of which is operably linked to the nucleotide sequence encoding the polypeptide of the present disclosure, e.g. the polypeptide of the present invention, as appropriate.

Also provided is a nucleic acid encoding a Nogo receptor-1 fusion protein of the present disclosure, e.g. a Nogo receptor-1 fusion protein of the invention. In some instances, e.g. in some embodiments, the nucleic acid encodes a Nogo receptor-1 fusion protein of the invention, including a fusion protein comprising a polypeptide selected from the group consisting of amino acid residues 26-344 of Nogo receptor-1 as shown in SEQ ID NOs: 6 and 8 or amino acid residues 27-344 of SEQ ID NO: 8 and amino acid residues 26-310 of Nogo receptor-1 as shown in SEQ ID NOs: 7 and 9 or amino acid residues 27-310 of SEQ ID NO: 9. In some instances, e.g. in some embodiments, the nucleic acid encoding a Nogo receptor-1 fusion protein further comprises a transcriptional promoter and optionally a signal sequence. In some instances, e.g. in some embodiments, the nucleotide sequence further encodes an immunoglobulin constant region. In some instances, e.g. in some embodiments, the immunoglobulin constant region is a heavy chain constant region. In some instances, e.g. in some embodiments, the nucleotide sequence further encodes an immunoglobulin heavy chain constant region joined to a hinge region. In some instances, e.g. in some embodiments, the nucleic acid further encodes Fc. In some instances, e.g. in some embodiments, the Nogo receptor-1 fusion proteins comprise an Fc fragment.

The encoding nucleic acids of the present disclosure, e.g. those of the present invention,may further be modified so as to contain a detectable label for diagnostic and probe purposes. A variety of such labels are known in the art and can readily be employed with the encoding molecules herein described. Suitable labels include, but are not limited to, biotin, radiolabeled nucleotides and the like. A skilled artisan can employ any of the art known labels to obtain a labeled encoding nucleic acid molecule.

### Compositions

Also provided are compositions comprising an immunogenic polypeptide selected from the group consisting of SEQ ID NO: 1, SEQ ID NO: 2, SEQ ID NO: 3, SEQ ID NO: 4 and SEQ ID NO: 5.

Further provided are compositions comprising an anti-Nogo receptor-1 antibody or an antigen-binding fragment thereof, or a soluble Nogo receptor-1 polypeptide or fusion protein of the present disclosure, e.g. of the present invention. Certain compositions of the disclosure provide a further aspect of the present invention, as set out in the appended claims.

In some instances, e.g. in some embodiments of the present invention, the compositions may contain suitable pharmaceutically acceptable carriers comprising excipients and auxiliaries which facilitate processing of the active compounds into preparations which can be used pharmaceutically for delivery to the site of action. Suitable formulations for parenteral administration include aqueous solutions of the active compounds in water-soluble form, for example, water-soluble salts. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides. Aqueous injection suspensions may contain substances which increase the viscosity of the suspension include, for example, sodium carboxymethyl cellulose, sorbitol and dextran. Optionally, the suspension may also contain stabilizers. Liposomes can also be used to encapsulate the molecules of this disclosure, e.g. the molecules of this invention, for delivery into the cell. Exemplary "pharmaceutically acceptable carriers" are any and all solvents, dispersion media, coatings, antibacterial and antifungal agents, isotonic and absorption delaying agents, and the like that are physiologically compatible, water, saline, phosphate buffered saline, dextrose, glycerol, ethanol and the like, as well as combinations thereof. In some instances, e.g. in some embodiments, the compositions comprise isotonic agents, for example, sugars, polyalcohols such as mannitol, sorbitol, or sodium chloride. In some instances, e.g. in some embodiments, the compositions comprise pharmaceutically acceptable substances such as wetting agents or minor amounts of auxiliary substances such as wetting or emulsifying agents, preservatives or buffers, which enhance the shelf life or effectiveness of the antibodies, antigen-binding fragments, soluble Nogo receptors or fusion proteins of the present disclosure, including those of the invention.

Compositions of the present disclosure, e.g. those of the invention, may be in a variety of forms, including, for example, liquid, semi-solid and solid dosage forms, such as liquid solutions (*e.g.,* injectable and infusible solutions), dispersions or suspensions. The preferred form depends on the intended mode of administration and therapeutic application. In one instance, e.g. in one embodiment, compositions are in the form of injectable or infusible solutions, such as compositions similar to those used for passive immunization of humans with other antibodies.

The compositions can be formulated as a solution, micro emulsion, dispersion, liposome, or other ordered structure suitable to high drug concentration. Sterile injectable solutions can be prepared by incorporating the anti-Nogo receptor-1 antibody in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

In some instances, e.g. in some embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See *e.g.,* Sustained and Controlled Release Drug Delivery Systems, J. R. Robinson, ed., Marcel Dekker, Inc., New York, 1978.

Supplementary active compounds also can be incorporated into the compositions. In some instances, e.g. in some embodiments, Nogo receptor-1 antibodies or antigen-binding fragments thereof, or soluble Nogo receptor-1 polypeptides or fusion proteins of the disclosure, including those of the invention, are coformulated with and/or coadministered with one or more additional therapeutic agents.

The pharmaceutical compositions may include a "therapeutically effective amount" or a "prophylactically effective amount" of an antibody, antigen-binding fragment, polypeptide(s), or fusion protein of the disclosure, e.g. of the invention. A "therapeutically effective amount" refers to an amount effective, at dosages and for periods of time necessary, to achieve the desired therapeutic result. A therapeutically effective amount of the Nogo receptor-1 antibody or antigen-binding fragment thereof, soluble Nogo receptor-1 polypeptide or Nogo receptor fusion protein may vary according to factors such as the disease state, age, sex, and weight of the individual. A therapeutically effective amount is also one in which any toxic or detrimental effects of the antibody, antigen-binding fragment, soluble Nogo receptor-1 polypeptide or Nogo receptor fusion protein are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

Dosage regimens may be adjusted to provide the optimum desired response (*e.g.,* a therapeutic or prophylactic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage unit form as used herein refers to physically discrete units suited as unitary dosages for the mammalian subjects to be treated, each unit containing a predetermined quantity of active compound calculated to produce the desired therapeutic effect in association with the required pharmaceutical carrier. The specification for the dosage unit forms of the present disclosure, e.g. of the present invention, are dictated by and directly dependent on (a) the unique characteristics of the antibody, antigen-binding fragment, and soluble receptor-1 polypeptide or Nogo receptor fusion protein and the particular therapeutic or prophylactic effect to be achieved, and (b) the limitations inherent in the art of compounding such an antibody, antigen-binding fragment, and soluble receptor-1 polypeptide or Nogo receptor fusion protein for the treatment of sensitivity in individuals. In some instances, e.g. in some embodiments, a therapeutically effective dose range for Nogo receptor-1 antibodies or antigen-binding fragments thereof is 0.1 - 4 mg/Kg per day. In some instances, e.g. in some embodiments, a therapeutically effective dose range for Nogo receptor-1 antibodies or antigen-binding fragments thereof is 0.2 - 4 mg/Kg per day. In some instances, e.g. in some embodiments, a therapeutically effective dose range for Nogo receptor-1 antibodies or antigen-binding fragments thereof is 0.2 mg/Kg per day.

### Uses of the Antibodies, Antigen-Binding Fragments, Soluble Receptors and Fusion Proteins

In some instances, the present disclosure provides methods for inhibiting Nogo receptor-1 activity by administering anti-Nogo receptor-1 antibodies, antigen-binding fragments of such antibodies, soluble Nogo receptor-1 polypeptides, or fusion proteins comprising such polypeptides to a mammal in need thereof.

In some instances, the disclosure provides a method of inhibiting Nogo receptor-1 binding to a ligand, comprising the step of contacting Nogo receptor-1 with an antibody or antigen-binding fragment of the present disclosure, e.g. of the present invention. In some instances, the ligand is selected from the group consisting of NogoA, NogoB, NogoC, MAG and OM-gp.

In some instances, the present disclosure provides a method for inhibiting growth cone collapse in a neuron, comprising the step of contacting the neuron with the antibody or antigen-binding fragment the present disclosure, e.g. of the present invention. In some instances, the disclosure provides a method for decreasing the inhibition of neurite outgrowth or sprouting in a neuron, comprising the step of contacting the neuron with the antibody or antigen-binding fragment of the present disclosure, e.g. of the present invention. In some instances, the neuron is a CNS neuron. In some of these methods, the neurite outgrowth or sprouting is axonal growth.

In some instances, the present disclosure provides a method of promoting the survival of a neuron in a mammal, which neuron is at risk of dying, comprising (a) providing a cultured host cell expressing (i) an anti-Nogo receptor-1 antibody or antigen-binding fragment thereof; or (ii) a soluble Nogo receptor-1 polypeptide; and (b) introducing the host cell into the mammal at or near the site of the neuron. Almudena Ramon-Cueto, M Isabel Cordero, Fernando F Santos-Benito and Jesus Avila (2000) Functional recovery of paralegic rats and motor axon regneration in their spinal cords by olfactory ensheathing cells. Neuron 25, 425-435.

In some instances, the present disclosure provides a gene therapy method of promoting survival of a neuron at risk of dying, which neuron is in a mammal, comprising administering at or near the site of the neuron a viral vector comprising a nucleotide sequence that encodes (a) an anti-Nogo receptor-1 antibody or antigen-binding fragment thereof; or (b) a soluble Nogo receptor-1 polypeptide, wherein the anti-Nogo receptor-1 antibody, antigen-binding fragment, or soluble Nogo receptor-1 polypeptide is expressed from the nucleotide sequence in the mammal in an amount sufficient to promote survival of the neuron. Viral vectors and methods useful for these embodiments are described in, *e.g.*, Noël et al., Human Gene Therapy, 13, 1483-93 (2002).

In some instances, the disclosure provides a method of inhibiting Nogo receptor-1 binding to a ligand, comprising the step of contacting the ligand with the soluble Nogo receptor-1 polypeptide or the Nogo receptor-1 fusion protein of the present disclosure, e.g. of the present invention.

In some instances, the disclosure provides a method of modulating an activity of a Nogo receptor-1 ligand, comprising the step of contacting the Nogo receptor-1 ligand with a soluble Nogo receptor-1 polypeptide or a Nogo receptor-1 fusion protein of the present disclosure e.g. of the present invention.

In some instances, the disclosure provides a method for inhibiting growth cone collapse in a neuron, comprising the step of contacting a Nogo receptor-1 ligand with a soluble Nogo receptor-1 polypeptide or a Nogo receptor-1 fusion protein of the present disclosure, e.g. of the present invention. In some instances, the disclosure provides a method for decreasing the inhibition of neurite outgrowth or sprouting in a neuron, comprising the step of contacting a Nogo receptor-1 ligand with the soluble Nogo receptor-1 polypeptide or the Nogo receptor-1 fusion protein of the present disclosure, e.g. of the present invention. In some instances, the neuron is a CNS neuron. In some instances, the ligand is selected from the group consisting of NogoA, NogoB, NogoC, MAG and OM-gp. In some instances, the neurite outgrowth or sprouting is axonal growth.

Any of the types of antibodies or receptors described herein may be used therapeutically. In some instances, the anti-Nogo receptor-1 antibody is a human antibody. In some instances, the mammal is a human patient. In some instances, the antibody or antigen-binding fragment thereof is administered to a non-human mammal expressing a Nogo receptor-1 with which the antibody cross-reacts (*e.g.*, a primate, cynomologous or rhesus monkey) for veterinary purposes or as an animal model of human disease. Such animal models may be useful for evaluating the therapeutic efficacy of antibodies of this disclosure, including the antibodies of this invention.

In some instances, administration of anti-Nogo receptor-1 antibody or antigen-binding fragment, or soluble Nogo receptor-1 polypeptide or fusion protein is used to treat a spinal cord injury to facilitate axonal growth throughout the injured site.

The anti-Nogo receptor-1 antibodies or antigen-binding fragments, or soluble Nogo receptor-1 polypeptides or fusion proteins of the present disclosure, including those of the present invention, can be provided alone, or in combination, or in sequential combination with other agents that modulate a particular pathological process. For example, anti-inflammatory agents may be coadministered following stroke as a means for blocking further neuronal damage and inhibition of axonal regeneration. As used herein, the Nogo receptor-1 antibodies, antigen-binding fragments, soluble Nogo receptor-1 and Nogo receptor fusion proteins, are said to be administered in combination with one or more additional therapeutic agents when the two are administered simultaneously, consecutively or independently.

The anti-Nogo receptor-1 antibodies, antigen-binding fragments, soluble Nogo receptor-1 polypeptides, or Nogo receptor-1 fusion proteins of the present disclosure, including those of the present invention, can be administered via parenteral, subcutaneous, intravenous, intramuscular, intraperitoneal, transdermal, inhalational or buccal routes. For example, an agent may be administered locally to a site of injury via microinfusion. Typical sites include, but are not limited to, damaged areas of the spinal cord resulting from injury. The dosage administered will be dependent upon the age, health, and weight of the recipient, kind of concurrent treatment, if any, frequency of treatment, and the nature of the effect desired.

The compounds of this disclosure, including those of the present invention, can be utilized in vivo, ordinarily in mammals, such as humans, sheep, horses, cattle, pigs, dogs, cats, rats and mice, or *in vitro.*
Methods and uses in accordance with the present invention are set out in the appended claims.

### Vectors of the Invention

The present disclosure, including the invention, provides recombinant DNA molecules (rDNA) that contain a coding sequence. As used herein, a rDNA molecule is a DNA molecule that has been subjected to molecular manipulation. Methods for generating rDNA molecules are well known in the art, for example, see Sambrook et al., (1989) Molecular Cloning - A Laboratory Manual, Cold Spring Harbor Laboratory Press. In some rDNA molecules, a coding DNA sequence is operably linked to expression control sequences and vector sequences.

Also provided are vectors comprising the nucleic acids encoding the polypeptides of the present disclosure, e.g. the polypeptides of the invention. Vectors that form part of the present invention are set out in the appended claims. The choice of vector and expression control sequences to which the nucleic acids of this disclosure, e.g. the nucleic acids of this invention, are operably linked depends directly, as is well known in the art, on the functional properties desired (*e.g.*, protein expression, and the host cell to be transformed). A vector of the present disclosure, e.g. a vector of the present invention, may be at least capable of directing the replication or insertion into the host chromosome, and preferably also expression, of the structural gene included in the rDNA molecule.

Expression control elements that are used for regulating the expression of an operably linked protein encoding sequence are known in the art and include, but are not limited to, inducible promoters, constitutive promoters, secretion signals, and other regulatory elements. Preferably, the inducible promoter is readily controlled, such as being responsive to a nutrient in the host cell's medium.

In one instance, e.g. in one embodiment, the vector containing a coding nucleic acid molecule will include a prokaryotic replicon, *i.e.,* a DNA sequence having the ability to direct autonomous replication and maintenance of the recombinant DNA molecule extra-chromosomally in a prokaryotic host cell, such as a bacterial host cell, transformed therewith. Such replicons are well known in the art. In addition, vectors that include a prokaryotic replicon may also include a gene whose expression confers a detectable or selectable marker such as a drug resistance. Typical of bacterial drug resistance genes are those that confer resistance to ampicillin or tetracycline.

Vectors that include a prokaryotic replicon can further include a prokaryotic or bacteriophage promoter capable of directing the expression (transcription and translation) of the coding gene sequences in a bacterial host cell, such as *E. coli.* A promoter is an expression control element formed by a DNA sequence that permits binding of RNA polymerase and transcription to occur. Promoter sequences compatible with bacterial hosts are typically provided in plasmid vectors containing convenient restriction sites for insertion of a DNA segment of the present invention. Examples of such vector plasmids are pUC8, pUC9, pBR322 and pBR329 (Bio-Rad^{®} Laboratories), pPL and pKK223 (Pharmacia). Any suitable prokaryotic host can be used to express a recombinant DNA molecule encoding a protein of the present disclosure, e.g. a protein of the present invention.

Expression vectors compatible with eukaryotic cells, preferably those compatible with vertebrate cells, can also be used to form rDNA molecules that contain a coding sequence. Eukaryotic cell expression vectors are well known in the art and are available from several commercial sources. Typically, such vectors are provided containing convenient restriction sites for insertion of the desired DNA segment. Examples of such vectors are pSVL and pKSV-10 (Pharmacia), pBPV-1, pML2d (International Biotechnologies), pTDT1 (ATCC^{®} 31255) and other eukaryotic expression vectors.

Eukaryotic cell expression vectors used to construct the rDNA molecules of the present disclosure, including rDNA molecules of the present invention, may further include a selectable marker that is effective in an eukaryotic cell, preferably a drug resistance selection marker. A preferred drug resistance marker is the gene whose expression results in neomycin resistance, *i.e.,* the neomycin phosphotransferase (neo) gene. (Southern et al., (1982) J. Mol. Anal. Genet. 1, 327-341). Alternatively, the selectable marker can be present on a separate plasmid, the two vectors introduced by co-transfection of the host cell, and transfectants selected by culturing in the appropriate drug for the selectable marker.

To express the antibodies, or antibody portions of the present disclosure, e.g. the antibodies or antibody portions of the present invention, DNAs encoding partial or full-length light and heavy chains are inserted into expression vectors such that the genes are operatively linked to transcriptional and translational control sequences. Expression vectors include plasmids, retroviruses, cosmids, YACs, EBV-derived episomes, and the like. The antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vectors. Both genes may, however, be inserted into the same expression vector. The antibody genes are inserted into the expression vector by standard methods (*e*.*g*., ligation of complementary restriction sites on the antibody gene fragment and vector, or blunt end ligation if no restriction sites are present).

A convenient vector is one that encodes a functionally complete human C_{H} or C_{L} immunoglobulin sequence, with appropriate restriction sites engineered so that any V_{H} or V_{L} sequence can be easily inserted and expressed, as described above. In such vectors, splicing usually occurs between the splice donor site in the inserted J region and the splice acceptor site preceding the human C region, and also at the splice regions that occur within the human C_{H} exons. Polyadenylation and transcription termination occur at native chromosomal sites downstream of the coding regions. The recombinant expression vector can also encode a signal peptide that facilitates secretion of the antibody chain from a host cell. The antibody chain gene may be cloned into the vector such that the signal peptide is linked in-frame to the amino terminus of the antibody chain gene. The signal peptide can be an immunoglobulin signal peptide or a heterologous signal peptide (i.e., a signal peptide from a non-immunoglobulin protein).

In addition to encoding the immunogenic polypeptides, Nogo receptor-1 antibodies, antigen-binding fragments, soluble Nogo receptor-1 polypeptides and soluble Nogo receptor-1 fusion proteins of the present disclosure, e.g. of the present invention, the recombinant expression vectors that are disclosed herein carry regulatory sequences that control their expression in a host cell. It will be appreciated by those skilled in the art that the design of the expression vector, including the selection of regulatory sequences may depend on such factors as the choice of the host cell to be transformed, the level of expression of protein desired, etc. Preferred regulatory sequences for mammalian host cell expression include viral elements that direct high levels of protein expression in mammalian cells, such as promoters and/or enhancers derived from retroviral LTRs, cytomegalovirus (CMV) (such as the CMV promoter/enhancer), Simian Virus 40 (SV40) (such as the SV40 promoter/enhancer), adenovirus, (*e.g.*, the adenovirus major late promoter (AdMLP)), polyoma and strong mammalian promoters such as native immunoglobulin and actin promoters. For further description of viral regulatory elements, and sequences thereof, see *e.g.,* U.S. Pat. No. 5,168,062 by Stinski, U.S. Pat. No. 4,510,245 by Bell et al. and U.S. Pat. No. 4,968,615 by Schaffner et al.

In addition to the heterologous genes and regulatory sequences, the recombinant expression vectors of the present disclosure, including those of the present invention, may carry additional sequences, such as sequences that regulate replication of the vector in host cells (*e.g.*, origins of replication) and selectable marker genes. The selectable marker gene facilitates selection of host cells into which the vector has been introduced (see *e.g.,* U.S. Pat. Nos. 4,399,216, 4,634,665 and 5,179,017, all by Axel et al.). For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in dhfr⁻ host cells with methotrexate selection/amplification) and the neo gene (for G418 selection).

### Host Cells and Methods of Recombinantly Producing Protein of the Invention

Nucleic acid molecules encoding anti-Nogo receptor-1 antibodies, immunogenic peptides, soluble Nogo receptor-1 polypeptides, or soluble Nogo receptor-1 fusion proteins of this disclosure, e.g. of this invention, and vectors comprising these nucleic acid molecules, can be used for transformation of a suitable host cell. Transformation can be by any known method for introducing polynucleotides into a host cell. Methods for introduction of heterologous polynucleotides into mammalian cells are well known in the art and include dextran-mediated transfection, calcium phosphate precipitation, polybrene-mediated transfection, protoplast fusion, electroporation, encapsulation of the polynucleotide(s) in liposomes, and direct microinjection of the DNA into nuclei. In addition, nucleic acid molecules may be introduced into mammalian cells by viral vectors.

Transformation of appropriate cell hosts with an rDNA molecule of the present disclosure, e.g. an rDNA molecule of the present invention, is accomplished by well known methods that typically depend on the type of vector used and host system employed. With regard to transformation of prokaryotic host cells, electroporation and salt treatment methods can be employed (see, for example, Sambrook et al., (1989) Molecular Cloning - A Laboratory Manual, Cold Spring Harbor Laboratory Press; Cohen et al., (1972) Proc. Natl. Acad. Sci. USA 69, 2110-2114). With regard to transformation of vertebrate cells with vectors containing rDNA, electroporation, cationic lipid or salt treatment methods can be employed (see, for example, Graham et al., (1973) Virology 52, 456-467; Wigler et al., (1979) Proc. Natl. Acad. Sci. USA 76, 1373-1376).

Successfully transformed cells, i.e., cells that contain an rDNA molecule of the present disclosure, e.g. an rDNA molecule of the present invention, can be identified by well known techniques including the selection for a selectable marker. For example, cells resulting from the introduction of an rDNA of the present disclosure, e.g. an rDNA molecule of the present invention, can be cloned to produce single colonies. Cells from those colonies can be harvested, lysed and their DNA content examined for the presence of the rDNA using a method such as that described by Southern, (1975) J. Mol. Biol. 98, 503-517 or the proteins produced from the cell may be assayed by an immunological method.

Mammalian cell lines available as hosts for expression are well known in the art and include many immortalized cell lines available from the American Type Culture Collection (ATCC^{®}). These include, inter alia, Chinese hamster ovary (CHO) cells, NSO, SP2 cells, HeLa cells, baby hamster kidney (BHK) cells, monkey kidney cells (COS), human hepatocellular carcinoma cells (*e.g.,* Hep G2), A549 cells, and a number of other cell lines. Cell lines of particular preference are selected through determining which cell lines have high expression levels. Other cell lines that may be used are insect cell lines, such as Sf9 cells. When recombinant expression vectors encoding the immunogenic polypeptides, Nogo receptor-1 antibodies or antigen-binding fragments, soluble Nogo receptor-1 polypeptides and soluble Nogo receptor-1 fusion proteins of the present disclosure, e.g. of the present invention, are introduced into mammalian host cells, they are produced by culturing the host cells for a period of time sufficient to allow for expression of the antibody, polypeptide and fusion polypeptide in the host cells or, more preferably, secretion of the immunogenic polypeptides, Nogo receptor-1 antibodies or antigen-binding fragments, soluble Nogo receptor-1 polypeptides and soluble Nogo receptor-1 fusion proteins into the culture medium in which the host cells are grown. Immunogenic polypeptides, Nogo receptor-1 antibodies or antigen-binding fragments, soluble Nogo receptor-1 polypeptides and soluble Nogo receptor-1 fusion proteins of the present disclosure, e.g. of the present invention, can be recovered from the culture medium using standard protein purification methods.

Further, expression of immunogenic polypeptides, Nogo receptor-1 antibodies or antigen-binding fragments, soluble Nogo receptor-1 polypeptides and soluble Nogo receptor-1 fusion proteins of the present disclosure, e.g. of the present invention, (or other moieties therefrom) from production cell lines can be enhanced using a number of known techniques. For example, the glutamine synthetase gene expression system (the GS system) is a common approach for enhancing expression under certain conditions. The GS system is discussed in whole or part in connection with European Patent Nos. 0 216 846, 0 256 055, and 0 323 997 and European Patent Application No. 89303964.4.

### Host Cells

Also provided are host cells transformed with a nucleic acid molecule that encodes a Nogo receptor-1 antibody, antigen-binding fragment, soluble Nogo receptor-1 polypeptide and/or soluble Nogo receptor-1 fusion protein of the present disclosure, e.g. a fusion protein of the invention. Certain host cells of the present disclosure provide further aspects of the present invention as set out out in the appended claims. Host cells can be either prokaryotic or eukaryotic. Eukaryotic cells useful for expression of a protein of the present disclosure, e.g. a protein of the present invention, are not limited, so long as the cell line is compatible with cell culture methods and compatible with the propagation of the expression vector and expression of the gene product. Preferred eukaryotic host cells include, but are not limited to, yeast, insect and mammalian cells, preferably vertebrate cells such as those from a mouse, rat, monkey or human cell line. Examples of useful eukaryotic host cells include Chinese hamster ovary (CHO) cells available from the ATCC^{®} as CCL61, NIH Swiss mouse embryo cells NIH-3T3 available from the ATCC as CRL1658, baby hamster kidney cells (BHK), and the like eukaryotic tissue culture cell lines.

### Production of Recombinant Proteins using a rDNA Molecule

Also provided are methods for producing a Nogo receptor-1 antibody or antigen-binding fragment, soluble Nogo receptor-1 polypeptide and/or soluble Nogo receptor-1 fusion protein of the present disclosure, e.g. of the invention, using nucleic acid molecules herein described. In general terms, the production of a recombinant form of a protein typically involves the following steps:

First, a nucleic acid molecule is obtained that encodes a protein of the present disclosure, e.g. a protein of the invention. If the encoding sequence is uninterrupted by introns, it is directly suitable for expression in any host.

The nucleic acid molecule is then optionally placed in operable linkage with suitable control sequences, as described above, to form an expression unit containing the protein open reading frame. The expression unit is used to transform a suitable host and the transformed host is cultured under conditions that allow the production of the recombinant protein. Optionally the recombinant protein is isolated from the medium or from the cells; recovery and purification of the protein may not be necessary in some instances where some impurities may be tolerated.

Each of the foregoing steps can be done in a variety of ways. For example, the desired coding sequences may be obtained from genomic fragments and used directly in appropriate hosts. The construction of expression vectors that are operable in a variety of hosts is accomplished using appropriate replicons and control sequences, as set forth above. The control sequences, expression vectors, and transformation methods are dependent on the type of host cell used to express the gene and were discussed in detail earlier. Suitable restriction sites can, if not normally available, be added to the ends of the coding sequence so as to provide an excisable gene to insert into these vectors. A skilled artisan can readily adapt any host/expression system known in the art for use with the nucleic acid molecules of the present disclosure, e.g. with the nucleic acid molecules of the present invention, to produce recombinant protein. Certain methods of producing proteins of the present disclosure provide yet further aspects of the present invention as set out in the appended claims.

In order that this invention may be better understood, the following examples are set forth. These examples are for purposes of illustration only and are not to be construed as limiting the scope of the invention in any manner.

### EXAMPLE 1

### Production of Murine Monoclonal Anti-Nogo Receptor-1 Antibodies

Anti-Nogo receptor-1 antibodies that specifically bind an immunogenic Nogo receptor-1 polypeptide of the invention were made using the following methods and procedures.

### Immunizations

Two immunization approaches were used:

### 1. COS-7 Cells or Cell Membranes Containing Nogo receptor-1 (NogoR-1) As the Immunogen

The rat Nogo receptor-1 gene (GenBank^{™} No. AF 462390) was subcloned into the mammalian expression vector pEAG1256 (Biogen^{®}) that contained the CMV promotor and geneticin resistance gene for drug selection. The recombinant plasmid was transfected into COS-7 cells using Superfect (Qiagen^{®}). Transfectants were selected using geneticin (Gibco^{™}, 2 mg/ml), cloned and verified for surface expression of Nogo receptor-1 protein by FACS. COS-7 membranes were prepared from these cells according to procedures as described [Wang et al., J. Neurochem., 75:1155-1161 (2000)] with two washings, and stored at 1 mg/ml [protein concentration] in 10% glycerol at -70°C.

Eight-week-old female RBF mice (Jackson Labs, Bar Harbor, ME) were immunized intraperitoneally either with an emulsion containing 50 g rat Nogo receptor-1-COS-7 membranes or whole COS-7 cells expressing Nogo receptor-1 on the surface and 50 µl RIBI MPL+TDM+CWS adjuvant (Sigma^{®} Chemical Co., St. Louis, MO) once every two weeks (Lipman et al., 1992). Sera from the immunized mice were collected before the first immunization, 7 days after the second and third immunizations, and 38 days after the third immunization and the anti-Nogo receptor-1 antibody titers were measured by ELISA as described below.

### 2. Specific Nogo receptor-1 Peptides as the Immunogen

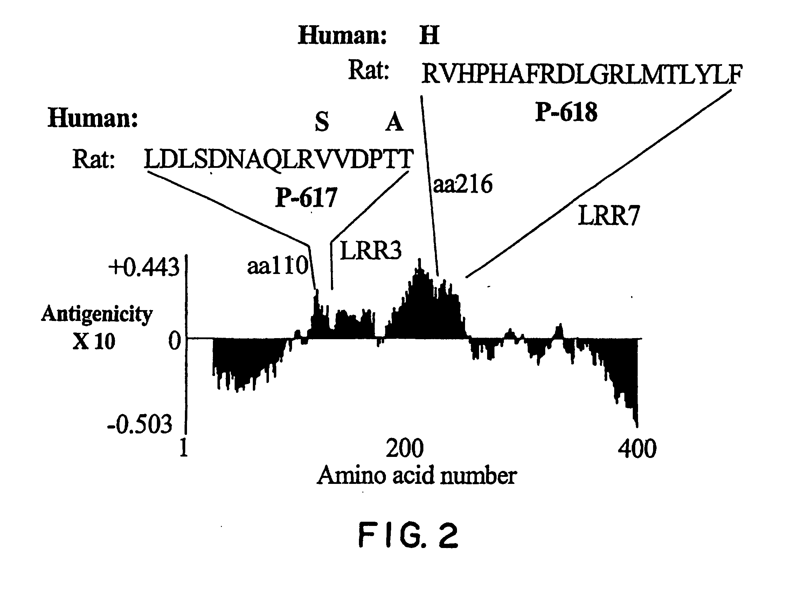

The rat Nogo receptor-1 gene sequence was subjected to antigenicity analyses using Vector NTi^{™} software (Fig. 2). Antigenic peptides,identified in the analyses were conjugated to Keyhole Limpet Hemocyanin (KLH) using standard glutaldehyde procedures.

Eight-week-old female RBF mice (Jackson Labs, Bar Harbor, ME) were immunized intraperitoneally with an emulsion containing 50 µg KLH-conjugated peptides and 50 µl complete Freund's adjuvant (Sigma^{®} Chemical Co., St. Louis, MO) once every two weeks. Serum from the immunized mice was collected before the first immunization and 1 week after the second and third immunizations and anti-Nogo receptor-1 antibody titers were measured. A booster dose was given after the third immunization. Three days after this booster dose immunization, fusion experiments were initiated.

### Hybridoma production and screening

Sera from mice immunized with antigenic Nogo receptor-1 peptides were screened by ELISA whereas sera from mice immunized with COS-7 cells expressing Nogo receptor-1 were screened by flow cytometry. Mice that were positive for antibodies that specifically bound Nogo receptor-1-COS-7 cells were identified by flow cytometry and were sacrificed. Splenocytes were isolated from the mice and fused to the FL653 myeloma (an APRT- derivative of a Ig-/HGPRT- Balb/c mouse myeloma, maintained in DMEM containing 10% FBS, 4500 mg/L glucose, 4 mM L-glutamine, and 20 mg/ml 8-azaguanine) as described (Kennett et al., 1993. Monoclonal Antibodies: A New Dimension in Biological Analysis. Plenum Press, New York). Fused cells were plated into 24- or 48-well plates (Corning Glass Works, Corning, NY), and fed with adenine, aminopterin and thymidine containing culture media. AAT resistant cultures were screened by ELISA or flow cytometry for binding to either Nogo receptor-1-COS-7 cells or to a Nogo receptor-1 antigenic peptide as described below. Cells in the positive wells were further subcloned by limiting dilution.

To screen for antibody binding to a Nogo receptor-1 antigenic peptide, the peptides that were used as immunogens were conjugated to BSA. 0.5 µg of the conjugated peptide in 50 µl of 0.1 M sodium bicarbonate buffer, pH 9.0 was added to each well of a 96-well MaxiSorp plate (Nunc^{™}). The plate was then incubated at 37°C for 1 hour or 4°C for 16 hours and non-specific binding sites were blocked using 25 mM HEPES, pH 7.4 containing 0.1% BSA, 0.1% ovalbumin, 0.1% blotto and 0.001% azide. Hybridoma supernatant was added and incubated at 25°C for 1 hour. After washing three times with PBS, 50 µl of a 1:10,000 dilution of horseradish peroxidase-conjugated goat anti-mouse secondary antibody (Jackson ImmunoResearch Inc.) was added to each well and incubated further for 1 hour. After three washings, color was developed by TMB (Pierce) and stopped with 2 M sulphuric acid. Color intensity was monitored in a spectrophotometer at 450 nm.

Antibodies were screened for binding to full length Nogo receptor-1 as follows. COS-7 cells were labeled with 0.1 uM CellTracker^{™} Green CMFDA^{I} (Molecular Probes, Eugene, OR) as described by the vendor. Equal volumes of CellTracker^{™} labeled control cells were mixed with washed Nogo receptor-1-COS-7 cells before incubation with anti-Nogo receptor-1 test sera. Fifty microliters of the cell mixture was dispensed into each well of a 96-well V-bottom polystyrene plates (Costar^{®} 3877, Corning, NY) and 100 µl of hybridoma supernatant or a control anti-Nogo receptor-1 antibody was added. After incubation at 4°C for 30 minutes, the cells were washed and incubated with 50 µl of R-phycoerythrin-conjugated affinity pure F(ab')2 fragment goat anti-mouse IgG Fc gamma specific second antibody (1:200, Jackson ImmunoResearch Laboratory, West Grove, PA) in PBS. At the end of the incubation, the cells were washed twice with PBS and suspended in 200 µl of PBS containing 1% FBS, and subjected to FACS analyses. Alternately, Nogo receptor-I-COS-7 cells were mixed with hybridoma supernatant and then treated with R-phycoerythrin-conjugated goat anti-mouse secondary antibody and directly subjected to standard FACS analyses.

We generated 25 anti-Nogo receptor-1 antibodies using a variety of immunogens. We generated two antibodies, 7E11 and 5B10, using a peptide sequence corresponding to rat Nogo receptor-1 residues 110-125 as the immunogen. We generated three antibodies, 1H2, 3G5 and 2F7, using membranes prepared from COS7 cells transfected with full length rat Nogo receptor-1 as the immunogen. We generated 13 antibodies using sNogoR310-Fc as the immunogen (1D9.3, 1E4.7, 1B4.3, 2C4.3, 1F10.3, 2H1.4, 1H3.3, 1G4.1, 1E4.1, 2G7.1, 2C4.1, 2F11.1, and 1H4.1) and 7 antibodies using a peptide sequence corresponding to rat Nogo receptor-1 residues 423-434 as the immunogen (2E8.1, 2G11.2, and 1B5.1).

### Sequence Analysis of Monoclonal Antibodies 7E11 and 5B10

We extracted total RNA using Qiagen^{®} RNeasy^{®} mini kit, and generated cDNA from the isolated RNA. We amplified the light chain sequence by PCR using primers 5'-TGAGGAGACGGTGACCGTGGTCCCTTGGCCCCAG-3' (SEQ ID NO: 12) and 5'-AGGTSMARCTGCAGSAGTCWGG-3' (SEQ ID NO: 25). We amplified the heavy chain sequence by PCR using primers 5'-GGGGATATCCACCATGAAGTTGCCTGTTAGGCTGTTG-3' (SEQ ID NO: 13) and 5'-GGGGATATCCACCATGAGGKCCCCWGCTCAGYTYCTKGGA-3' (SEQ ID NO: 14). These primers comprise degenerate nucleotides as follows: S represents G or C; M represents A or C, R represents G or A; W represents A or T; K represents G or T; and Y represents T or C. We cloned the PCR fragments into a sequencing vector and determined the DNA sequence of the CDRs by dideoxychain termination using primers specific for the sequencing vector. We conceptually translated the DNA sequences and partial amino acid sequences of the CDR regions of the heavy of light chains of the monoclonal antibodies 7E11 and 5B10 are shown in Table 2. the 3 CDRs from the heavy and light chains of the mAbs are underlined in Table 2. The light chains of 7E11 and 5B10 are have 94% amino acid sequence identity and the heavy chains have 91% amino acid sequence identity. mAbs 7E11, 5B10, and 1H2 are of the IgG1 isotype and mAbs 3G5 and 2F7 are of the IgG2a isotype. Each of these five mAbs has a light chain of the kappa isotype. We analyze the sequence of the other monoclonal antibodies by this approach.

**Table 2. Amino Acid Sequence of mAbs 7E11 and 5B10**

| | Sequence | SEQ ID NO: |
|---|---|---|
| 7E11 Light Chain | | 15 |
| 5B10 Light Chain | | 16 |
| 7E11 Heavy Chain | | 17 |
| 5B10 Heavy Chain | | 18 |

### Inhibition of Ligand Binding to Soluble Nogo receptor-1 by Monoclonal anti-Nogo receptor-1 antibody

We tested the anti-Nogo receptor-1 monoclonal antibodies produced as described above to determine whether they inhibited ligand binding to Nogo receptor-1.

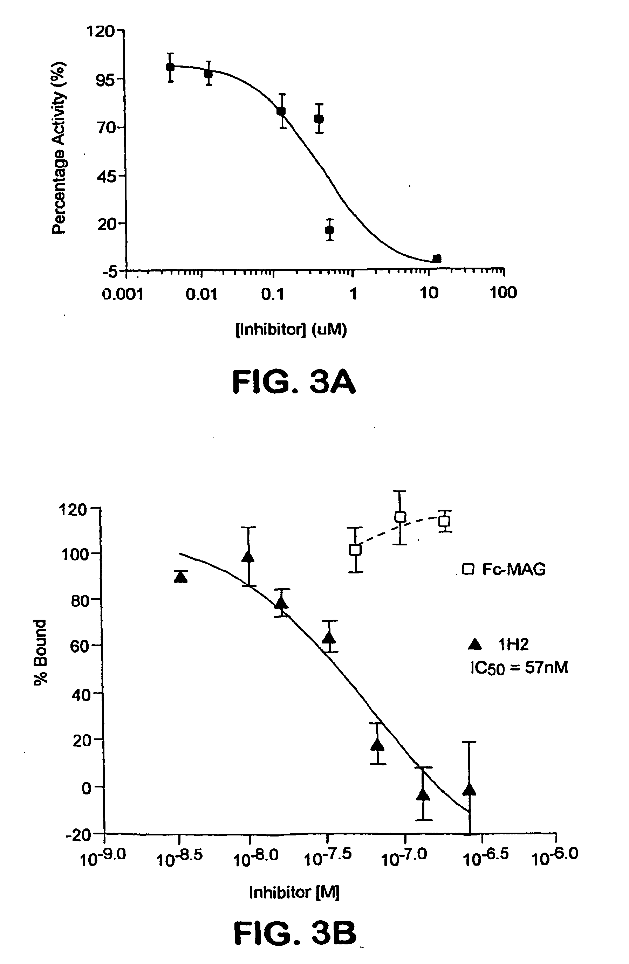

0.5 µg of a soluble Nogo receptor-1 fusion protein comprising amino acid residues 26-344 of rat Nogo receptor-1 and the hinge and Fc region of the rat IgG1 molecule (sNogoR344-Fc) produced as described below was immobilized on 250 µg of protein-A- or wheatgerm agglutinin-conjugated SPA beads (Amersham Pharmacia Biotech) for 2 hours at 25°C. SPA beads coupled with Fc-sNogoR-1 **[WHICH sNOGO IS THIS?]**, anti-Nogo receptor-1 mAb and 1µl ¹²⁵I-Nogo66 (Amersham, 2000 Ci/mmol, 1nM) in 50 µl of the HEPES-buffered incubation medium (10 mM HEPES, pH 7.4, 0.1% bovine serum albumin, 0.1% ovalbumin, 2 mM MgCl₂, 2 mM CaCl₂ and protease inhibitors) was added to each sample well. After 16 hours, radioactivity was measured in quadruplicate samples using a TopCount^{®} (Packard). IC₅₀ values were calculated from a curve-fit analysis (Fig. 3) (PRISM, GraphPad Software, NJ). In some experiments, we also used AP-ligand conjugates (e.g. AP-Nogo66) and detected binding by monitoring alkaline phosphatase activity. We also assayed the ability of the mAbs to block binding of the ligands MAG-Fc and AP-OM-gp to Nogo receptor-1.

Monoclonal antibodies 7E11, 5B10, 1H2, 3G5 and 2F7 all inhibited binding of Nogo66, MAG and OM-gp to sNogoR344-Fc. The calculated IC₅₀ for Nogo66 for 7E11 and 1H2 were 400 nM and 60 nM, respectively. The data from ELISAs monitoring mAb-mediated inhibition of binding of the three ligands to Nogo receptor-1 are summarized in Table 3.

**Table 3. mAbs Inhibit Binding of Nogo66, MAG and OM-gp to Nogo receptor-1.**

| mAb | MAG + sNogoR344-Fc | Nogo66 + sNogoR344-FC | OM-gp + sNogoR344-Fc |
|---|---|---|---|
| 7E11 | 30 nM (60%) | EC₅₀ = 1.7 µM | EC₅₀ = 150 nM |
| | EC₅₀ = 0.5 µM | | |
| 1H2 | 30 nM (60%) | ND | ND |
| 3G5 | 30 nM (60%) | EC₅₀ = 9 nM | ND |
| 2F7 | 30 nM (55%) | EC₅₀ = 10 nM | EC₅₀ = 5 nM |
| 1D9.3 | 30 nM (70%) | EC₅₀ = 13 nM | EC₅₀ = 5.2 nM |
| | EC₅₀ = 2.7 nM | | |
| 2G7.1 | 30 nM (84%) | EC₅₀ = 18 nM | EC₅₀ = 1 nM |
| 1E4.1 | 30 nM (75%) | - | EC₅₀ = 9.1 nM |
| | EC₅₀ = 2.8 nM | | |
| 1G4.1 | 30 nM (90%) | - | EC₅₀ = 8.2 nM |
| | EC₅₀ = 9.9 nM | | |
| 2C4.1 | 30 nM (50%) | - | ND |
| 2F11.1 | 30 nM (45%) | ND | ND |
| 1H4.1 | - | ND | ND |
| 2E8.1 | 30 nM (87%) | EC₅₀ = 1.5 nM | EC₅₀ = 42.9 nM |
| | EC₅₀ = 9.2 nM | | |
| 2G11.2 | 30 nM (80%) | ND | ND |
| 1B5.1 | 30 nM (0%) | ND | ND |

The percent displacement is shown at 30 nM antibody and the EC₅₀ for certain mAbs determined from curve-fit analysis as described. "-" indicates no detectable activity and "ND" indicates not determined.

### EXAMPLE 2

### Production of Fab-Phage Anti-Nogo Receptor-1 Antibodies

Anti-Nogo receptor-1 Fab-phage antibodies that specifically bind an immunogenic Nogo receptor-1 polypeptide of the invention were also made by screening a Fab-phage library as follows.

The MorphoSys Fab-phage library HuCAL^{®} GOLD was screened against recombinant rat soluble sNogoR310-Fc protein and COS7 cells expressing rat Nogo receptor-1. Fab-phages that specifically bound to Nogo receptor-1 were purified and characterized. The heavy chain of 14D5 is derived from the V_{H}2 gene and the light chain is derived from the V_{K}1 gene. The amino acid sequences of the CDRs of the heavy chain and light chain of one of these Fab-phages, 14D5, are shown in Table 4.

**Table 4. Amino Acid Sequence of CDRs of 14D5**

| | Amino Acid Sequence | SEQ ID NO: |
|---|---|---|
| Heavy Chain CDR1 | GFSLSTSGGSVG | 19 |
| Heavy Chain CDR2 | LIYSNDTKYYSTSLKT | 20 |
| Heavy Chain CDR3 | SRFWTGEYDV | 21 |
| Light Chain CDR1 | RASQNIAITLN | 22 |
| Light Chain CDR2 | LASSLQS | 23 |
| Light Chain CDR3 | QQYDNYPL | 24 |

14D5 binds to rat Nogo receptor-1 in both monovalent and bivalent forms. In addition, 14D5 binds to mouse and human Nogo receptor-1 and human Nogo receptor-2 but not mouse Nogo receptor-3.

### EXAMPLE 3

### Immunoprecipitation of Nogo receptor-1 by Anti- Nogo receptor-1 Monoclonal Antibodies

To perform the immunoprecipitation, 100 µl lysed cells or 50 µl PiPLC treated cells were mixed with 400 or 450 µl extraction buffer [10 mM Tris-HCl, pH 7.2, 0.5% Tween-20^{™}, 0.2 mM PMSF] or RIPA buffer, respectively in the presence of 30 µl Protein A or G and 1-2 µg antibody. The mixture was incubated in a shaker at 4°C for 16 hours.

Samples were spun gently to pellet the protein A or G coupled beads. The beads were washed three times with 1 ml wash buffer (10 mM Tris-HCl, pH 7.2, 0.1% Tween-20^{™}). The final wash was performed using 10% of original wash buffer.

Beads were resuspended in 100 µl of 2X SDS with 10% beta-mercaptoethanol. Samples were incubated at room temperature before being run on a 4-20% Tris-Glycine gel for SDS-PAGE. As determined by SDS-PAGE gel analysis, monoclonal antibodies, 3G5 and 2F7, immunoprecipitate Nogo receptor-1.

### EXAMPLE 4

### Determining Antibody Specificity by ELISA

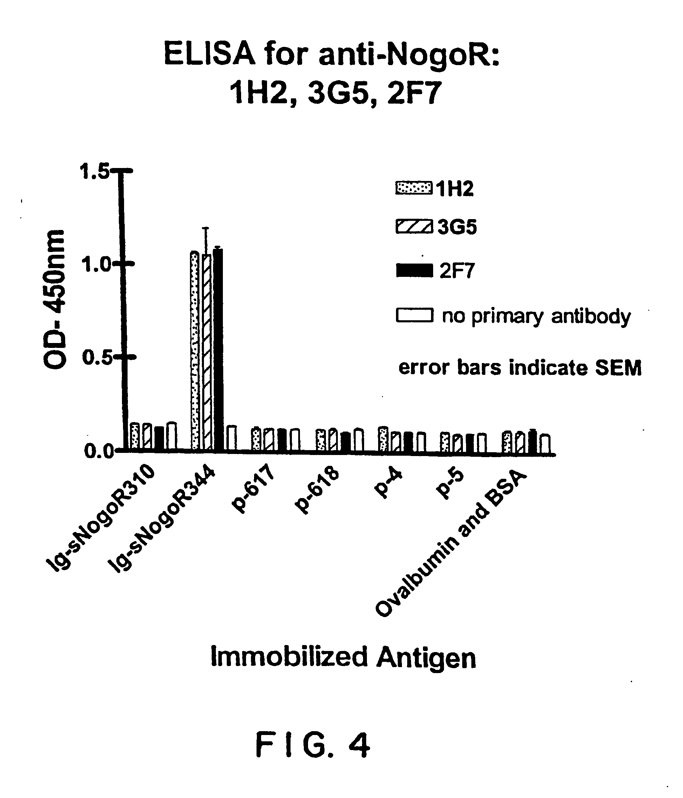

To determine the specificity of the monoclonal and Fab-phage antibodies produced in Examples 1 and 2, we performed an ELISA using a panel of Nogo receptor-1 polypeptides. The panel consisted of sNogoR310-Fc (a fusion protein comprising amino acids 26-310 of rat Nogo receptor-1 and a rat Fc fragment), sNogoR344-Fc (see supra), polypeptide p-617 (SEQ ID NO: 1), polypeptide p-618 (a 19-amino acid polypeptide from the LRR7 region of rat Nogo receptor-1; Fig. 2; SEQ ID NO: 11) and polypeptides p-4 and p-5 (polypeptides from the LRR5 and LRRCT regions of Nogo receptor-1, respectively). Ovalbumin and BSA were used as controls. As shown in Fig. 4, mAbs 1H2, 3G5 and 2F7 all specifically bound to sNogoR344-Fc. In similar experiments, those antibodies also specifically bound a polypeptide consisting of amino acids 310-344 of rat Nogo receptor-1 (SEQ ID NO: 3) and mAbs 7E11 and 5B10 specifically bound polypeptide p-617 (SEQ ID NO: 1).

Ten of the antibodies (1D9.3, 1E4.7, 1B4.3, 2C4.3, 1F10.3, 2H1.4, 1H3.3, 1G4.1, 1E4.1, and 2G7.1) from the sNogoR310-Fc immunization diplaced each other for binding, indicating that they recognize a similar or overlapping epitopes on sNogoR310-Fc. The other three antibodies from the sNogoR310-Fc immunization (2C4.1, 2F11.1, and 1H4.1) recognize different epitopes located in amino acid residues 26-310.

We also performed ELISA binding assays using the Fab-phage 14D5. Where AP-Nogo66, AP-OM-gp and MAG-Fc ligands were allowed to bind to immobilized sNogoR344-Fc, 1 µM 14D5 completely inhibited Nogo and MAG binding. 10 µM of 14D5 was required to completely inhibit the binding of OM-gp to sNogoR344-Fc.

### EXAMPLE 5

### Neurite Outgrowth Assay

To test the ability of the monoclonal and Fab-phage antibodies produced above to lessen the inhibitory effect of CNS myelin on neurons, Lab-Tek^{®} culture slides (4 wells) were coated with 0.1 mg/ml poly-D-lysine (Sigma^{®}). CNS myelin or PBS was spotted as 3 µl drops. Fluorescent microspheres (Polysciences) were added to the myelin/PBS to allow later identification of the drops (Grandpre et al, Nature 403, 2000). Lab-Tek^{®} slides were then rinsed and coated with 10 µg/ml laminin (Gibco^{™}). Dorsal root ganglions (DRG's) from P3-4 Sprague Dawley rat pups were dissociated with 1 mg/ml collagenase type 1 (Worthington), triturated with fire-polished Pasteur pipettes pre-plated to enrich in neuronal cells and finally plated at 23,000 cells/well on the pre-coated Lab-Tek^{®} culture slides. The culture medium was F12 containing 5% heat inactivated donor horse serum, 5% heat inactivated fetal bovine serum and 50 ng/ml mNGF and incubated at 37°C and 5% CO₂ for 6 hours. Fifteen µg/ml of mAb 7E11 was added immediately after plating.

Slides were fixed for 20 minutes with 4% paraformaldehyde containing 20% sucrose and stained for the neuronal marker anti beta-III-tubulin (Covance TUJ1) diluted 1:500. As secondary antibody anti-mouse Alexa Fluo^{®} 594 (Molecular Probes) was diluted 1:300 and slides were coverslipped with Gel/Mount^{™} (Biømeda^{™}). 5x digital images were acquired with OpenLab^{™} software and analysed by using the MetaMorph^{®} software for quantification of neurite outgrowth.

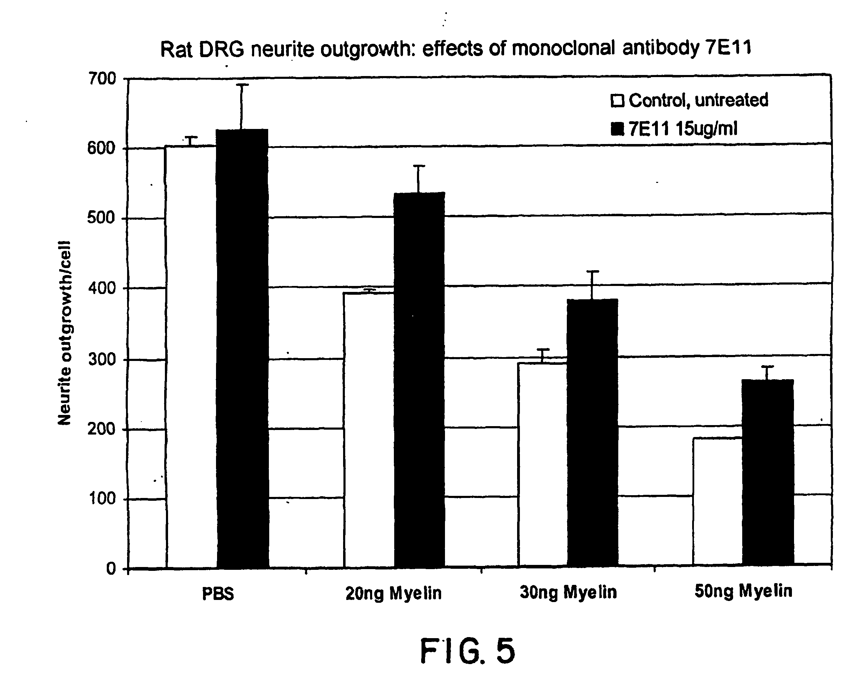

MAb 7E11 protected DRG neurons from myelin-mediated inhibition of neurite outgrowth. (Fig. 5). Similar results were observed with mAbs 1H2 and 3G5.

In a neurite outgrowth protection assay where rat P7 DRG neurons were cultured on a CNS myelin substrate, bivalent 14D5 also efficiently promoted neurite outgrowth.

### EXAMPLE 6

### Immunohistochemistry with 7E11 on Cells Transfected with Nogo Receptor-1

To further characterize the binding properties of anti-Nogo receptor-1 mAbs produced as described in Example 1, we compared binding to both fixed and live COS-7 or 293 cells expressing rat or human Nogo receptor-1.

### Fixed cells:

Nogo receptor-1 transfected and non-transfected cells were plated in 8-well Lab-Tek^{®} culture slides, fixed with 4% paraformaldehyde for 15 minutes, blocked with 10% normal goat serum, 0.1% Triton X-100 in PBS for 1 hour. Mab 7E11 was added at 15 µg/ml and 1.5 µg/ml in blocking solution and incubated for 2 hours at room temperature; Alexa^{®}-conjugated secondary antibody anti-mouse (Molecular Probes) was incubated at a 1:300 dilution in blocking solution for 1 hour; DAPI was added at 5 µg/ml to the secondary antibody to label all nuclei.

### Live cells:

Transfected and non-transfected cells were plated in 8 well Lab-Tek^{®} culture slides, blocked with FACS buffer (containing 4% donor horse serum) for 30 minutes at 4°C, incubated with 7E11 at 15 µg/ml and 1.5 µg/ml in FACS buffer for 1 hour at 4°C, rinsed and incubated with secondary antibody anti-mouse-Alexa^{®} (1:300 in FACS buffer) for 30 minutes at 4°C.

Immunohistochemical staining experiments demonstrated that all of the mAbs bound cells expressing rat Nogo receptor-1. mAbs 7E11, 2G7.1 and 2C4.1 bound both fixed and live cells expressing human Nogo receptor-1.

### EXAMPLE 7

### Mouse Model of Spinal Cord Contusive Injury

To test the effect of anti-Nogo receptor-1 mAbs produced in Example 1 on neurons in vivo, we use a mouse spinal cord contusion injury model.

Female mice (18-22 g) are treated prophylactically with analgesic and antibiotic agents. Mice are anesthetized and placed in a stereotaxic apparatus with vertebral column fixation under a stereomicroscope. Trauma to the spinal cord is introduced by a modified version of the weight-drop method (M. Li et al., Functional role and therapeutic implications of neuronal caspase-1 and -3 in a mouse model of traumatic spinal cord injury. Neuroscience Vol. 99, pp. 333-342, 2000).

Briefly, a T9 and T10 laminectomy is made and the vertebral column is stabilized using a pair of mouse transverse clamps supporting the T9-T10 transverse processes bilaterally. A stainless steel impact rod with a diameter of 1.4 mm and weight of 2 g, is raised 2.5 cm above the dura and dropped onto the spinal cord at the T10 level. During the surgery, mice are kept on a 37°C warming blanket and 1 ml of warmed sterile saline is administered subcutaneously to each mouse after surgery to avoid dehydration. The bladder is manually expressed once daily until reflexive bladder control is regained.

All animals receive post-operative analgesia every 8-12 hours after surgery and antibiotic treatment twice daily for 7 days thereafter. Animals have free access to food and water for the duration of the study. Anti-Nogo receptor-1 antibodies are delivered to the injury site via intrathecal injection for 28 days as described in the rat spinal cord transection model below.

### EXAMPLE 8

### Characterization of Soluble Nogo receptor-1 Fusion Proteins

To characterize soluble Nogo receptor-1 polypeptides (sNogoR-1) and fusion proteins (Fc-sNogoR-1) we performed the following experiment.

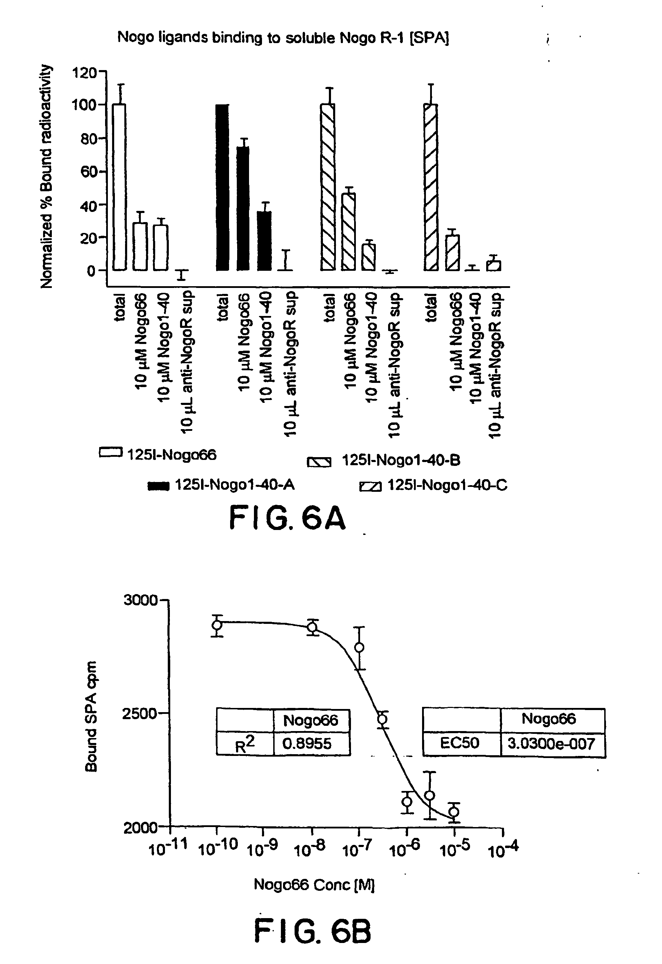

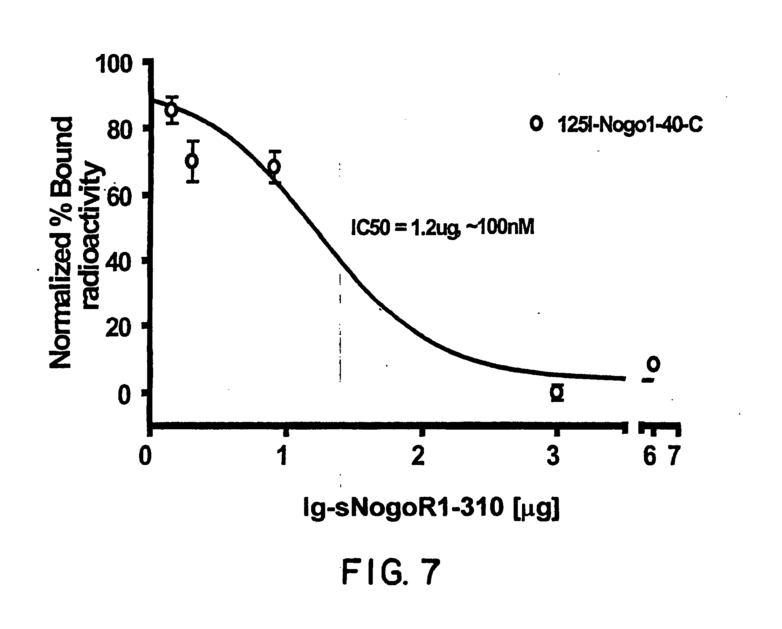

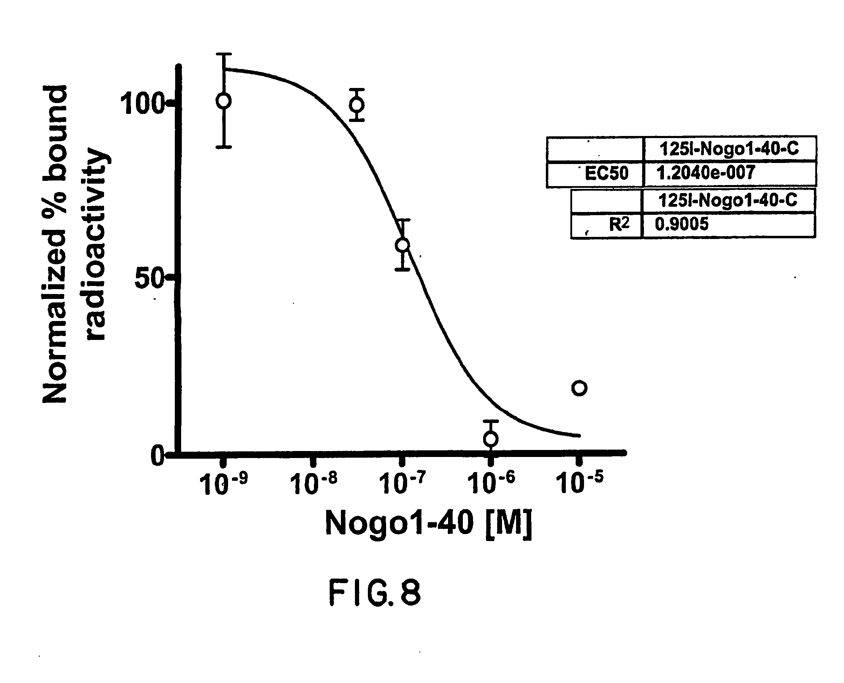

Three µg of soluble Nogo receptors (sNogoR310-Fc and sNogoR344-Fc) were immobilized on 250 µg WGA-SPA beads and received 0.5 µL of radioactive ligand (final concentration 0.5 nM) in a final volume of 100 µL of binding buffer (20 mM HEPES, pH 7.4, 2 mM Ca, 2 mM Mg, 0.1% BSA, 0.1% ovalubmin and protease inhibitors). Ligands included 10 µM Nogo66, 10 µM ¹²⁵I-Nogo40 (amino acids 1-40 of NogoA) and 10 µL of anti-Nogo receptor-1 antibody supernatant for each ligand set. The three tyrosines on Nogo40 were separately iodinated and designated as Nogo40-A,-B and -C respectively. Mean values of triplicates are presented as normalized % bound radioactivity (Figs. 6, 7 and 8). Error bars indicate SEM. Bound radioactivity in the absence of inhibitors was taken as 100% and the lowest bound radioactivity in the presence of 10 µM Nogo40 was taken as the 0% for data normalization.

### EXAMPLE 9

### Inhibition of Ligand Binding to Soluble Nogo receptor-1 Fusion Protein

A binding assay similar to the binding assay of Example 8 was used to test the ability of two mAbs produced in Example 1 to inhibit ¹²⁵I-Nogo66 binding to sNogoR344-Fc. Mabs 2F7 and 3G5 inhibited ¹²⁵I-Nogo66 binding to sNogoR344-Fc.

### EXAMPLE 10

### Neurite Outgrowth Assay

Lab-Tek^{®} culture slides (4 wells) were coated with 0.1 mg/ml poly-D-lysine (Sigma^{®}). CNS myelin alone or mixed with sNogoR310, sNogoR310-Fc fusion protein, mAb 5B10 or control PBS were separately spotted as 3µl drops. Fluorescent microspheres (Polysciences) were added to the myelin/PBS to allow later identification of the drops (Grandpre et al, Nature 403, 2000). Lab-Tek^{®} slides were then rinsed and coated with 10 µg/ml laminin (Gibco^{™}).

Dorsal root ganglions (DRG's) from P3-4 Sprague Dawley rat pups were dissociated with 1 mg/ml collagenase type 1 (Worthington), triturated with fire-polished Pasteur pipettes pre-plated to enrich in neuronal cells and finally plated at 23,000 cells/well on the pre-coated Labtek culture slides. The culture medium was F12 containing 5% heat inactivated donor horse serum, 5% heat inactivated fetal bovine serum and 50 ng/ml mNGF and incubated at 37°C and 5% CO₂ for 6 hours.

Slides were fixed for 20 minutes with 4% paraformaldehyde containing 20% sucrose and stained for the neuronal marker anti beta-III-tubulin (Covance TUJ1) diluted 1:500. As secondary antibody anti-mouse Alexa Fluor^{®} 594 (Molecular Probes) was diluted 1:300 and slides were coverslipped with Gel/Mount^{™} (Biømeda^{™}). 5x digital images were acquired with OpenLab^{™} software and analyzed by using the MetaMorph^{®} software for quantification of neurite outgrowth.

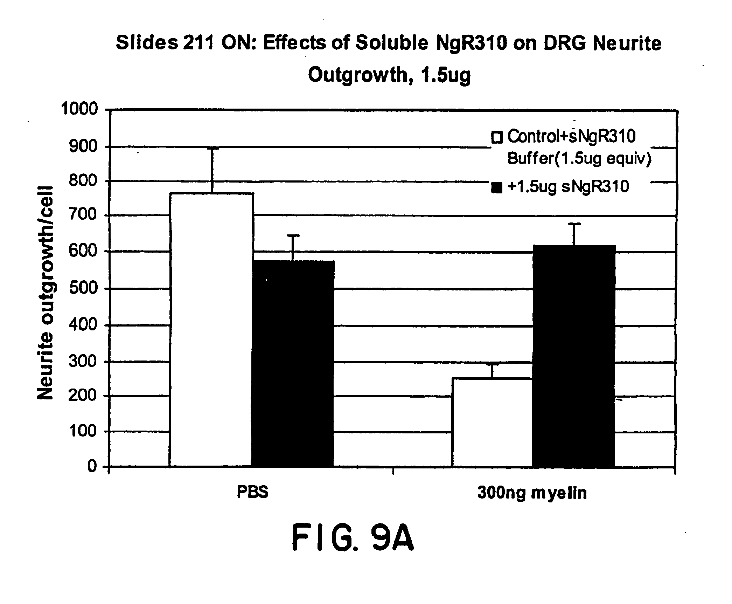

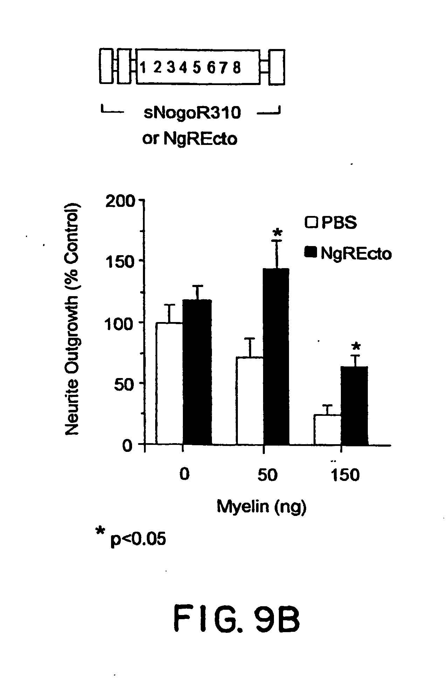

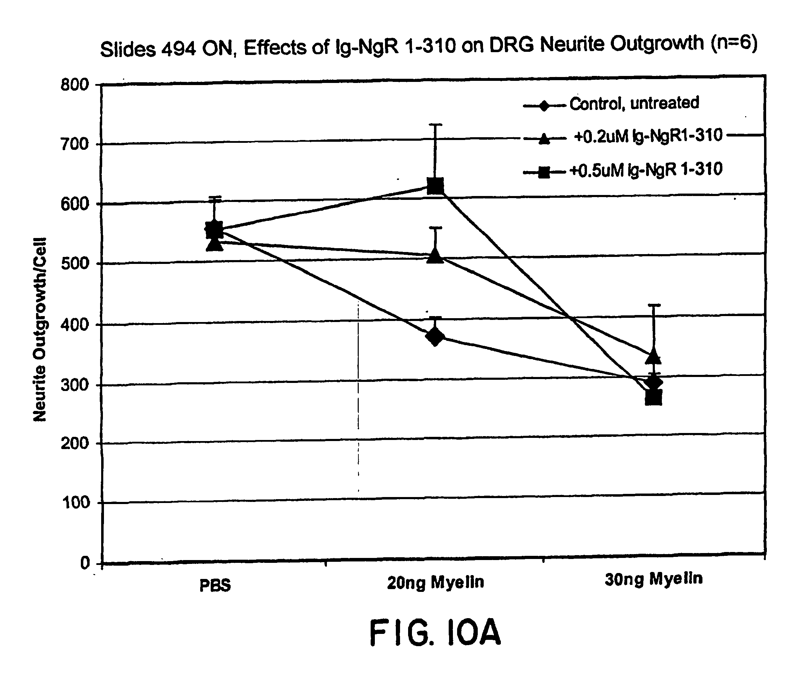

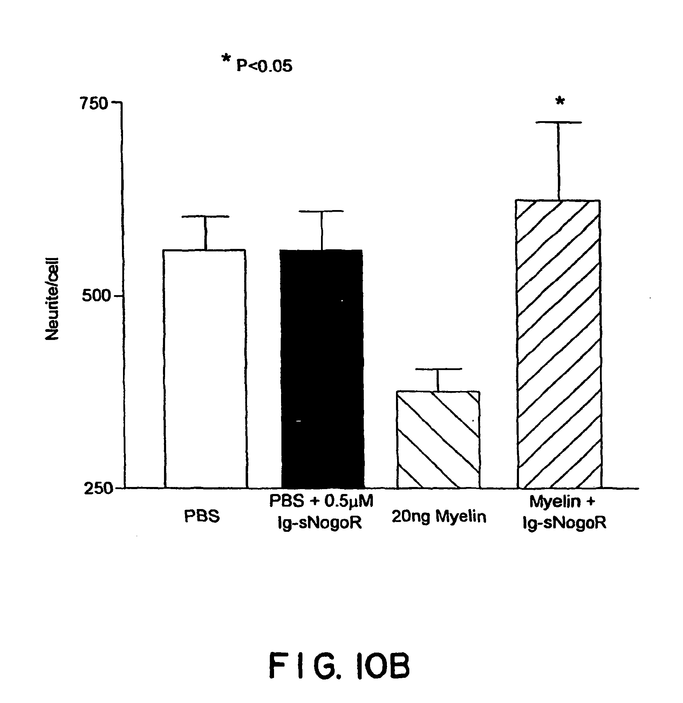

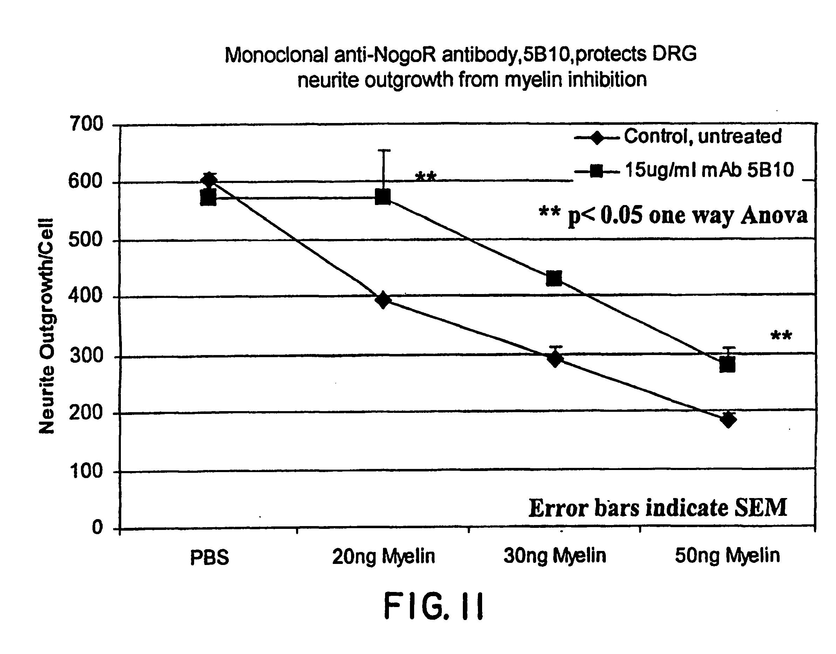

sNogoR310, sNogoR310-Fc and mAb 5B10 all protected DRG neurons from myelin-mediated inhibition of neurite outgrowth (Figs. 9-11). sNogoR310 was used in a similar assay using chick neurons and was found to be protective.

We also tested the neuro-protective effect of soluble Nogo receptors by performing experiments with cells grown in the presence and absence of laminin. Neuronal cell growth in media without laminin is poor and models neuronal stress conditions.

DRG's were dissected from post-natal day 6-7 rat pups (P6-7), dissociated into single cells and plated on 96-well plates pre-coated with poly-D-lysine as described above. In some wells 2 µg/ml laminin was added for 2-3 hours and rinsed before the cells were plated. After an 18-20 h incubation the plates were fixed with 4% paraformaldehyde, stained with rabbit anti-Beta-III-tubulin antibody diluted 1:500 (Covance^{®}) and anti-HuC/D diluted 1:100 (Molecular Probes), and fluorescent secondary antibodies (Molecular Probes) were added at 1:200 dilution. The ArrayScan^{®} II (Cellomics^{®}) was used to capture 5x digital images and to quantify neurite outgrowth as average neurite outgrowth/neuron per well, by using the Neurite outgrowth application. Nine 5x images from 3 wells/condition were analyzed.

In some experiments, a sub-clone of PC12 cells (Neuroscreen^{™}) was used (Cellomics^{®}). The Neuroscreen^{™} cells were pre-differentiated for 7 days with 200 ng/ml NGF, detached and replated on 96-well plates pre-coated with poly-D-lysine. In some wells 5 µg/ml laminin was added for 2-3 hours and rinsed before the cells were plated. After 2 days incubation the plates were fixed with 4% paraformaldehyde, stained with rabbit anti-Beta-III-tubulin antibody diluted 1:500 (Covance^{®}) and Hoechst (nuclear stain). The ArrayScan^{®} II was used to quantify neurite outgrowth as in the DRG cells.

sNogoR344-Fc or rat IgG were added in solution to P6-7 DRG neurons and to differentiated Neuroscreen^{™} cells at the time of plating.

The neuro-protective effect of sNogoR344-Fc was observed at 1 µM and 10 µM when P6 DRG neurons were grown in the absence of laminin. Quantification of neurite outgrowth showed a dose-dependent increase with the addition of sNogoR344-Fc. Addition of sNogoR344-Fc at the same concentrations to DRG neurons growing on a laminin substrate, did not produce any unusual effect, indicating that sNogoR344-Fc is only active on stressed cells. The neuro-protective effect of sNogoR344-Fc at the same concentrations in the absence of laminin also was seen with Neuroscreen cells.

### EXAMPLE 11

### Production and Purification of Fc-sNogoR-1 Fusion Protein

A cDNA construct encoding amino acids 1-310 of rat Nogo receptor-1 was fused to rat IgG1 Fc contained in a mammalian expression vector and this vector was electroporated into Chinese hamster ovary (CHO) (DG44) cells. Cells were maintained in alpha-MEM, supplemented with 10% dialyzed fetal bovine serum, 2 mM glutamine and antibiotic-antimycotic reagents. Two days after transfection, the conditioned media was collected and analyzed by Western blot under reducing conditions. A protein band about 60 kDa was detected using a polyclonal rabbit anti-Nogo receptor-1 antibody. Cells were expanded and sorted using a R-PE conjugated goat anti-rat IgG antibody. After the second sorting, cells were plated at a density of one cell/well in 96-well plates. Secreted soluble Nogo receptor-1 protein levels from individual wells was tested and compared using a Sandwich ELISA. ELISA plate was coated with goat anti-rat IgG Fcκ specific antibody. Conditioned media was applied. The bound soluble Nogo receptor-1 protein was detected by HRP conjugated donkey anti-rat IgG Fab, Fc-specific antibody. Clone 4C12 had the highest secretion level. 4C12 was expanded and grown in CHO-M7 media in spinner flask. The secretion level was about 10 mg/L at 37°C.

CHO cells expressing the sNogoR310-Fc fusion protein were cultured in large scale. 1.7 L of concentrated conditioned media was obtained from a 10 L bioreactor run. The pH was raised by addition of one-tenth volume 1.0 M Tris-HCl, pH 8.9. Solid sodium chloride and glycine were added to 3.0 M and 1.5 M respectively. A 60 mL protein A-Sepharose^{™} column equilibrated with 10 mM Tris-HCl, 3 M sodium chloride, 1.5 M glycine, pH 8.9 was prepared. Concentrated conditioned media was applied to the column at 1.5 mL/min using a peristaltic pump. The column was washed with 300 mL of 10 mM Tris-HCl, 3 M sodium chloride, 1.5 M glycine, pH 8.9 followed with 120 mL 5 mM Tris-HCl, 3 M sodium chloride, pH 8.9. Protein was eluted with 25 mM sodium phosphate, 100 mM sodium chloride, pH 2.8. 10 mL fractions were collected in tubes containing 1.0 mL of 1.0 M HEPES, pH 8.5. Protein fractions were pooled and dialyzed against 3 x 2 L of 5 mM sodium phosphate, 300 mM NaCl, pH 7.4.

### EXAMPLE 12

### Spinal Cord Transection Assay

To test their ability to promote functional recovery *in vivo*, an sNogoR-1 fusion protein was tested in a rat spinal cord transection assay.

Alzet^{®} osmotic pumps were loaded with test solution (sNogoR310-Fc in PBS) made up freshly on the day of use. The loading concentration was calculated to be 5 and 50 µM. Pumps were primed for >40 hours at 37°C prior to implantation into animals. Female Long Evans rats were given pre-operative analgesia and tranquilizer and anesthetized using isoflurane (3% in O₂).

Rats were placed in a stereotaxic frame and the motor cortex exposed for infusion of the tract tracing agent BDA (10,000 MW) bilaterally. Rats then underwent dorsal hemisection of the spinal cord at T5-T6 followed by implantation of the intrathecal catheter and pump system to deliver test compound (n=11 per group).

Rats were allowed to recover and survive up to 28 days after surgery. Behavioral scoring using the BBB system was recorded up to 28 days after induction of injury, just prior to termination of the in-life phase of the study. Following perfusion and fixation, spinal cords were removed, cryoprotected, sectioned, stained and axonal counts performed.

The Basso-Beattie-Bresnahan (BBB) locomotor rating scale (Basso et al., 1996, Neurotrauma 13, 343-359), the inclined plane test and the inclined grid walking test (Li and Strittmatter, 2003, J Neurosci. 2003, 23, 4219-27) were monitored in rats and mice after injury. For the inclined plane test, we measured the maximal angle to which a 50 cm x 60 cm board could be angled for 5 sec without the mouse sliding off. For inclined grid walking, the mice were trained to climb a wire grid (35 cm long with 2.54 cm squares) at a slope of 45 degrees. The number of instances in which the hindpaw dropped below the grid plane was scored for each excursion from bottom to top. For the rat behavioral testing, BBB locomotor scale, grid walking and footprint analysis were performed. For grid walking, the rats were trained to walk on a wire grid (70 cm long with 2.54 cm squares), and the number of instances in which the hindpaw dropped below the grid plane was counted. For footprint analysis, the walking patterns of rat hindpaws were recorded with ink during a continuous locomotion across a 90 cm runway, and stride length on each side and stride width were calculated (Metz et al., 2000, Brain Res., 883, 165-177). All of these behavioral tests were performed by at least two individuals. Throughout the surgery, behavioral testing and histologic analysis, researchers were blind to the identity of the compound in the minipump.

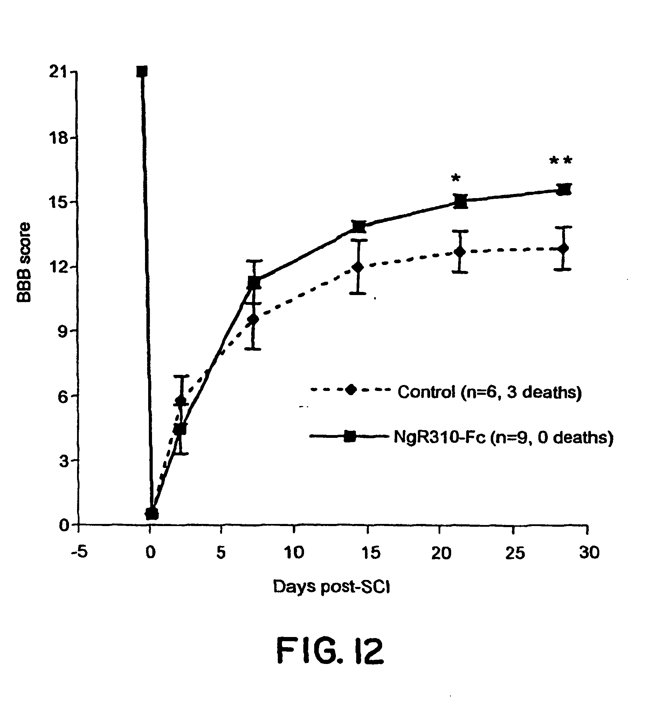

sNogoR310-Fc promoted functional recovery (Fig. 12).

### EXAMPLE 13

### Rat Spinal Cord Contusion Assay

The effect of soluble Nogo receptor-1 polypeptides and fusion proteins on neurons *in vivo* are tested in a rat spinal cord contusion assay.

Female hooded Long Evans rats (170-190 g) are treated prophylactically with analgesic and antibiotic agents. Ten minutes before surgery, animals are tranquilized with 2.5 mg/kg Midazolam i.p. and anesthetized in 2-3% isoflurane in O₂. Rats are then shaved, wiped down with alcohol and betadine, and ocular lubricant applied to their eyes. Next, an incision is made down the midline and the T7 to T12 vertebrae exposed.

A dorsal laminectomy is performed at T9 1/2 and T10 to expose the cord. The rat is mounted on the Impactor. T7 and T8 segments are first clamped and then the T11 and T12 segments are attached to the caudal clamp. A soft material is placed underneath the chest of the rat. The Impactor rod is set to the zero position and the electrical ground clip is attached to the wound edge. The Impactor rod is then raised to 25.0 mm and appropriately adjusted to a position directly above the exposed spinal cord. Next, the Impactor rod is released to hit the exposed cord and the Impactor rod is immediately lifted.

The rat is then dismounted, and Gelfoam^{®} placed on the wound. The muscle over the wound is sutured, and the incision is surgically stapled. Animals are placed in an incubator until they recover from anesthesia. Rats are given antibiotics, analgesics, and saline as required. Bladders are expressed every morning and evening thereafter until function is recovered.

Soluble Nogo receptor-1 fusion protein (e.g., sNogoR310-Fc) is administered intrathecally as described in the rat spinal cord transection model above. BBB scoring is performed one-day after surgery, then every week thereafter until 4 to 6 weeks.

### EXAMPLE 14

### Expression of sNogoR310 in Transgenic Mice

We produced transgenic mice expressing soluble Nogo receptor-1 protein to test its effect when expressed *in vivo*.

We cloned the mouse sNogoR310 cDNA (corresponding to amino acids 1-310 of the Nogo receptor-1) into the NotI site of the C-3123 vector. In this vector, sNogoR310 expression is under the control of the glial fibrillary acidic protein (gfap) gene regulatory elements, which allow high level expression with enhanced secretion from reactive astrocytes at site of injury. We digested the resulting vector sequentially with AatII and SfiI and isolated the *gfap::sNogoR310* construct on a 3.4 kb fragment. We microinjected this fragment into embryos to generate transgenic mice. We verified by PCR that the transgene had integrated and identified five founder lines. We crossed heterozygous males of the two founder lines with the highest expression levels to female C57BL/6J mice. We confirmed that the GFAP-positive cells express and secrete sNogoR310 in heterozygous transgenic mice by Western blot analysis using antibody raised against Nogo receptor-1.

We homogenized the cortex and spinal cord in Tris-buffered saline supplemented with protease inhibitors (Roche) and centrifuged the homogenate at 40,000 rpm for 20 min at 4°C. We treated the supernatant with 4% paraformaldehyde for 20 min to enhance antibody specificity and dialyzed prior to immunoblotting. We homogenized the particulate fraction by sonication in RIPA buffer (1% Triton^{®} X-100, 0.5% sodium deoxycholate, 0.1% SDS in PBS), centrifuged the resulting homogenate and treated this supernatant (detergent-soluble particulate fraction) as above. We analyzed 20 µg of brain or spinal cord protein by immunoblot using rabbit antiserum raised against Nogo receptor-1 at 1:2000 dilution. We visualized immunoreactivity by incubation with AP-conjugated anti-rabbit IgG and NBT/BCIP AP substrates.

We detected secreted 37 kDa sNogoR310 in detergent-free soluble extracts of cortex and spinal cord from the two transgenic lines Tg08 and Tg01, but little if any soluble Nogo receptor-1 protein at 37 or 81 kDa is present in littermate wild type (WT) mice. Examination of the particulate fractions demonstrated that there were comparable levels of endogenous Nogo receptor-1 in both WT and transgenic mice.

### EXAMPLE 15

### Expression of sNogoR310 in Transgenic Mice After Injury

We tested the effect of CNS injury on sNogoR310 expression in transgenic mice by performing a dorsal over-hemisection injury. We obtained sNogoR310 transgenic and nontransgenic control animals by mating heterozygous males with C57/BL6 females as described in Example 14.

We deeply anesthetized adult female heterozygous transgenic or littermate WT mice (10-16 weeks of age) and performed a complete laminectomy, fully exposing the dorsal part of spinal cord at T6 and T7 levels. We performed a dorsal over-hemisection at T6 with a 30-gauge needle and a pair of microscissors to completely sever the dorsal and dorsolateral corticospinal tracts (CSTs). We passed a marked needle across the dorsal part of the spinal cord several times to assure that the lesion was at a depth of 1.0 mm. We sutured the muscle layers over the laminectomies and closed the skin on the back with surgical staples. To trace the corticospinal tracts, we made a burr hole overlying cerebral cortex on the right side into the skull 14 days after spinal cord injury. We applied the tracer BDA (MW 10,000, 10% in PBS) (Molecular Probes, Eugene, OR) to 4 injection sites at a depth of 0.7 mm from the cortical surface. Four weeks after injury, the mice were perfused transcardially with PBS, followed by 4% paraformaldehyde. Mice used for sNogoR310 expression experiments did not receive any tracer injection.

For the mice used for western blot analysis, the spinal cord at a level between T3 and L3 was collected without perfusion 14 days after injury. Mice used for Nogo receptor-1 immunohistochemical staining were perfused with 4% paraformaldehyde 10 days after hemisection, and the injured spinal cord was removed for sectioning. To examine sNogoR310 expression in the injured brain of transgenic and WT mice, a cortex stab injury was performed with a number 11 scalpel blade held in a stereotaxic apparatus (David Kopf, Tujunga, CA). A 4 mm parasagittal cut was made, 0.5 mm posterior to Bregma, 1.5 mm laterally from midline and 3.5 mm deep.

We detected increased levels of sNogoR310 in soluble extracts of spinal cords ten days after the injury in transgenic mice but not in WT mice, consistent with the upregulation after injury of GFAP around the lesion. To confirm that this was not due to compensatory upregulation of Nogo-A, we tested its expression and found that it was similar in either intact or injured cortex and spinal cord from either WT and transgenic mice.

We examined the cellular expression of sNogoR310 in injured CNS by immunostaining the injured brain and spinal cord containing the lesion area with antibodies against Nogo receptor-1 and GFAP. The general morphology of reactive astrocytic glia does not differ between WT and transgenic mice, but the density stained for Nogo receptor-1 in both intra- and extracellular space is remarkably higher in the *gfap::sNogoR310* transgenic mice than in WT mice, indicating increased sNogoR310 expression around the lesion in transgenic mice. Nogo receptor-1 protein is co-localized with astrocytic marker GFAP only in the transgenic mice. There is also a greatly enhanced diffuse non-cellular staining in the transgenic samples, consistent with sNogoR310in the extracellular space. Neuronal cell body Nogo receptor-1 staining is detected in both WT and transgenic mice.

### EXAMPLE 16

### Secreted sNogoR310 Induces CST Sprouting in Transgenic Mice

We tested whether increased expression of sNogoR310 around the lesion in transgenic mice results in the regeneration of injured axons.

We investigated the integrity of descending corticospinal tracts (CST) by injecting anterograde tracer biotin dextran amine (BDA) into the right motor cortex as described in Li and Strittmatter, 2003, J. Neurosci., 23, 4219-27. In littermate WT mice, the prominent dorsal CST (dCST) is tightly bundled rostral to the lesion, and a few dorsolateral CST fibers are visible ipsilaterally. A small number of BDA-labeled short collateral sprouts project into gray matter, particularly in the ventral cord, but the sprouting is largely confined to the side of the cord contralateral to the tracer injection. However, the sections rostral to dorsal hemisection from injured sNogoR310 transgenic mice indicate a quite different BDA labeling pattern. A high density of BDA-labeled CST fibers are observed outside of prominent dCST in all the transgenic mice from line Tg08 or line Tg01. Ectopic fibers extend throughout the gray matter area, and some fibers reach into lateral and dorsolateral white matter. Several fibers (4-12 sprouts per transverse section) are seen on the opposite side of the spinal cord (ipsilateral to the tracer injection site). Micro densitometric measurement of the collateral sprouts indicates approximately a tenfold increase in sprouting density in sNogoR310 transgenic mice. Examination of parasagittal longitudinal sections from 1 to 4 mm rostral to the lesion reveals that dCST fibers extend a large number of branching sprouts into the ventral gray matter area in sNogoR310 transgenic mice, in contrast to the littermate WT animals. Generally, the pattern and extent of sprouting rostral to the lesion in transgenic mice are similar to those observed in the mice treated systemically with Nogo receptor-1 antagonist peptide NEP1-40 (Li and Strittmatter, 2003).

These results demonstrate that secreted sNogoR310 induces CST sprouting in the transgenic mice.

### EXAMPLE 17

### Regenerating CST Axons Bypass the Lesion Site into Distal Spinal Cord in sNogoR310 Transgenic Mice

We isolated spinal cord 4 mm rostral to and 4 mm caudal to the lesion site (8 mm long in total) from transgenic mice and embedded it in a glutaraldehyde-polymerized albumin matrix, and cut parasagittally on a vibratome (30 µm thick). We collected transverse sections (50 µm) from the spinal cord 5-7 mm rostral to and 5-7 mm caudal to the injury site. For sNogoR310-Fc injection experiments in rats, the spinal cord extending from 10 mm rostral to 10 mm caudal from the lesion site was cut parasaggitally (50 µm) on a vibrating microtome. Transverse sections were collected from the spinal cord 11-16 mm rostral to and 11-16 mm caudal to the injury site. We incubated the sections with avidin-biotin-peroxidase complex and visualized the BDA tracer by nickel-enhanced diaminobenzidine HRP reaction (Grandpre, 2002, Nature, 417, 547-551). We processed some sections for serotonin immunohistochemistry (anti-5-HT antibody) by indirect immunofluoresence. To visualize the lesion area, we double-stained some sections with antibodies directed against GFAP (Sigma^{®}, St. Louis, MO). We mounted, dehydrated and covered the sections with mounting medium.

We tested whether the fibers induced by sNogoR310 expressed in transgenic mice after injury (see Example 16) cross the lesion area into the caudal spinal cord to provide functional recovery.

Consecutive parasaggital sections across the injury site drawn in camera lucida display the overall distribution pattern of the regenerating CST fibers a few millimeters from the lesion. Sections from WT mice show no CST fibers extending beyond the injury site. Similar sections from sNogoR310 transgenic mice display numerous CST fibers that cross the transection area and project into the distal gray and white matter areas in a highly branched pattern. Immediately rostral to hemisection, a high density of BDA-labeled CST sprouting originated from prominent dCST projects into the lesion area, but most CST sprouts failed to pass the transection area where scar formation and tissue cavitation are prominent. A small but highly significant fraction of the regenerating axons bypass the lesion site through the remaining tissue bridges of the ventral and ventrolateral gray and white matter. In addition, a few CST fibers appear to cross the transection area itself via the lesioned dorsal and dorsolateral spinal cord into distal regions. In the vicinity of lesion, the course of regenerating fibers was typically tortuous and quite distinct from the normal straight fibers in the rostral CST. Collaterals and arborized fibers are most frequently seen in gray matter area of distal spinal cord. The reconstructions demonstrate 5-15 BDA-labeled regenerating fibers coursing in the rostral-caudal axis at any level 1-4 mm caudal to the lesion in each transgenic mouse. For transverse sections 5-7 mm caudal to dorsal hemisection, BDA-labeled CST axons are seen in both the gray matter and white matter areas in each transgenic mouse. The fiber counts for the transgenic mice indicate approximately a similar number of BDA-labeled CST fibers to the proximal levels in the sagittal sections.

In addition to CST fibers, the other descending tracts, such as raphespinal fibers, also contribute to locomotor function in mice. In this mouse dorsal over-hemisection model, the transection injures a majority of the serotonergic fibers, decreasing the density of these fibers by approximately 80% in the ventral horn. Analysis of total length of serotonin fibers in the ventral horn of caudal spinal cord indicates a much greater number of these fibers in transgenic mice than WT group, indicating that the growth-promoting effects of sNogoR310 in transgenic mice are not limited to one axon descending pathway.

### EXAMPLE 18

### Transgenic Expression of sNogoR310 Improves Locomotor Recovery

The CST axon tracing and serotonergic fiber analysis indicate that the sNogoR310 released from astrocytes in transgenic mice stimulates extensive anatomical regeneration of injured descending axons in the spinal cord. We performed several behavioral tests as described in Example 12 to determine whether these regenerated fibers benefit functional recovery.

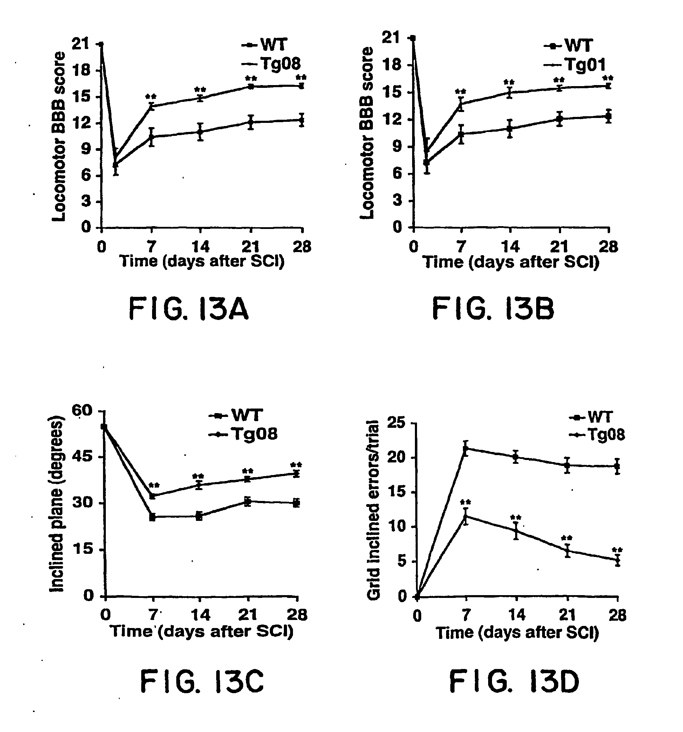

As assessed by the BBB test, the WT mice partially recover locomotor function during a 4-week period of survival. At 4 weeks post-injury, most WT mice recover a level characterized by consistent plantar stepping with consistent weight support, but they exhibit only occasional to frequent forelimb-hindlimb coordination, with a rotation of predominant paw position when making initial contact with surface. In contrast, the BBB scores of sNogoR310 transgenic mice from both lines Tg08 and Tg 01 are significantly higher than control group throughout the 7-28 day observation period (Figs. 13A and 13B). At 28 days after injury, most transgenic mice show consistent forelimb-hindlimb coordination, and the predominant paw position is parallel to the body.

We employed two more behavioral tests to further characterize the performance of sNogoR310 transgenic mice. First, we measured the maximal angle to which a board would be tilted without a mouse losing its grip within 5 sec. Before dorsal hemisection injury, both transgenic and WT mice can sustain their posture on board angled at 55 degrees. On days 7-28 after injury, the sustainable angle is reduced in all mice, but the angles sustainable by the transgenic mice are significantly greater than those for the control group (Fig. 13C). In another behavioral test, mice climbed a grid placed at a 45 degree angle to vertical and excursions of the hindlimbs below the plane of the grid were counted (Metz et al., 2000). No mice made errors on this test during the pre-injury training. There are numerous foot fault errors with only minimal improvement in WT mice during the period 2-6 weeks post-injury. In contrast, the sNogoR310 transgenic mice exhibit a progressive improvement in grid climbing during this period, with the majority of improvement occurring betweenl-3 weeks post-injury (Fig. 13D). Thus, transgenic mice secreting sNogoR310 from astrocytes exhibit CST regeneration, raphespinal sprouting and improved motor function after thoracic spinal hemisection.

### Example 19

### Intrathecal Administration of sNogoR310-Fc Protein Induces CST Sprouting

As a second test of the growth-promoting benefit of soluble Nogo receptor-1 after spinal trauma, we administered the purified protein intrathecally.

We fused the ligand binding domain (27-310) of rat Nogo receptor-1 to the rat IgG1 Fc domain to promote stability and purification. We purified protein from stably transfected CHO cells. This protein blocks Nogo-66, MAG and myelin action *in vitro*, as shown previously for mouse sNogoR310-Myc His (Fournier et al., 2002, J Neurosci., 22, 8876-8883; Liu et al., 2002, Science, 297, 1190-1193). We delived sNogoR310-Fc protein intrathecally to rats with a mid-thoracic dorsal hemisection injury through an osmotic minipump. During a four-week survival period after injury, 1.2 mg sNogoR310-Fc protein was locally administered in each rat. In rats receiving the vehicle treatment (1.2 mg rat IgG), sections rostral to hemisection display the tightly bundled prominent dorsal CST and very few ectopic BDA-labeled CST fibers above the lesion site. Sections rostral to lesion from injured rats receiving sNogoR310-Fc protein exhibit a quite different pattern of labeling. Numerous ectopic fibers sprouting from the BDA-labeled CST are observed from transverse and parasagittal sections. In some cases, projections cross from the dCST area near the midline to the circumference of the cord, becoming intermingled with the dorsolateral CST. The sprouting axons extend through gray matter to a greater extent than white matter. A measure of ectopic sprouting fibers (≥100 µm in transverse sections, ≥200 µm in sagittal sections) adjacent to the dCST reveals a greater increase in the sNogoR310-Fc-treated rats.

### Example 20

### CST Axons Regenerate into Distal Spinal Cord in sNogoR310-Fc Treated Rats

We deeply anaesthetized female Sprague-Dawley rats (190-250 g) and conducted laminectomies at spinal levels of T6-7, exposing the spinal cord. We cut the dorsal half of the spinal cord with a 30-gauge needle and a pair of microscissors to sever the dorsal parts of CSGT tracts, and assured the depth of the lesion (1.8 mm) by passing the sharp part of a number 11 blade across the dorsal half of the cord (Grandpre et al., 2002, Nature, 417, 547-551). An osmotic minipump (Alzet^{®} 2ML4, 2 ml volume, 2.5 µl/h, 28 day delivery), which was filled with 1.2 mg rat IgG in PBS or 1.2 mg sNogoR310-Fc fusion protein in PBS, was sutured to muscles under the skin on the back of the animals. A catheter connected to the outlet of the minipump was inserted into the intrathecal space of the spinal cord at the T7-8 level through a small hole in the dura.

Nogo receptor-1 antagonist protein infusion induced extensive sprouting rostral to a rat hemisection, but a more critical issue is whether the sprouting CST fibers project to distal spinal cord and contribute to locomotor recovery. Longitudinal sections across lesion site from vehicle-treated rats display no detectable or a very small number of BDA-labeled ventral CST fibers below the lesion level (GrandPre et al., 2002; Weidner et al., 2001, Proc. Natl. Acad. Sci. USA, 98, 3513-3518). The similar sections from sNogoR310-Fc treated rats demonstrate many BDA-labeled fibers bypass the transection site and project to the caudal spinal cord largely through the bridging tissues of the ventral and ventrolateral spinal cord. Immunostaining for astrocytic marker GFAP display that the extent of transection reached deeper than central canal area. Unlike the linear profile of rostral fibers in prominent dorsal CST, the regenerated CST fibers usually follow a highly branching trajectory in the distal spinal cord, particularly in gray matter area. These fibers are detected in many regions of spinal cord, but they are more easily seen in the central part and dorsal half of spinal cord throughout the spinal cord. Counts of CST fibers from sagittal sections indicate approximately 20 BDA-labeled axons at 1-2 mm caudal to lesion and 15 traced axons at 7-8 mm distal to lesion from each sNogoR310-Fc-treated rat.

Generally, the branching pattern of these fibers is similar to that observed from local NEP 1-40 peptide treated animals, but more collateral branching in each sprout is seen from the sections treated with sNogoR310-Fc protein. A measure of the sprouts from distal spinal cord demonstrates that the total collateral length of each sprout in sNogoR310-Fc-treated rats is twice as great as that from NEP 1-40-treated animals. The number of sprouts (≥200 µm in length) at 1-10 mm caudal to spinal cord in both Nogo receptor-1 antagonist-treated groups is approximately 20-40 times greater than control groups. More sprouts are seen from sNogoR310-Fc treated rats than local NEP 1-40 treatment (∼50 vs. 25 sprouts/rat), but this difference is not statistically significant (p=0.1713, t-test).

Regenerating CST axons are observed in transverse sections of spinal cord 11-15 mm caudal to hemisection in rats receiving sNogoR310-Fc treatment. These fibers are detected in both gray matter and white matter of the spinal cord. The fibers detected in gray matter often exhibit more collateral branching than in white matter area. In contrast, in transverse sections from vehicle-treated group, only occasional BDA-labeled are seen in the ventral white matter area, consistent with the uninjured ventral CST axons. At this level of distal spinal cord, the average number of BDA-labeled CST fibers from both Nogo receptor-1 antagonist-treated groups [sNogoR310-Fc and NEP 1-40] are approximately 20-fold greater than vehicle-treated rats. Taken together, both Nogo receptor antagonists, sNogoR310-Fc protein and NEP 1-40 peptide, result in dramatic CST axon regeneration in distal spinal cord, but the sprouting induced by the former exhibits a more highly branched pattern.

### Example 21

### Local sNogoR310-Fc Induces Sprouting of Rubropinal and Serotonergic Axons in Injured Rat Spinal Cord

Fourteen days after hemisection, a burr hole was made on each side of the skull overlying the sensorimotor cortex of the lower limbs to trace CST fibers. The anterograde neuronal tracer BDA (10% in PBS, 3.5 µl per cortex) was applied at seven injection sites at a depth of 1.5 mm from dura on each side (Grandpre, 2002). For rubrospinal tract tracing in rats, the tracer BDA (1 µl; MW 10,000; 10% in PBS) was injected into red nucleus on the left side (5.8 mm posterior to bregma, 0.7 mm lateral, 7.0 mm ventral to the skull surface). Two weeks after BDA injection, these animals were perfused with PBS, followed by 4% paraformadehyde, and tissue was collected for histology.

Repair of injured rubrospinal tract (RST) fibers contribute to functional improvements after spinal cord injury (Liu et al., 1999, J. Neurosci., 19, 4370-4387). The widespread distribution of Nogo receptor-1 in CNS neurons (Wang et al., 2002, J. Neurosci., 22, 5505-5515) makes it possible that inhibition of Nogo receptor-1 with its antagonist may result in regrowth of RST axons after injury. To test effects of sNogoR310-Fc on injured RST, the integrity of this pathway was traced by injecting BDA into left red nucleus. At the spinal cord level, RST fibers are normally located in dorsolateral white matter area of spinal cord, and are transected by the dorsal hemisections of this study. In transverse sections 11-15 mm rostral to lesion from control rats, a small number of short BDA-labeled fibers are seen between the prominent RST and dorsal horn gray matter. Sections at same level treated with sNogoR310-Fc exhibit many linking fibers between the main RST and dorsal horn gray matter. Transverse sections 11-15 mm distal to SCI, no BDA-labeled RST fibers in vehicle-treated rats. In contrast, sections at the same level receiving sNogoR310-Fc treatment display many BDA-labeled RST fibers in both gray and white matter contralateral to tracer injection. Some sprouts with a branching pattern are seen in the gray matter ipsilateral to BDA injection.

Ruphespinal spinal fibers were also examined in sNogoR310-Fc treated spinal injured rats. Immunostaining demonstrates the density of serotonergic fibers 11-15 mm rostral to lesion that is similar between vehicle and sNogoR310-Fc treated groups. In the sections 11-15 mm below the lesion, the seroton fibers in sNogoR310-Fc treated rats are twice as numerous as those in the control group. These results demonstrate that the responsiveness to Nogo receptor-1 inhibition by sNogoR310-Fc protein is not limited to CST fibers, and that the other descending tracts, such as rubrospinal and serotonergic axons, are also responsive to Nogo receptor-1 antagonism.

### Example 22

### Local Treatment with sNogoR310-Fc Improves Functional Recovery in Rats

Intrathecal administration of sNogoR310-Fc protein stimulates axon regeneration in several descending pathways after traumatic spinal cord injury. We tested whether the protein also improves functional recovery in the injured spinal cord.

At 2 weeks after the hemisection, the locomotor BBB score in vehicle-treated rats reaches a stable level of 12 (Fig. 14A). At 4 weeks after lesion, most of controls (6 out of 7) have frequent-consistent weight-supported plantar steps and frequent-consistent forelimb-hindlimb coordination, but they have a rotation of predominant paw position when making initial contact with surface. In contrast, in rats receiving sNogoR310-Fc protein treatment, the locomotor score continues to improve between 2-4 weeks post-trauma. At 4 weeks after injury, all 9 of the sNogoR310-Fc treated animals had consistent forelimb-hindlimb coordination and a parallel paw position at initial contact with the testing surface.

Grid walking has been used to assess the deficits in descending fine motor control after spinal cord injury (Metz et al., 2000). This performance requires forelimb-hindlimb coordination and voluntary movement control mediated by ventrolateral, corticospinal and rubrospinal fibers. During the pre-injury training, all the rats accurately place their hindlimbs on the grid bars. At 2-4 weeks post-injury, control rats make 8-9 errors per session with only minimal improvement over time. In contrast, the rats treated with sNogoR310-Fc exhibit a progressive improvement on grid walking and make significant fewer errors (4-7/session on average). The majority of the improvement occurs at 2-3 weeks after injury. Analysis of hindpaw footprints in control group displays that stride length is significantly decreased and stance width is increased at 4 weeks post-hemisection, compared with uninjured rats or injured animals receiving sNogoR310-Fc treatment (Fig. 14C). Therefore, these multiple behavioral tests demonstrate that blockade of Nogo receptor-1 function with local injection of antagonist protein improves locomotor recovery after injury.

### Biological Deposits

Hybridomas HB 7E11 (ATCC^{®} accession No. PTA-4587), HB 1H2 (ATCC^{®} accession No. PTA-4584), HB 3G5 (ATCC^{®} accession No. PTA-4586), HB 5B10 (ATCC^{®} accession No. PTA-4588) and HB 2F7 (ATCC^{®} accession No. PTA-4585) were deposited with the American Type Culture Collection ("ATCC^{®}"), 10801 University Boulevard, Manassas, VA 20110-2209, USA, on August 9, 2002.

## Claims

1. A polypeptide selected from the group consisting of SEQ ID NO: 1, SEQ ID NO: 2, SEQ ID NO: 3, SEQ ID NO: 4, and SEQ ID NO: 5.

2. A nucleic acid molecule encoding a polypeptide according to claim 1.

3. The nucleic acid molecule according to claim 2 operably linked to an expression control sequence.

4. A vector comprising the nucleic acid molecule according to claim 2 or 3.

5. A host cell comprising the nucleic acid molecule according to claim 2 or 3 or comprising the vector according to claim 4.

6. A method of producing the polypeptide according to claim 1 comprising the steps of:
(a) culturing the host cell according to claim 5; and
(b) recovering the polypeptide from the host cell or culture medium.

7. A method of producing an antibody comprising the steps of:
(a) immunizing a host with a polypeptide according to claim 1 or a host cell according to claim 5; and
(b) recovering the antibody.

8. An antibody or an antigen-binding fragment thereof that specifically binds to a polypeptide according to claim 1, or produced by the method according to claim 7, wherein said antibody or antigen-binding fragment:
(a) inhibits growth cone collapse of a neuron;
(b) decreases the inhibition of neurite outgrowth and sprouting in a neuron; or
(c) inhibits Nogo receptor-1 binding to a ligand.

9. The antibody or antigen-binding fragment according to claim 8, wherein the neurite outgrowth and sprouting is axonal growth.

10. The antibody or antigen-binding fragment according to claim 8 or 9, wherein the neuron is a central nervous system neuron.

11. The antibody or antigen-binding fragment according to any one of claims 8 to 10, wherein:
(a) the antibody is a monoclonal antibody;
(b) the antibody is a murine antibody; or
(c) the antibody is selected from the group consisting of a humanized antibody, a chimeric antibody, and a single chain antibody.

12. A hybridoma cell line selected from the group consisting of: HB 7E11 as deposited with the ATCC under accession No. PTA-4587, HB 1H2 as deposited with the ATCC under accession No. PTA-4584, HB 3G5 as deposited with the ATCC under accession No. PTA-4586, HB 5B10 as deposited with the ATCC under accession No. PTA-4588, and HB 2F7 as deposited with the ATCC under accession No. PTA-4585.

13. An antibody or antigen-binding fragment thereof produced by the hybridoma cell line according to claim 12.

14. An antibody or antigen-binding fragment thereof that competitively inhibits the binding of an antibody according to claim 13 to the polypeptide according to claim 1 or to a Nogo receptor-1, wherein said antibody or antigen-binding fragment:
(a) inhibits growth cone collapse of a neuron;
(b) decreases the inhibition of neurite outgrowth and sprouting in a neuron; or
(c) inhibits Nogo receptor-1 binding to a ligand.

15. An antibody or an antigen-binding fragment thereof that specifically binds Nogo receptor-1, wherein said antibody or fragment comprises a light chain comprising an amino acid sequence selected from the group consisting of:
(i) the amino acid sequence of SEQ ID NO: 15;
(ii) the amino acid sequence of SEQ ID NO: 16; and
(iii) an amino acid sequence comprising the CDR1, CDR2, and CDR3 amino acid sequences of SEQ ID NOs: 22, 23, and 24; and
wherein said antibody or antigen-binding fragment:
(a) inhibits growth cone collapse of a neuron;
(b) decreases the inhibition of neurite outgrowth and sprouting in a neuron; or
(c) inhibits Nogo receptor-1 binding to a ligand.

16. The antibody or antigen-binding fragment of claim 15 further comprising a heavy chain comprising an amino acid sequence selected from the group consisting of:
(i) the amino acid sequence of SEQ ID NO: 17;
(ii) the amino acid sequence of SEQ ID NO: 18; and
(iii) an amino acid sequence comprising the CDR1, CDR2, and CDR3 amino acid sequences of SEQ ID NOs: 19, 20, and 21.

17. An antibody or an antigen-binding fragment thereof that specifically binds Nogo receptor-1, wherein said antibody or fragment comprises a heavy chain comprising an amino acid sequence selected from the group consisting of:
(i) the amino acid sequence of SEQ ID NO: 17;
(ii) the amino acid sequence of SEQ ID NO: 18; and
(iii) an amino acid sequence comprising the CDR1, CDR2, and CDR3 amino acid sequences of SEQ ID NOs: 19, 20, and 21; and
wherein said antibody or antigen-binding fragment:
(a) inhibits growth cone collapse of a neuron;
(b) decreases the inhibition of neurite outgrowth and sprouting in a neuron; or
(c) inhibits Nogo receptor-1 binding to a ligand.

18. The antibody or antigen-binding fragment of claim 17 further comprising a light chain comprising an amino acid sequence selected from the group consisting of:
(i) the amino acid sequence of SEQ ID NO: 15;
(ii) the amino acid sequence of SEQ ID NO: 16; and
(iii) an amino acid sequence comprising the CDR1, CDR2, and CDR3 amino acid sequences of SEQ ID NOs: 22, 23, and 24.

19. A soluble Nogo receptor-1 polypeptide selected from the group consisting of: amino acid residues 26-344 of SEQ ID NO: 6; amino acid residues 26-310 of SEQ ID NO: 7; amino acid residues 26-344 of SEQ ID NO: 8; amino acid residues 26-310 of SEQ ID NO: 9; amino acid residues 27-344 of SEQ ID NO: 8; and amino acid residues 27-310 of SEQ ID NO: 9.

20. The soluble Nogo receptor-1 polypeptide according to claim 19 joined to a signal sequence.

21. A nucleic acid molecule encoding the soluble Nogo receptor-1 polypeptide according to any one of claims 19 to 20.

22. The nucleic acid molecule according to claim 21 operably linked to an expression control sequence.

23. A vector comprising the nucleic acid molecule according to claim 21 or 22.

24. A host cell comprising the nucleic acid molecule according to claim 21 or 22 or comprising the vector according to claim 23.

25. A method of producing the soluble Nogo receptor-1 polypeptide according to any one of claims 19 to 20 comprising the steps of:
(a) culturing the host cell according to claim 24; and
(b) recovering the polypeptide from the host cell or culture medium.

26. A Nogo receptor-1 fusion protein comprising:
(a) amino acid residues 26-344 of SEQ ID NO: 6; amino acid residues 26-310 of SEQ ID NO: 7; amino acid residues 26-344 of SEQ ID NO: 8; amino acid residues 26-310 of SEQ ID NO: 9; amino acid residues 27-344 of SEQ ID NO: 8; and amino acid residues 27-310 of SEQ ID NO: 9; and
(b) a heterologous polypeptide comprising:
(i) an immunoglobulin constant region; or
(ii) an Fc region.

27. The Nogo receptor-1 fusion protein according to claim 26, which is a dimer.

28. The Nogo receptor-1 fusion protein according to claim 26 or 27, wherein the immunoglobulin constant region is an immunoglobulin heavy chain constant region.

29. The Nogo receptor-1 fusion protein according to claim 28 wherein the immunoglobulin heavy chain constant region is an IgG heavy chain constant region.

30. The Nogo receptor-1 fusion protein according to any one of claims 26 to 29, further comprising a signal sequence.

31. A nucleic acid molecule encoding the Nogo receptor-1 fusion protein according to any one of claims 26 to 30.

32. The nucleic acid molecule according to claim 31 operably linked to an expression control sequence.

33. A vector comprising the nucleic acid molecule according to claim 31 or 32.

34. A host cell comprising the nucleic acid molecule according to claim 31 or 32 or comprising the vector according to claim 33.

35. A method of producing the Nogo receptor-1 fusion protein according to any one of claims 26 to 30 comprising the steps of:
(a) culturing the host cell according to claim 34; and
(b) recovering the Nogo fusion protein from the host cell or culture medium.

36. A method of producing a Nogo receptor-1-specific antibody comprising the steps of:
(a) immunizing a host with a soluble Nogo receptor-1 polypeptide according to any one of claims 19 to 20, a Nogo receptor-1 fusion protein according to any one of claims 26 to 30, or a host cell according to claim 24 or 34; and
(b) recovering the antibody.

37. An antibody or antigen-binding fragment thereof that specifically binds to a soluble Nogo receptor-1 polypeptide according to any one of claims 19 to 20, or produced by the method according to claim 36, wherein said antibody or antigen-binding fragment:
(a) inhibits growth cone collapse of a neuron;
(b) decreases the inhibition of neurite outgrowth and sprouting in a neuron; or
(c) inhibits Nogo receptor-1 binding to a ligand.

38. An *in vitro* method of inhibiting Nogo receptor-1 binding to a ligand;
comprising the step of:
(a) contacting Nogo receptor-1 with an antibody or antigen-binding fragment according to any one of claims 8 to 11 and 13 to 18; or
(b) contacting the ligand with the soluble Nogo receptor-1 polypeptide according to any one of claims 19 to 20 or the Nogo receptor-1 fusion protein according to any one of claims 26 to 30.

39. Use of an antibody or antigen-binding fragment according to any one of claims 8 to 11 and 13 to 18, or the soluble Nogo receptor-1 polypeptide according to any one of claims 19 to 20, or the Nogo receptor-1 fusion protein according to any one of claims 26 to 30, for the preparation of a pharmaceutical composition for treating a mammal displaying signs or symptoms of multiple sclerosis, ALS, Huntington's disease, Alzheimer's disease, Parkinson's disease, diabetic neuropathy, stroke, traumatic brain injuries or spinal cord injury.

40. An *in vitro* methods of modulating an activity of a Nogo receptor-1 ligand, comprising the step of contacting the Nogo receptor-1 ligand with the polypeptide according to any one of claims 19 to 20 or the Nogo receptor-1 fusion protein according to any one of claims 26 to 30.

41. Use of the polypeptide according to any one of claims 19 to 20, or the Nogo receptor-1 fusion protein according to any one of claims 26 to 30, for the preparation of a pharmaceutical composition for treating a mammal displaying signs or symptoms of multiple sclerosis, ALS, Huntington's disease, Alzheimer's disease, Parkinson's disease, diabetic neuropathy, stroke, traumatic brain injuries or spinal cord injury.

42. An *in vitro* method for inhibiting growth cone collapse in a neuron, comprising the step of:
(a) contacting the neuron with the antibody or antigen-binding fragment thereof according to any one of claims 8 to 11 and 13 to 18; or
(b) contacting a Nogo receptor-1 ligand with the polypeptide according to any one of claims 19 to 20 or the Nogo receptor-1 fusion protein according to any one of claims 26 to 30.

43. An *in vitro* method for decreasing the inhibition of neurite outgrowth or sprouting in a neuron, comprising the step of:
(a) contacting the neuron with the antibody or antigen-binding fragment thereof according to any one of claims 8 to 11 and 13 to 18; or
(b) contacting a Nogo receptor-1 ligand with the polypeptide according to any one of claims 19 to 20 or the Nogo receptor-1 fusion protein according to any one of claims 26 to 30.

44. The method according to any one of claims 38, 40, 42 and 43, wherein the ligand is selected from the group consisting of NogoA, NogoB, NogoC, MAG and OM-gp.

45. The method according to claim 43, wherein the neurite outgrowth or sprouting is axonal growth.

46. The method according to any one of claims 42, 43 and 45, wherein the neuron is a CNS neuron.

47. A composition comprising a pharmaceutically acceptable carrier and a component selected from:
(a) the antibody or an antigen-binding fragment according to any one of claims 8 to 11, 13 to 18 and 37;
(b) the soluble Nogo receptor-1 polypeptide according to any one of claims 19 to 20; and
(c) the fusion protein according to any one of claims 26 to 30.

48. The composition according to claim 47 further comprising one or more additional therapeutic agents.

49. An *in vitro* method of promoting survival of a neuron at risk of dying, comprising contacting the neuron with an effective amount of:
(a) an anti-Nogo receptor-1 antibody or antigen-binding fragment according to any one of claims 8 to 11 and 13 to 18; or
(b) a soluble Nogo receptor-1 polypeptide according to any one of claims 19 to 20; or
(c) a Nogo receptor-1 fusion protein according to any one of claims 26 to 30.

50. The method of claim 49, or the use of claim 39 or 41, wherein the fusion protein is an Fc fusion protein.

51. The method or use of claim 50, wherein the Fc fusion protein is amino acid residues 26-344 of SEQ IQ NO: 8 and the hinge and Fc region of the rat IgG1 molecule (Ig-sNogoR344).

52. The method of any one of claims 49 to 51, wherein the neuron is from a mammal.

53. The method of claim 52, wherein the mammal displays signs or symptoms of multiple sclerosis, ALS, Huntington's disease, Alzheimer's disease, Parkinson's disease, diabetic neuropathy, stroke, traumatic brain injuries or spinal cord injury.

54. Use of a cultured host cell expressing:
(a) an anti-Nogo receptor-1 antibody or antigen-binding fragment thereof according to any one of claims 8 to 11 and 13 to 18; or
(b) a soluble Nogo receptor-1 polypeptide according to any one of claims 19 to 20; or
(c) a Nogo receptor-1 fusion protein according to any one of claims 26 to 30;
for the preparation of a pharmaceutical composition for treating a mammal displaying signs or symptoms of multiple sclerosis, ALS, Huntington's disease, Alzheimer's disease, Parkinson's disease, diabetic neuropathy, stroke, traumatic brain injuries or spinal cord injury, wherein said host cell is to be introduced into the mammal at or near the site of a neuron which is at risk of dying.

55. Use of a viral vector comprising a nucleotide sequence that encodes:
(a) an anti-Nogo receptor-1 antibody or antigen-binding fragment thereof according to any one of claims 8 to 11 and 13 to 18; or
(b) a soluble Nogo receptor-1 polypeptide according to any one of claims 19 to 20; or
(c) a Nogo receptor-1 fusion protein according to any one of claims 26 to 30;
for the preparation of a pharmaceutical composition for treating a mammal displaying signs or symptoms of multiple sclerosis, ALS, Huntington's disease, Alzheimer's disease, Parkinson's disease, diabetic neuropathy, stroke, traumatic brain injuries or spinal cord injury, wherein the anti-Nogo receptor-1 antibody, antigen-binding fragment, Nogo receptor-1 fusion protein, or soluble Nogo receptor-1 polypeptide is to be expressed from the nucleotide sequence in the mammal in an amount sufficient to promote survival of a neuron at risk of dying.

## Patentansprüche

1. Polypeptid, welches aus der Gruppe ausgewählt ist, die aus SEQ ID Nr.: 1, SEQ ID Nr.: 2, SEQ ID Nr.: 3, SEQ ID Nr.: 4 und SEQ ID Nr.: 5 besteht.

2. Nukleinsäuremolekül, welches ein Polypeptid gemäß Anspruch 1 kodiert.

3. Nukleinsäuremolekül gemäß Anspruch 2, welches mit einer Expressionskontrollsequenz funktionell verbunden ist.

4. Vektor, umfassend das Nukleinsäuremolekül gemäß Anspruch 2 oder 3.

5. Wirtszelle, umfassend das Nukleinsäuremolekül gemäß Anspruch 2 oder 3 oder umfassend den Vektor gemäß Anspruch 4.

6. Verfahren zur Herstellung des Polypeptids gemäß Anspruch 1, umfassend die Schritte von:
(a) Kultivieren der Wirtszelle gemäß Anspruch 5; und
(b) Gewinnen des Polypeptids aus der Wirtszelle oder dem Kulturmedium.

7. Verfahren zur Herstellung eines Antikörpers, umfassend die Schritte von:
(a) Immunisieren eines Wirts mit einem Polypeptid gemäß Anspruch 1 oder einer Wirtszelle gemäß Anspruch 5; und
(b) Gewinnen des Antikörpers.

8. Antikörper oder Antigen-bindendes Fragment davon, welche spezifisch an ein Polypeptid gemäß Anspruch 1 binden oder durch das Verfahren gemäß Anspruch 7 hergestellt wurden, wobei der Antikörper oder das Antigen-bindende Fragment davon:
(a) einen Wachstumskegelzusammenbruch eines Neurons inhibieren;
(b) die Inhibierung von Neuritauswuchs und -aussprossung in einem Neuron erniedrigen; oder
(c) eine Nogo-Rezeptor-1-Bindung an einen Liganden inhibieren.

9. Antikörper oder Antigen-bindendes Fragment gemäß Anspruch 8, wobei der bzw. die Neuritauswuchs und -aussprossung axonales Wachstum sind.

10. Antikörper oder Antigen-bindendes Fragment gemäß Anspruch 8 oder 9, wobei das Neuron ein Zentralnervensystemneuron ist.

11. Antikörper oder Antigen-bindendes Fragment gemäß einem der Ansprüche 8 bis 10, wobei:
(a) der Antikörper ein monoklonaler Antikörper ist;
(b) der Antikörper ein muriner Antikörper ist; oder
(c) der Antikörper aus der Gruppe ausgewählt ist, die aus einem humanisierten Antikörper, einem chimären Antikörper und einem Einzelketten-Antikörper besteht.

12. Hybridomzelllinie, welche aus der Gruppe ausgewählt ist, die besteht aus: HB 7E11 wie hinterlegt bei der ATCC unter der Zugangsnr. PTA-4587, HB 1H2 wie hinterlegt bei der ATCC unter der Zugangsnr. PTA-4584, HB 3G5 wie hinterlegt bei der ATCC unter der Zugangsnr. PTA-4586, HB 5B10 wie hinterlegt bei der ATCC unter der Zugangsnr. PTA-4588 und HB 2F7 wie hinterlegt bei der ATCC unter der Zugangsnr. PTA-4585.

13. Antikörper oder Antigen-bindendes Fragment davon, welche durch die Hybridomzelllinie gemäß Anspruch 12 hergestellt wurden.

14. Antikörper oder Antigen-bindendes Fragment davon, welche die Bindung eines Antikörpers gemäß Anspruch 13 an dem Polypeptid gemäß Anspruch 1 oder an einem Nogo-Rezeptor-1 kompetitiv inhibieren, wobei der Antikörper oder das Antigen-bindende Fragment:
(a) einen Wachstumskegelzusammenbruch eines Neurons inhibieren;
(b) die Inhibierung von Neuritauswuchs und -aussprossung in einem Neuron erniedrigen; oder
(c) eine Nogo-Rezeptor-1-Bindung an einen Liganden inhibieren.

15. Antikörper oder Antigen-bindendes Fragment davon, welche einen Nogo-Rezeptor-1 spezifisch binden, wobei der Antikörper oder das Fragment eine leichte Kette umfassen, umfassend eine Aminosäuresequenz, welche aus der Gruppe ausgewählt ist, die besteht aus:
(i) der Aminosäuresequenz SEQ ID Nr.: 15;
(ii) der Aminosäuresequenz SEQ ID Nr.: 16; und
(iii) einer Aminosäuresequenz, welche die CDR1-, CDR2- und CDR3-Aminosäuresequenzen von SEQ ID Nr.: 22, 23 und 24 umfasst; und
wobei der Antikörper oder das Antigen-bindende Fragment:
(a) einen Wachstumskegelzusammenbruch eines Neurons inhibieren;
(b) die Inhibierung von Neuritauswuchs und -aussprossung in einem Neuron erniedrigen; oder
(c) eine Nogo-Rezeptor-1-Bindung an einen Liganden inhibieren.

16. Antikörper oder Antigen-bindendes Fragment nach Anspruch 15, ferner umfassend eine schwere Kette, umfassend eine Aminosäuresequenz, welche aus der Gruppe ausgewählt ist, die besteht aus:
(i) der Aminosäuresequenz SEQ ID Nr.: 17;
(ii) der Aminosäuresequenz SEQ ID Nr.: 18; und
(iii) einer Aminosäuresequenz, welche die CDR1-, CDR2- und CDR3-Aminosäuresequenzen von SEQ ID Nr.: 19, 20 und 21 umfasst.

17. Antikörper oder Antigen-bindendes Fragment davon, welche einen Nogo-Rezeptor-1 spezifisch binden, wobei der Antikörper oder das Fragment eine schwere Kette umfassen, umfassend eine Aminosäuresequenz, welche aus der Gruppe ausgewählt ist, die besteht aus:
(i) der Aminosäuresequenz SEQ ID Nr.: 17;
(ii) der Aminosäuresequenz SEQ ID Nr.: 18; und
(iii) einer Aminosäuresequenz, welche die CDR1-, CDR2- und CDR3-Aminosäuresequenzen von SEQ ID Nr.: 19, 20 und 21 umfasst; und
wobei der Antikörper oder das Antigen-bindende Fragment:
(a) einen Wachstumskegelzusammenbruch eines Neurons inhibieren;
(b) die Inhibierung von Neuritauswuchs und -aussprossung in einem Neuron erniedrigen; oder
(c) eine Nogo-Rezeptor-1-Bindung an einen Liganden inhibieren.

18. Antikörper oder Antigen-bindendes Fragment nach Anspruch 17, ferner umfassend eine leichte Kette, umfassend eine Aminosäuresequenz, welche aus der Gruppe ausgewählt ist, die besteht aus:
(i) der Aminosäuresequenz SEQ ID Nr.: 15;
(ii) der Aminosäuresequenz SEQ ID Nr.: 16; und
(iii) einer Aminosäuresequenz, welche die CDR1-, CDR2- und CDR3-Aminosäuresequenzen von SEQ ID Nr.: 22, 23 und 24 umfasst.

19. Lösliches Nogo-Rezeptor-1-Polypeptid, welches aus der Gruppe ausgewählt ist, die besteht aus: Aminosäureresten 26-344 von SEQ ID Nr.: 6; Aminosäureresten 26-310 von SEQ ID Nr.: 7; Aminosäureresten 26-344 von SEQ ID Nr.: 8; Aminosäureresten 26-310 von SEQ ID Nr.: 9; Aminosäureresten 27-344 von SEQ ID Nr.: 8; und Aminosäureresten 27-310 von SEQ ID Nr.: 9.

20. Lösliches Nogo-Rezeptor-1-Polypeptid gemäß Anspruch 19, welches an eine Signalsequenz gebunden ist.

21. Nukleinsäuremolekül, welches das lösliche Nogo-Rezeptor-1-Polypeptid gemäß einem der Ansprüche 19 bis 20 kodiert.

22. Nukleinsäuremolekül gemäß Anspruch 21, welches mit einer Expressionskontrollsequenz funktionell verbunden ist.

23. Vektor, umfassend das Nukleinsäuremolekül gemäß Anspruch 21 oder 22.

24. Wirtszelle, umfassend das Nukleinsäuremolekül gemäß Anspruch 21 oder 22 oder umfassend den Vektor gemäß Anspruch 23.

25. Verfahren zur Herstellung des löslichen Nogo-Rezeptor-1-Polypeptids gemäß einem der Ansprüche 19 bis 20, umfassend die Schritte von:
(a) Kultivieren der Wirtszelle gemäß Anspruch 24; und
(b) Gewinnen des Polypeptids aus der Wirtszelle oder dem Kulturmedium.

26. Nogo-Rezeptor-1-Fusionsprotein, umfassend:
(a) Aminosäurereste 26-344 von SEQ ID Nr.: 6; Aminosäurereste 26-310 von SEQ ID Nr.: 7; Aminosäurereste 26-344 von SEQ ID Nr.: 8; Aminosäurereste 26-310 von SEQ ID Nr.: 9; Aminosäurereste 27-344 von SEQ ID Nr.: 8; und Aminosäurereste 27-310 von SEQ ID Nr.: 9; und
(b) ein heterologes Polypeptid, umfassend:
(i) eine konstante Immunglobulinregion; oder
(ii) eine Fc-Region.

27. Nogo-Rezeptor-1-Fusionsprotein gemäß Anspruch 26, welches ein Dimer ist.

28. Nogo-Rezeptor-1-Fusionsprotein gemäß Anspruch 26 oder 27, wobei die konstante Immunglobulinregion eine konstante Region einer schweren Kette eines Immunglobulins ist.

29. Nogo-Rezeptor-1-Fusionsprotein gemäß Anspruch 28, wobei die konstante Region einer schweren Kette eines Immunglobulins eine konstante Region einer schweren Kette von IgG ist.

30. Nogo-Rezeptor-1-Fusionsprotein gemäß einem der Ansprüche 26 bis 29, ferner umfassend eine Signalsequenz.

31. Nukleinsäuremolekül, welches das Nogo-Rezeptor-1-Fusionsprotein gemäß einem der Ansprüche 26 bis 30 kodiert.

32. Nukleinsäuremolekül gemäß Anspruch 31, welches mit einer Expressionskontrollsequenz funktionell verbunden ist.

33. Vektor, umfassend das Nukleinsäuremolekül gemäß Anspruch 31 oder 32.

34. Wirtszelle, umfassend das Nukleinsäuremolekül gemäß Anspruch 31 oder 32 oder umfassend den Vektor gemäß Anspruch 33.

35. Verfahren zur Herstellung des Nogo-Rezeptor-1-Fusionsproteins gemäß einem der Ansprüche 26 bis 30, umfassend die Schritte von:
(a) Kultivieren der Wirtszelle gemäß Anspruch 34; und
(b) Gewinnen des Nogo-Rezeptor-1-Fusionsproteins aus der Wirtszelle oder dem Kulturmedium.

36. Verfahren zur Herstellung eines Nogo-Rezeptor-1-spezifischen Antikörpers, umfassend die Schritte von:
(a) Immunisieren eines Wirts mit einem löslichen Nogo-Rezeptor-1-Polypeptid gemäß einem der Ansprüche 19 bis 20, einem Nogo-Rezeptor-1-Fusionsprotein gemäß einem der Ansprüche 26 bis 30 oder einer Wirtszelle gemäß Anspruch 24 oder 34; und
(b) Gewinnen des Antikörpers.

37. Antikörper oder Antigen-bindendes Fragment davon, welche an ein lösliches Nogo-Rezeptor-1-Polypeptid gemäß einem der Ansprüche 19 bis 20 spezifisch binden oder durch das Verfahren gemäß Anspruch 36 hergestellt wurden, wobei der Antikörper oder das Antigen-bindende Fragment:
(a) einen Wachstumskegelzusammenbruch eines Neurons inhibieren;
(b) die Inhibierung von Neuritauswuchs und -aussprossung in einem Neuron erniedrigen; oder
(c) eine Nogo-Rezeptor-1-Bindung an einen Liganden inhibieren.

38. *In vitro*-Verfahren zur Inhibierung von Nogo-Rezeptor-1-Bindung an einen Liganden, umfassend den Schritt von:
(a) Inkontaktbringen eines Nogo-Rezeptor-1 mit einem Antikörper oder Antigen-bindenden Fragment gemäß einem der Ansprüche 8 bis 11 und 13 bis 18; oder
(b) Inkontaktbringen des Liganden mit dem löslichen Nogo-Rezeptor-1-Polypeptid gemäß einem der Ansprüche 19 bis 20 oder dem Nogo-Rezeptor-1-Fusionsprotein gemäß einem der Ansprüche 26 bis 30.

39. Verwendung eines Antikörpers oder Antigen-bindenden Fragments gemäß einem der Ansprüche 8 bis 11 und 13 bis 18 oder des löslichen Nogo-Rezeptor-1-Polypeptids gemäß einem der Ansprüche 19 bis 20 oder des Nogo-Rezeptor-1-Fusionsproteins gemäß einem der Ansprüche 26 bis 30 zur Herstellung einer pharmazeutischen Zusammensetzung zur Behandlung eines Säugers, welcher Anzeichen oder Symptome von Multipler Sklerose, ALS, Huntington-Krankheit, Alzheimer-Krankheit, Parkinson-Krankheit, diabetischer Neuropathie, Schlaganfall, traumatischen Gehirnverletzungen oder Rückenmarkverletzung zeigt.

40. *In vivo*-Verfahren zum Modulieren einer Aktivität eines Nogo-Rezeptor-1-Liganden, umfassend den Schritt von Inkontaktbringen des Nogo-Rezeptor-1-Liganden mit dem Polypeptid gemäß einem der Ansprüche 19 bis 20 oder dem Nogo-Rezeptor-1-Fusionsprotein gemäß einem der Ansprüche 26 bis 30.

41. Verwendung des Polypeptids gemäß einem der Ansprüche 19 bis 20 oder des Nogo-Rezeptor-1-Fusionsproteins gemäß einem der Ansprüche 26 bis 30 zur Herstellung einer pharmazeutischen Zusammensetzung zur Behandlung eines Säugers, welcher Anzeichen oder Symptome von Multipler Sklerose, ALS, Huntington-Krankheit, Alzheimer-Krankheit, Parkinson-Krankheit, diabetischer Neuropathie, Schlaganfall, traumatischen Gehirnverletzungen oder Rückenmarkverletzung zeigt.

42. *In vitro*-Verfahren zur Inhibierung eines Wachstumskegelzusammenbruchs in einem Neuron, umfassend den Schritt von:
(a) Inkontaktbringen des Neurons mit dem Antikörper oder Antigen-bindenden Fragment davon gemäß einem der Ansprüche 8 bis 11 und 13 bis 18; oder
(b) Inkontaktbringen eines Nogo-Rezeptor-1-Liganden mit dem Polypeptid gemäß einem der Ansprüche 19 bis 20 oder dem Nogo-Rezeptor-1-Fusionsprotein gemäß einem der Ansprüche 26 bis 30.

43. *In vitro*-Verfahren zur Erniedrigung der Inhibierung von Neuritauswuchs oder -aussprossung in einem Neuron, umfassend den Schritt von:
(a) Inkontaktbringen des Neurons mit dem Antikörper oder Antigen-bindenden Fragment davon gemäß einem der Ansprüche 8 bis 11 und 13 bis 18; oder
(b) Inkontaktbringen eines Nogo-Rezeptor-1-Liganden mit dem Polypeptid gemäß einem der Ansprüche 19 bis 20 oder dem Nogo-Rezeptor-1-Fusionsprotein gemäß einem der Ansprüche 26 bis 30.

44. Verfahren gemäß einem der Ansprüche 38, 40, 42 und 43, wobei der Ligand aus der Gruppe ausgewählt ist, die aus NogoA, NogoB, NogoC, MAG und OM-gp besteht.

45. Verfahren gemäß Anspruch 43, wobei der bzw. die Neuritauswuchs oder -aussprossung axonales Wachstum sind.

46. Verfahren gemäß einem der Ansprüche 42, 43 und 45, wobei das Neuron ein ZNS-Neuron ist.

47. Zusammensetzung, umfassend einen pharmazeutisch verträglichen Träger und eine Komponente, welche ausgewählt ist aus:
(a) dem Antikörper oder einem Antigen-bindenden Fragment gemäß einem der Ansprüche 8 bis 11, 13 bis 18 und 37;
(b) dem löslichen Nogo-Rezeptor-1-Polypeptid gemäß einem der Ansprüche 19 bis 20; und
(c) dem Fusionsprotein gemäß einem der Ansprüche 26 bis 30.

48. Zusammensetzung gemäß Anspruch 47, ferner umfassend ein oder mehrere zusätzliche therapeutische Mittel.

49. *In vitro*-Verfahren zur Förderung des Überlebens eines Neurons, welches abzusterben droht, umfassend das Inkontaktbringen des Neurons mit einer wirksamen Menge von:
(a) einem anti-Nogo-Rezeptor-1-Antikörper oder Antigen-bindenden Fragment gemäß einem der Ansprüche 8 bis 11 und 13 bis 18; oder
(b) einem löslichen Nogo-Rezeptor-1-Polypeptid gemäß einem der Ansprüche 19 bis 20; oder
(c) einem Nogo-Rezeptor-1-Fusionsprotein gemäß einem der Ansprüche 26 bis 30.

50. Verfahren nach Anspruch 49 oder Verwendung nach Anspruch 39 oder 41, wobei das Fusionsprotein ein Fc-Fusionsprotein ist.

51. Verfahren oder Verwendung nach Anspruch 50, wobei das Fc-Fusionsprotein Aminosäurereste 26-344 von SEQ ID Nr.: 8 und die Hinge- und Fc-Region des Ratte-IgG1-Moleküls (Ig-sNogoR344) ist.

52. Verfahren nach einem der Ansprüche 49 bis 51, wobei das Neuron von einem Säuger stammt.

53. Verfahren nach Anspruch 52, wobei der Säuger Anzeichen oder Symptome von Multipler Sklerose, ALS, Huntington-Krankheit, Alzheimer-Krankheit, Parkinson-Krankheit, diabetischer Neuropathie, Schlaganfall, traumatischen Gehirnverletzungen oder Rückenmarkverletzung zeigt.

54. Verwendung einer kultivierten Wirtszelle, welche exprimiert:
(a) einen anti-Nogo-Rezeptor-1-Antikörper oder ein Antigen-bindendes Fragment davon gemäß einem der Ansprüche 8 bis 11 und 13 bis 18; oder
(b) ein lösliches Nogo-Rezeptor-1-Polypeptid gemäß einem der Ansprüche 19 bis 20; oder
(c) ein Nogo-Rezeptor-1-Fusionsprotein gemäß einem der Ansprüche 26 bis 30; zur Herstellung einer pharmazeutischen Zusammensetzung zur Behandlung eines Säugers, welcher Anzeichen oder Symptome von Multipler Sklerose, ALS, Huntington-Krankheit, Alzheimer-Krankheit, Parkinson-Krankheit, diabetischer Neuropathie, Schlaganfall, traumatischen Gehirnverletzungen oder Rückenmarkverletzung zeigt, wobei die Wirtszelle in den Säuger an oder nahe der Stelle eines Neurons eingebracht wird, welches abzusterben droht.

55. Verwendung eines viralen Vektors, umfassend eine Nukleotidsequenz, welche kodiert:
(a) einen anti-Nogo-Rezeptor-1-Antikörper oder ein Antigen-bindendes Fragment davon gemäß einem der Ansprüche 8 bis 11 und 13 bis 18; oder
(b) ein lösliches Nogo-Rezeptor-1-Polypeptid gemäß einem der Ansprüche 19 bis 20; oder
(c) ein Nogo-Rezeptor-1-Fusionsprotein gemäß einem der Ansprüche 26 bis 30; zur Herstellung einer pharmazeutischen Zusammensetzung zur Behandlung eines Säugers, welcher Anzeichen oder Symptome von Multipler Sklerose, ALS, Huntington-Krankheit, Alzheimer-Krankheit, Parkinson-Krankheit, diabetischer Neuropathie, Schlaganfall, traumatischen Gehirnverletzungen oder Rückenmarkverletzung zeigt, wobei der anti-Nogo-Rezeptor-1-Antikörper, das Antigen-bindende Fragment, das Nogo-Rezeptor-1-Fusionsprotein oder das lösliche Nogo-Rezeptor-1-Polypeptid von der Nukleotidsequenz in dem Säuger in einer Menge exprimiert werden, welche ausreichend ist, das Überleben eines Neurons zu fördern, welches abzusterben droht.

## Revendications

1. Polypeptide choisi dans le groupe constitué par SEQ ID NO : 1, SEQ ID NO : 2, SEQ ID NO : 3, SEQ ID NO : 4 et SEQ ID NO : 5.

2. Molécule d'acide nucléique codant pour un polypeptide selon la revendication 1.

3. Molécule d'acide nucléique selon la revendication 2, liée de manière fonctionnelle à une séquence de contrôle de l'expression.

4. Vecteur comprenant la molécule d'acide nucléique selon la revendication 2 ou 3.

5. Cellule hôte comprenant la molécule d'acide nucléique selon la revendication 2 ou 3 ou comprenant le vecteur selon la revendication 4.

6. Procédé de production du polypeptide selon la revendication 1 comprenant les étapes consistant à :
(a) cultiver la cellule hôte selon la revendication 5 ; et
(b) récupérer le polypeptide à partir de la cellule hôte ou du milieu de culture.

7. Procédé de production d'un anticorps comprenant les étapes consistant à :
(a) immuniser un hôte avec un polypeptide selon la revendication 1 ou une cellule hôte selon la revendication 5 ; et
(b) récupérer l'anticorps.

8. Anticorps ou fragment de liaison de l'antigène de celui-ci qui se lie spécifiquement à un polypeptide selon la revendication 1, ou produit par le procédé selon la revendication 7, où ledit anticorps ou fragment de liaison de l'antigène :
(a) inhibe le collapsus du cône de croissance d'un neurone ;
(b) diminue l'inhibition de la croissance des neurites et du bourgeonnement dans un neurone ; ou
(c) inhibe la liaison du récepteur Nogo 1 à un ligand.

9. Anticorps ou fragment de liaison de l'antigène selon la revendication 8, où la croissance des neurites et le bourgeonnement représentent la croissance axonale.

10. Anticorps ou fragment de liaison de l'antigène selon la revendication 8 ou 9, où le neurone est un neurone du système nerveux central.

11. Anticorps ou fragment de liaison de l'antigène selon l'une quelconque des revendications 8 à 10, où :
(a) l'anticorps est un anticorps monoclonal ;
(b) l'anticorps est un anticorps murin ; ou
(c) l'anticorps est choisi dans le groupe constitué par un anticorps humanisé, un anticorps chimérique et un anticorps à chaîne unique.

12. Lignée cellulaire d'hybridome choisie dans le groupe constitué par : HB 7E11 déposée auprès de l'ATCC avec le No. d'accession PTA-4587, HB 1H2 déposée auprès de l'ATCC avec le No. d'accession PTA-4584, HB 3G5 déposée auprès de l'ATCC avec le No. d'accession PTA-4586, HB 5B10 déposée auprès de l'ATCC avec le No. d'accession PTA-4588 et HB 2F7 déposée auprès de l'ATCC avec le No. d'accession PTA-4585.

13. Anticorps ou fragment de liaison de l'antigène de celui-ci produit par la lignée cellulaire d'hybridome selon la revendication 12.

14. Anticorps ou fragment de liaison de l'antigène de celui-ci qui inhibe de manière compétitive la liaison d'un anticorps selon la revendication 13 au polypeptide selon la revendication 1 ou à un récepteur Nogo 1, où ledit anticorps ou fragment de liaison de l'antigène :
(a) inhibe le collapsus du cône de croissance d'un neurone ;
(b) diminue l'inhibition de la croissance des neurites et du bourgeonnement dans un neurone ; ou
(c) inhibe la liaison du récepteur Nogo 1 à un ligand.

15. Anticorps ou fragment de liaison de l'antigène de celui-ci qui se lie spécifiquement au récepteur Nogo 1, où ledit anticorps ou fragment comprend une chaîne légère comprenant une séquence d'acides aminés choisie dans le groupe constitué par :
(i) la séquence d'acides aminés de SEQ ID NO : 15 ;
(ii) la séquence d'acides aminés de SEQ ID NO : 16 ; et
(iii) une séquence d'acides aminés comprenant les séquences d'acides aminés de SEQ ID NO : 22, 23 et 24 des CDR1, CDR2 et CDR3 ; et
où ledit anticorps ou fragment de liaison de l'antigène :
(a) inhibe le collapsus du cône de croissance d'un neurone ;
(b) diminue l'inhibition de la croissance des neurites et du bourgeonnement dans un neurone ; ou
(c) inhibe la liaison du récepteur Nogo 1 à un ligand.

16. Anticorps ou fragment de liaison de l'antigène selon la revendication 15, comprenant en outre une chaîne lourde comprenant une séquence d'acides aminés choisie dans le groupe constitué par :
(i) la séquence d'acides aminés de SEQ ID NO : 17 ;
(ii) la séquence d'acides aminés de SEQ ID NO : 18 ; et
(iii) une séquence d'acides aminés comprenant les séquences d'acides aminés de SEQ ID NO : 19, 20 et 21 des CDR1, CDR2 et CDR3.

17. Anticorps ou fragment de liaison de l'antigène de celui-ci qui se lie spécifiquement au récepteur Nogo 1, où ledit anticorps ou fragment comprend une chaîne lourde comprenant une séquence d'acides aminés choisie dans le groupe constitué par :
(i) la séquence d'acides aminés de SEQ ID NO : 17 ;
(ii) la séquence d'acides aminés de SEQ ID NO : 18 ; et
(iii) une séquence d'acides aminés comprenant les séquences d'acides aminés de SEQ ID NO : 19, 20 et 21 des CDR1, CDR2 et CDR3 ; et
où ledit anticorps ou fragment de liaison de l'antigène:
(a) inhibe le collapsus du cône de croissance d'un neurone ;
(b) diminue l'inhibition de la croissance des neurites et du bourgeonnement dans un neurone ; ou
(c) inhibe la liaison du récepteur Nogo 1 à un ligand.

18. Anticorps ou fragment de liaison de l'antigène selon la revendication 17, comprenant en outre une chaîne légère comprenant une séquence d'acides aminés choisie dans le groupe constitué par :
(i) la séquence d'acides aminés de SEQ ID NO : 15 ;
(ii) la séquence d'acides aminés de SEQ ID NO : 16 ; et
(iii) une séquence d'acides aminés comprenant les séquences d'acides aminés de SEQ ID NO : 22, 23 et 24 des CDR1, CDR2 et CDR3.

19. Polypeptide du récepteur Nogo 1 soluble choisi dans le groupe constitué par : les résidus d'acides aminés 26 à 344 de SEQ ID NO : 6 ; les résidus d'acides aminés 26 à 310 de SEQ ID NO : 7 ; les résidus d'acides aminés 26 à 344 de SEQ ID NO : 8 ; les résidus d'acides aminés 26 à 310 de SEQ ID NO : 9 ; les résidus d'acides aminés 27 à 344 de SEQ ID NO : 8 ; et les résidus d'acides aminés 27 à 310 de SEQ ID NO : 9.

20. Polypeptide du récepteur Nogo 1 soluble selon la revendication 19 joint à une séquence signal.

21. Molécule d'acide nucléique codant pour le polypeptide du récepteur Nogo 1 soluble selon l'une quelconque des revendications 19 à 20.

22. Molécule d'acide nucléique selon la revendication 21 liée de manière fonctionnelle à une séquence de contrôle de l'expression.

23. Vecteur comprenant la molécule d'acide nucléique selon la revendication 21 ou 22.

24. Cellule hôte comprenant la molécule d'acide nucléique selon la revendication 21 ou 22 ou comprenant le vecteur selon la revendication 23.

25. Procédé de production du polypeptide du récepteur Nogo 1 soluble selon l'une quelconque des revendications 19 à 20, comprenant les étapes consistant à :
(a) cultiver la cellule hôte selon la revendication 24 ; et
(b) récupérer le polypeptide à partir de la cellule hôte ou du milieu de culture.

26. Protéine de fusion du récepteur Nogo 1 comprenant :
(a) les résidus d'acides aminés 26 à 344 de SEQ ID NO : 6 ; les résidus d'acides aminés 26 à 310 de SEQ ID NO : 7 ; les résidus d'acides aminés 26 à 344 de SEQ ID NO : 8 ; les résidus d'acides aminés 26 à 310 de SEQ ID NO : 9 ; les résidus d'acides aminés 27 à 344 de SEQ ID NO : 8 ; et les résidus d'acides aminés 27 à 310 de SEQ ID NO : 9 ; et
(b) un polypeptide hétérologue comprenant :
(i) une région constante d'immunoglobuline ; ou
(ii) une région Fc.

27. Protéine de fusion du récepteur Nogo 1 selon la revendication 26, qui est un dimère.

28. Protéine de fusion du récepteur Nogo 1 selon la revendication 26 ou 27, dans laquelle la région constante de l'immunoglobuline est une région constante de chaîne lourde d'immunoglobuline.

29. Protéine de fusion du récepteur Nogo 1 selon la revendication 28, dans laquelle la région constante de chaîne lourde d'immunoglobuline est une région constante de chaîne lourde d'IgG.

30. Protéine de fusion du récepteur Nogo 1 selon l'une quelconque des revendications 26 à 29, comprenant en outre une séquence signal.

31. Molécule d'acide nucléique codant pour la protéine de fusion du récepteur Nogo 1 selon l'une quelconque des revendications 26 à 30.

32. Molécule d'acide nucléique selon la revendication 31 liée de manière fonctionnelle à une séquence de contrôle de l'expression.

33. Vecteur comprenant la molécule d'acide nucléique selon la revendication 31 ou 32.

34. Cellule hôte comprenant la molécule d'acide nucléique selon la revendication 31 ou 32 ou comprenant le vecteur selon la revendication 33.

35. Procédé de production de la protéine de fusion du récepteur Nogo 1 selon l'une quelconque des revendications 26 à 30, comprenant les étapes consistant à :
(a) cultiver la cellule hôte selon la revendication 34 ; et
(b) récupérer la protéine de fusion du récepteur Nogo 1 à partir de la cellule hôte ou du milieu de culture.

36. Procédé de production d'un anticorps spécifique du récepteur Nogo 1, comprenant les étapes consistant à :
(a) immuniser un hôte avec un polypeptide du récepteur Nogo 1 soluble selon l'une quelconque des revendications 19 à 20, une protéine de fusion du récepteur Nogo 1 selon l'une quelconque des revendications 26 à 30 ou une cellule hôte selon la revendication 24 ou 34 ; et
(b) récupérer l'anticorps.

37. Anticorps ou fragment de liaison de l'antigène de celui-ci qui se lie spécifiquement à un polypeptide du récepteur Nogo 1 soluble selon l'une quelconque des revendications 19 à 20, ou produit par le procédé selon la revendication 36, où ledit anticorps ou fragment de liaison de l'antigène :
(a) inhibe le collapsus du cône de croissance d'un neurone ;
(b) diminue l'inhibition de la croissance des neurites et du bourgeonnement dans un neurone ; ou
(c) inhibe la liaison du récepteur Nogo 1 à un ligand.

38. Procédé *in vitro* d'inhibition de la liaison du récepteur Nogo 1 à un ligand, comprenant l'étape consistant à :
(a) mettre en contact le récepteur Nogo 1 avec un anticorps ou un fragment de liaison de l'antigène selon l'une quelconque des revendications 8 à 11 et 13 à 18 ; ou
(b) mettre en contact le ligand avec le polypeptide du récepteur Nogo 1 soluble selon l'une quelconque des revendications 19 à 20 ou la protéine de fusion du récepteur Nogo 1 selon l'une quelconque des revendications 26 à 30.

39. Utilisation d'un anticorps ou d'un fragment de liaison de l'antigène selon l'une quelconque des revendications 8 à 11 et 13 à 18 ou du polypeptide du récepteur Nogo 1 soluble selon l'une quelconque des revendications 19 à 20 ou de la protéine de fusion du récepteur Nogo 1 selon l'une quelconque des revendications 26 à 30, pour la préparation d'une composition pharmaceutique destinée au traitement d'un mammifère affichant des signes ou des symptômes de sclérose en plaques, SLA, maladie de Huntington, maladie d'Alzheimer, maladie de Parkinson, neuropathie diabétique, accident vasculaire cérébral, lésions cérébrales traumatiques ou lésion de la moelle épinière.

40. Procédé *in vitro* de modulation d'une activité d'un ligand du récepteur Nogo 1, comprenant l'étape consistant à mettre en contact le ligand du récepteur Nogo 1 avec le polypeptide selon l'une quelconque des revendications 19 à 20 ou la protéine de fusion du récepteur Nogo 1 selon l'une quelconque des revendications 26 à 30.

41. Utilisation du polypeptide selon l'une quelconque des revendications 19 à 20 ou de la protéine de fusion du récepteur Nogo 1 selon l'une quelconque des revendications 26 à 30, pour la préparation d'une composition pharmaceutique destinée au traitement d'un mammifère affichant des signes ou des symptômes de sclérose en plaques, SLA, maladie de Huntington, maladie d'Alzheimer, maladie de Parkinson, neuropathie diabétique, accident vasculaire cérébral, lésions cérébrales traumatiques ou lésion de la moelle épinière.

42. Procédé *in vitro* d'inhibition du collapsus du cône de croissance dans un neurone, comprenant l'étape consistant à :
(a) mettre en contact le neurone avec l'anticorps ou le fragment de liaison de l'antigène de celui-ci selon l'une quelconque des revendications 8 à 11 et 13 à 18 ; ou
(b) mettre en contact un ligand du récepteur Nogo 1 avec le polypeptide selon l'une quelconque des revendications 19 à 20 ou la protéine de fusion du récepteur Nogo 1 selon l'une quelconque des revendications 26 à 30.

43. Procédé *in vitro* de diminution de l'inhibition de la croissance des neurites ou du bourgeonnement dans un neurone, comprenant l'étape consistant à :
(a) mettre en contact le neurone avec l'anticorps ou le fragment de liaison de l'antigène de celui-ci selon l'une quelconque des revendications 8 à 11 et 13 à 18 ; ou
(b) mettre en contact un ligand du récepteur Nogo 1 avec le polypeptide selon l'une quelconque des revendications 19 à 20 ou la protéine de fusion du récepteur Nogo 1 selon l'une quelconque des revendications 26 à 30.

44. Procédé selon l'une quelconque des revendications 38, 40, 42 et 43, dans lequel le ligand est choisi dans le groupe constitué par NogoA, NogoB, NogoC, MAG et OM-gp.

45. Procédé selon la revendication 43, dans lequel la croissance des neurites ou le bourgeonnement représente la croissance axonale.

46. Procédé selon l'une quelconque des revendications 42, 43 et 45, dans lequel le neurone est un neurone du SNC.

47. Composition comprenant un support pharmaceutiquement acceptable et un composant choisi parme :
(a) l'anticorps ou un fragment de liaison de l'antigène selon l'une quelconque des revendications 8 à 11, 13 à 18 et 37 ;
(b) le polypeptide du récepteur Nogo 1 soluble selon l'une quelconque des revendications 19 à 20 ; et
(c) la protéine de fusion selon l'une quelconque des revendications 26 à 30.

48. Composition selon la revendication 47, comprenant en outre un ou plusieurs agents thérapeutiques supplémentaires.

49. Procédé *in vitro* permettant de favoriser la survie d'un neurone à risque de mourir, comprenant la mise en contact du neurone avec une quantité efficace :
(a) d'un anticorps anti-récepteur Nogo 1 ou d'un fragment de liaison de l'antigène selon l'une quelconque des revendications 8 à 11 et 13 à 18 ; ou
(b) d'un polypeptide du récepteur Nogo 1 soluble selon l'une quelconque des revendications 19 à 20 ; ou
(c) d'une protéine de fusion du récepteur Nogo 1 selon l'une quelconque des revendications 26 à 30.

50. Procédé selon la revendication 49 ou utilisation selon la revendication 39 ou 41, où la protéine de fusion est une protéine de fusion de Fc.

51. Procédé ou utilisation selon la revendication 50, où la protéine de fusion de Fc représente les résidus d'acides aminés 26 à 344 de SEQ ID NO : 8 et la région charnière et Fc de la molécule d'IgG1 de rat (Ig-sNogoR344).

52. Procédé selon l'une quelconque des revendications 49 à 51, dans lequel le neurone provient d'un mammifère.

53. Procédé selon la revendication 52, dans lequel le mammifère affiche des signes ou des symptômes de sclérose en plaques, SLA, maladie de Huntington, maladie d'Alzheimer, maladie de Parkinson, neuropathie diabétique, accident vasculaire cérébral, lésions cérébrales traumatiques ou lésion de la moelle épinière.

54. Utilisation d'une cellule hôte cultivée exprimant :
(a) un anticorps anti-récepteur Nogo 1 ou un fragment de liaison de l'antigène de celui-ci selon l'une quelconque des revendications 8 à 11 et 13 à 18 ; ou
(b) un polypeptide du récepteur Nogo 1 soluble selon l'une quelconque des revendications 19 à 20 ; ou
(c) une protéine de fusion du récepteur Nogo 1 selon l'une quelconque des revendications 26 à 30 ;
pour la préparation d'une composition pharmaceutique destinée au traitement d'un mammifère affichant des signes ou des symptômes de sclérose en plaques, SLA, maladie de Huntington, maladie d'Alzheimer, maladie de Parkinson, neuropathie diabétique, accident vasculaire cérébral, lésions cérébrales traumatiques ou lésion de la moelle épinière, où ladite cellule hôte doit être introduite chez le mammifère au niveau ou près du site d'un neurone qui est à risque de mourir.

55. Utilisation d'un vecteur viral comprenant une séquence nucléotidique qui code pour :
(a) un anticorps anti-récepteur Nogo 1 ou un fragment de liaison de l'antigène de celui-ci selon l'une quelconque des revendications 8 à 11 et 13 à 18 ; ou
(b) un polypeptide du récepteur Nogo 1 soluble selon l'une quelconque des revendications 19 à 20 ; ou
(c) une protéine de fusion du récepteur Nogo 1 selon l'une quelconque des revendications 26 à 30 ;
pour la préparation d'une composition pharmaceutique destinée au traitement d'un mammifère affichant des signes ou des symptômes de sclérose en plaques, SLA, maladie de Huntington, maladie d'Alzheimer, maladie de Parkinson, neuropathie diabétique, accident vasculaire cérébral, lésions cérébrales traumatiques ou lésion de la moelle épinière, où l'anticorps anti-récepteur Nogo 1, le fragment de liaison de l'antigène, la protéine de fusion du récepteur Nogo 1 ou le polypeptide du récepteur Nogo 1 soluble doit être exprimé à partir de la séquence nucléotidique chez le mammifère dans une quantité suffisante pour favoriser la survie d'un neurone à risque de mourir.


### SEQUENCE LISTING

<110> Yale University, et al
<120> NOGO RECEPTOR ANTAGONISTS
<130> P29268EP-PCT
<140> EP03785123.5
   <141> 2003-08-07
<150> 60/402,866
   <151> 2002-08-10
<160> 25
<170> PatentIn Ver. 2.1
<210> 1
   <211> 16
   <212> PRT
   <213> Rattus sp.
<400> 1
   Leu Asp Leu Ser Asp Asn Ala Gln Leu Arg Val Val Asp Pro Thr Thr
     1               5                  10                  15
<210> 2
   <211> 16
   <212> PRT
   <213> Homo sapiens
<400> 2
   Leu Asp Leu Ser Asp Asn Ala Gln Leu Arg Ser Val Asp Pro Ala Thr
     1               5                  10                  15
<210> 3
   <211> 35
   <212> PRT
   <213> Rattus sp.
<400> 3
   Ala Val Ala Ser Gly Pro Phe Arg Pro Phe Gln Thr Asn Gln Leu Thr
     1               5                  10                  15
   Asp Glu Glu Leu Leu Gly Leu Pro Lys Cys Cys Gln Pro Asp Ala Ala
                20                  25                  30
   Asp Lys Ala
            35
<210> 4
   <211> 35
   <212> PRT
   <213> Homo sapiens
<400> 4
   Ala Val Ala Thr Gly Pro Tyr His Pro Ile Trp Thr Gly Arg Ala Thr
   1               5                   10                  15
   Asp Glu Glu Pro Leu Gly Leu Pro Lys Cys Cys Gln Pro Asp Ala Ala
               20                  25                  30
   Asp Lys Ala
           35
<210> 5
   <211> 10
   <212> PRT
   <213> Mus musculus
<400> 5
   Cys Arg Leu Gly Gln Ala Gly Ser Gly Ala
   1               5                   10
<210> 6
   <211> 344
   <212> PRT
   <213> Homo sapiens
<400> 6
   Met Lys Arg Ala Ser Ala Gly Gly Ser Arg Leu Leu Ala Trp Val Leu
   1               5                   10                  15
   Trp Leu Gln Ala Trp Gln Val Ala Ala Pro Cys Pro Gly Ala Cys Val
               20                  25                  30
   Cys Tyr Asn Glu Pro Lys Val Thr Thr Ser Cys Pro Gln Gln Gly Leu
           35                  40                  45
   Gln Ala Val Pro Val Gly Ile Pro Ala Ala Ser Gln Arg Ile Phe Leu
       50                  55                  60
   His Gly Asn Arg Ile Ser His Val Pro Ala Ala Ser Phe Arg Ala Cys
   65                  70                  75                  80
   Arg Asn Leu Thr Ile Leu Trp Leu His Ser Asn Val Leu Ala Arg Ile
                   85                  90                  95
   Asp Ala Ala Ala Phe Thr Gly Leu Ala Leu Leu Glu Gln Leu Asp Leu
               100                 105                 110
   Ser Asp Asn Ala Gln Leu Arg Ser Val Asp Pro Ala Thr Phe His Gly
           115                 120                 125
   Leu Gly Arg Leu His Thr Leu His Leu Asp Arg Cys Gly Leu Gln Glu
       130                 135                 140
   Leu Gly Pro Gly Leu Phe Arg Gly Leu Ala Ala Leu Gln Tyr Leu Tyr
   145                 150                 155                 160
   Leu Gln Asp Asn Ala Leu Gln Ala Leu Pro Asp Asp Thr Phe Arg Asp
                   165                 170                 175
   Leu Gly Asn Leu Thr His Leu Phe Leu His Gly Asn Arg Ile Ser Ser
               180                 185                 190
   Val Pro Glu Arg Ala Phe Arg Gly Leu His Ser Leu Asp Arg Leu Leu
           195                 200                 205
   Leu His Gln Asn Arg Val Ala His Val His Pro His Ala Phe Arg Asp
       210                 215                 220
   Leu Gly Arg Leu Met Thr Leu Tyr Leu Phe Ala Asn Asn Leu Ser Ala
   225                 230                 235                 240
   Leu Pro Thr Glu Ala Leu Ala Pro Leu Arg Ala Leu Gln Tyr Leu Arg
                   245                 250                 255
   Leu Asn Asp Asn Pro Trp Val Cys Asp Cys Arg Ala Arg Pro Leu Trp
               260                 265                 270
   Ala Trp Leu Gln Lys Phe Arg Gly Ser Ser Ser Glu Val Pro Cys Ser
           275                 280                 285
   Leu Pro Gln Arg Leu Ala Gly Arg Asp Leu Lys Arg Leu Ala Ala Asn
       290                 295                 300
   Asp Leu Gln Gly Cys Ala Val Ala Thr Gly Pro Tyr His Pro Ile Trp
   305 310 315 320
   Thr Gly Arg Ala Thr Asp Glu Glu Pro Leu Gly Leu Pro Lys Cys Cys
   325 330 335
   Gln Pro Asp Ala Ala Asp Lys Ala
   340
<210> 7
   <211> 310
   <212> PRT
   <213> Homo sapiens
<400> 7
   Met Lys Arg Ala Ser Ala Gly Gly Ser Arg Leu Leu Ala Trp Val Leu
   1               5                   10                  15
   Trp Leu Gln Ala Trp Gln Val Ala Ala Pro Cys Pro Gly Ala Cys Val
               20                  25                  30
   Cys Tyr Asn Glu Pro Lys Val Thr Thr Ser Cys Pro Gln Gln Gly Leu
           35                  40                  45
   Gln Ala Val Pro Val Gly Ile Pro Ala Ala Ser Gln Arg Ile Phe Leu
       50                  55                  60
   His Gly Asn Arg Ile Ser His Val Pro Ala Ala Ser Phe Arg Ala Cys
   65                  70                  75                  80
   Arg Asn Leu Thr Ile Leu Trp Leu His Ser Asn Val Leu Ala Arg Ile
                   85                  90                  95
   Asp Ala Ala Ala Phe Thr Gly Leu Ala Leu Leu Glu Gln Leu Asp Leu
               100                 105                 110
   Ser Asp Asn Ala Gln Leu Arg Ser Val Asp Pro Ala Thr Phe His Gly
           115                 120                 125
   Leu Gly Arg Leu His Thr Leu His Leu Asp Arg Cys Gly Leu Gln Glu
       130                 135                 140
   Leu Gly Pro Gly Leu Phe Arg Gly Leu Ala Ala Leu Gln Tyr Leu Tyr
   145                 150                 155                 160
   Leu Gln Asp Asn Ala Leu Gln Ala Leu Pro Asp Asp Thr Phe Arg Asp
                   165                 170                 175
   Leu Gly Asn Leu Thr His Leu Phe Leu His Gly Asn Arg Ile Ser Ser
               180                 185                 190
   Val Pro Glu Arg Ala Phe Arg Gly Leu His Ser Leu Asp Arg Leu Leu
           195                 200                 205
   Leu His Gln Asn Arg Val Ala His Val His Pro His Ala Phe Arg Asp
       210                 215                 220
   Leu Gly Arg Leu Met Thr Leu Tyr Leu Phe Ala Asn Asn Leu Ser Ala
   225                 230                 235                 240
   Leu Pro Thr Glu Ala Leu Ala Pro Leu Arg Ala Leu Gln Tyr Leu Arg
                   245                 250                 255
   Leu Asn Asp Asn Pro Trp Val Cys Asp Cys Arg Ala Arg Pro Leu Trp
               260                 265                 270
   Ala Trp Leu Gln Lys Phe Arg Gly Ser Ser Ser Glu Val Pro Cys Ser
           275                 280                 285
   Leu Pro Gln Arg Leu Ala Gly Arg Asp Leu Lys Arg Leu Ala Ala Asn
       290                 295                 300
   Asp Leu Gln Gly Cys Ala
   305                 310
<210> 8
   <211> 344
   <212> PRT
   <213> Rattus sp.
<400> 8
   Met Lys Arg Ala Ser Ser Gly Gly Ser Arg Leu Pro Thr Trp Val Leu
   1               5                   10                  15
   Trp Leu Gln Ala Trp Arg Val Ala Thr Pro Cys Pro Gly Ala Cys Val
               20                  25                  30
   Cys Tyr Asn Glu Pro Lys Val Thr Thr Ser Arg Pro Gln Gln Gly Leu
           35                  40                  45
   Gln Ala Val Pro Ala Gly Ile Pro Ala Ser Ser Gln Arg Ile Phe Leu
       50                  55                  60
   His Gly Asn Arg Ile Ser Tyr Val Pro Ala Ala Ser Phe Gln Ser Cys
   65                  70                  75                  80
   Arg Asn Leu Thr Ile Leu Trp Leu His Ser Asn Ala Leu Ala Gly Ile
                   85                  90                  95
   Asp Ala Ala Ala Phe Thr Gly Leu Thr Leu Leu Glu Gln Leu Asp Leu
               100                 105                 110
   Ser Asp Asn Ala Gln Leu Arg Val Val Asp Pro Thr Thr Phe Arg Gly
           115                 120                 125
   Leu Gly His Leu His Thr Leu His Leu Asp Arg Cys Gly Leu Gln Glu
       130                 135                 140
   Leu Gly Pro Gly Leu Phe Arg Gly Leu Ala Ala Leu Gln Tyr Leu Tyr
   145                 150                 155                 160
   Leu Gln Asp Asn Asn Leu Gln Ala Leu Pro Asp Asn Thr Phe Arg Asp
                   165                 170                 175
   Leu Gly Asn Leu Thr His Leu Phe Leu His Gly Asn Arg Ile Pro Ser
               180                 185                 190
   Val Pro Glu His Ala Phe Arg Gly Leu His Ser Leu Asp Arg Leu Leu
           195                 200                 205
   Leu His Gln Asn His Val Ala Arg Val His Pro His Ala Phe Arg Asp
       210                 215                 220
   Leu Gly Arg Leu Met Thr Leu Tyr Leu Phe Ala Asn Asn Leu Ser Met
   225                 230                 235                 240
   Leu Pro Ala Glu Val Leu Val Pro Leu Arg Ser Leu Gln Tyr Leu Arg
                   245                 250                 255
   Leu Asn Asp Asn Pro Trp Val Cys Asp Cys Arg Ala Arg Pro Leu Trp
               260                 265                 270
   Ala Trp Leu Gln Lys Phe Arg Gly Ser Ser Ser Gly Val Pro Ser Asn
           275                 280                 285
   Leu Pro Gln Arg Leu Ala Gly Arg Asp Leu Lys Arg Leu Ala Thr Ser
       290                 295                 300
   Asp Leu Glu Gly Cys Ala Val Ala Ser Gly Pro Phe Arg Pro Phe Gln
   305                 310                 315                 320
   Thr Asn Gln Leu Thr Asp Glu Glu Leu Leu Gly Leu Pro Lys Cys Cys
                   325                 330                 335
   Gln Pro Asp Ala Ala Asp Lys Ala
   340
<210> 9
   <211> 310
   <212> PRT
   <213> Rattus sp.
<400> 9
   Met Lys Arg Ala Ser Ser Gly Gly Ser Arg Leu Pro Thr Trp Val Leu
    1               5                  10                  15
   Trp Leu Gln Ala Trp Arg Val Ala Thr Pro Cys Pro Gly Ala Cys Val
               20                  25                  30
   Cys Tyr Asn Glu Pro Lys Val Thr Thr Ser Arg Pro Gln Gln Gly Leu
           35                  40                  45
   Gln Ala Val Pro Ala Gly Ile Pro Ala Ser Ser Gln Arg Ile Phe Leu
       50                  55                  60
   His Gly Asn Arg Ile Ser Tyr Val Pro Ala Ala Ser Phe Gln Ser Cys
   65                  70                  75                  80
   Arg Asn Leu Thr Ile Leu Trp Leu His Ser Asn Ala Leu Ala Gly Ile
                   85                  90                  95
   Asp Ala Ala Ala Phe Thr Gly Leu Thr Leu Leu Glu Gln Leu Asp Leu
               100                 105                 110
   Ser Asp Asn Ala Gln Leu Arg Val Val Asp Pro Thr Thr Phe Arg Gly
           115                 120                 125
   Leu Gly His Leu His Thr Leu His Leu Asp Arg Cys Gly Leu Gln Glu
       130                 135                 140
   Leu Gly Pro Gly Leu Phe Arg Gly Leu Ala Ala Leu Gln Tyr Leu Tyr
   145                 150                 155                 160
   Leu Gln Asp Asn Asn Leu Gln Ala Leu Pro Asp Asn Thr Phe Arg Asp
                   165                 170                 175
   Leu Gly Asn Leu Thr His Leu Phe Leu His Gly Asn Arg Ile Pro Ser
               180                 185                 190
   Val Pro Glu His Ala Phe Arg Gly Leu His Ser Leu Asp Arg Leu Leu
           195                 200                 205
   Leu His Gln Asn His Val Ala Arg Val His Pro His Ala Phe Arg Asp
       210                 215                 220
   Leu Gly Arg Leu Met Thr Leu Tyr Leu Phe Ala Asn Asn Leu Ser Met
   225                 230                 235                 240
   Leu Pro Ala Glu Val Leu Val Pro Leu Arg Ser Leu Gln Tyr Leu Arg
                   245                 250                 255
   Leu Asn Asp Asn Pro Trp Val Cys Asp Cys Arg Ala Arg Pro Leu Trp
               260                 265                 270
   Ala Trp Leu Gln Lys Phe Arg Gly Ser Ser Ser Gly Val Pro Ser Asn
           275                 280                 285
   Leu Pro Gln Arg Leu Ala Gly Arg Asp Leu Lys Arg Leu Ala Thr Ser
       290                 295                 300
   Asp Leu Glu Gly Cys Ala
   305                 310
<210> 10
   <211> 15
   <212> PRT
   <213> Artificial Sequence
<220>
   <223> Description of Artificial Sequence: Synthetic linker
<400> 10
   Gly Gly Gly Gly Ser Gly Gly Gly Gly Ser Gly Gly Gly Gly Ser
   1               5                   10                  15
<210> 11
   <211> 19
   <212> PRT
   <213> Rattus sp.
<400> 11
   Arg Val His Pro His Ala Phe Arg Asp Leu Gly Arg Leu Met Thr Leu
   1               5                   10                  15
   Tyr Leu Phe
<210> 12
   <211> 34
   <212> DNA
   <213> Artificial Sequence
<220>
   <223> Description of Artificial Sequence: Primer
<400> 12
   tgaggagacg gtgaccgtgg tcccttggcc ccag 34
<210> 13
   <211> 37
   <212> DNA
   <213> Artificial Sequence
<220>
   <223> Description of Artificial Sequence: Primer
<400> 13
   ggggatatcc accatgaagt tgcctgttag gctgttg 37
<210> 14
   <211> 40
   <212> DNA
   <213> Artificial Sequence
<220>
   <223> Description of Artificial Sequence: Degenerate primer
<400> 14
   ggggatatcc accatgaggk ccccwgctca gytyctkgga 40
<210> 15
   <211> 144
   <212> PRT
   <213> Artificial sequence
<220>
   <223> Description of Artificial Sequence: Synthetic light chain peptide sequence
<400> 15
   Met Lys Leu Pro Val Arg Leu Leu Val Leu Met Phe Trp Ile Pro Ala
    1               5                  10                  15
   Ser Ser Ser Asp Val Val Met Thr Gln Thr Pro Leu Ser Leu Pro Val
               20                  25                  30
   Ser Leu Gly Asp Gln Ala Ser Ile Ser Cys Arg Ser Ser Gln Ser Leu
           35                  40                  45
   Val His Ser Asn Gly Asn Thr Tyr Leu His Trp Tyr Leu Gln Lys Pro
       50                  55                  60
   Gly Gln Ser Pro Lys Leu Leu Ile Tyr Lys Val Ser Asn Arg Phe Ser
   65                  70                  75                  80
   Gly Val Pro Asp Arg Phe Ser Gly Ser Gly Ser Gly Thr Asp Phe Thr
                   85                  90                  95
   Leu Lys Ile Ser Arg Val Asp Ala Glu Asp Leu Gly Val Tyr Phe Cys
               100                 105                 110
   Ser Gln Ser Thr His Val Pro Phe Thr Phe Gly Gly Gly Thr Lys Leu
           115                 120                 125
   Glu Ile Lys Arg Ala Asp Ala Ala Pro Thr Val Ser Ile Ser His His
       130                 135                 140
<210> 16
   <211> 144
   <212> PRT
   <213> Artificial sequence
<220>
   <223> Description of Artificial Sequence: Synthetic light chain peptide sequence
<400> 16
   Met Lys Leu Pro Val Arg Leu Leu Val Leu Met Phe Trp Ile Pro Ala
   1               5                   10                  15
   Ser Ser Ser Asp Val Val Met Thr Gln Thr Pro Leu Ser Leu Pro Val
               20                  25                  30
   Ser Leu Gly Asp Gln Ala Ser Ile Ser Cys Arg Ser Ser Gln Ser Leu
           35                  40                  45
   Val His Ser Asn Gly Tyr Thr Tyr Leu His Trp Tyr Leu Gln Arg Pro
       50                  55                  60
   Gly Gln Ser Pro Lys Leu Leu Ile Tyr Lys Val Ser Asn Arg Phe Ser
   65                  70                  75                  80
   Gly Val Pro Asp Arg Phe Ser Gly Ser Gly Ser Gly Thr Asp Phe Thr
                   85                  90                  95
   Leu Lys Ile Ser Arg Val Asp Ala Glu Asp Leu Gly Val Tyr Phe Cys
               100                 105                 110
   Ser Gln Ser Thr His Val Pro Tyr Thr Phe Gly Gly Gly Thr Lys Leu
           115                 120                 125
   Glu Ile Lys Arg Ala Asp Ala Ala Pro Thr Val Ser Ile Ser His His
       130                 135                 140
<210> 17
   <211> 116
   <212> PRT
   <213> Artificial Sequence
<220>
   <223> Description of Artificial Sequence: Synthetic heavy chain peptide sequence
<400> 17
   Val Gln Leu Gln Glu Ser Gly Ala Glu Leu Val Met Pro Gly Ala Ser
   1               5                   10                  15
   Val Lys Met Ser Cys Lys Ala Ser Gly Tyr Thr Phe Thr Asp Tyr Trp
               20                  25                  30
   Met His Trp Val Lys Gln Arg Pro Gly Gln Gly Leu Glu Trp Ile Gly
           35                  40                  45
   Ala Ile Asp Pro Ser Asp Ser Tyr Ser Ser Tyr Asn Gln Asn Phe Lys
       50                  55                  60
   Gly Lys Ala Thr Leu Thr Val Asp Gly Ser Ser Ser Thr Ala Tyr Met
   65                  70                  75                  80
   Gln Leu Ser Ser Leu Thr Ser Glu Asp Ser Ala Val Tyr Tyr Cys Ala
                   85                  90                  95
   Arg Arg Ile Thr Glu Ala Gly Ala Trp Phe Ala Tyr Trp Gly Gln Gly
               100                 105                 110
   Thr Thr Val Thr
           115
<210> 18
   <211> 114
   <212> PRT
   <213> Artificial Sequence
<220>
   <223> Description of Artificial Sequence: Synthetic heavy chain peptide sequence
<220>
   <221> MOD_RES
   <222> (3)
   <223> variable amino acid
<400> 18
   Leu Gln Xaa Ser Gly Ala Glu Ile Val Met Pro Gly Thr Ala Val Thr
   1               5                   10                  15
   Met Ser Cys Lys Ala Ser Gly Tyr Thr Phe Thr Asp Phe Trp Met His
               20                  25                  30
   Trp Val Lys Gln Arg Pro Gly Gln Gly Leu Glu Trp Ile Gly Ala Ile
           35                  40                  45
   Asp Pro Ser Asp Ser Tyr Ser Arg Ile Asn Gln Lys Phe Lys Gly Lys
       50                  55                  60
   Ala Thr Leu Thr Val Asp Glu Ser Ser Ser Thr Ala Tyr Met Gln Leu
   65                  70                  75                  80
   Ser Ser Leu Thr Ser Glu Asp Ser Ala Val Tyr Tyr Cys Ala Arg Arg
                   85                  90                  95
   Ile Thr Glu Ala Gly Ala Trp Phe Ala Tyr Trp Gly Gln Gly Thr Thr
               100                 105                 110
   Val Thr
<210> 19
   <211> 12
   <212> PRT
   <213> Artificial Sequence
<220>
   <223> Description of Artificial Sequence: Synthetic heavy chain peptide sequence
<400> 19
   Gly Phe Ser Leu Ser Thr Ser Gly Gly Ser Val Gly
   1               5                   10
<210> 20
   <211> 16
   <212> PRT
   <213> Artificial Sequence
<220>
   <223> Description of Artificial Sequence: Synthetic heavy chain peptide sequence
<400> 20
   Leu Ile Tyr Ser Asn Asp Thr Lys Tyr Tyr Ser Thr Ser Leu Lys Thr
   1 5 10 15
<210> 21
   <211> 10
   <212> PRT
   <213> Artificial Sequence
<220>
   <223> Description of Artificial sequence: Synthetic heavy chain peptide sequence
<400> 21
   Ser Arg Phe Trp Thr Gly Glu Tyr Asp Val
   1 5 10
<210> 22
   <211> 11
   <212> PRT
   <213> Artificial Sequence
<220>
   <223> Description of Artificial Sequence: Synthetic light chain peptide sequence
<400> 22
   Arg Ala Ser Gln Asn Ile Ala Ile Thr Leu Asn
   1 5 10
<210> 23
   <211> 7
   <212> PRT
   <213> Artificial Sequence
<220>
   <223> Description of Artificial Sequence: Synthetic light chain peptide sequence
<400> 23
   Leu Ala Ser Ser Leu Gln Ser
   1 5
<210> 24
   <211> 8
   <212> PRT
   <213> Artificial Sequence
<220>
   <223> Description of Artificial sequence: Synthetic light chain peptide sequence
<400> 24
   Gln Gln Tyr Asp Asn Tyr Pro Leu
    1               5
<210> 25
   <211> 22
   <212> DNA
   <213> Artificial Sequence
<220>
   <223> Description of Artificial Sequence: Degenerate primer
<400> 25
   aggtsmarct gcagsagtcw gg 22